# EUROPEAN PATENT SPECIFICATION

(11) **EP 3 341 487 B1**
(45) Date of publication and mention of the grant of the patent: **01.10.2025**
(21) Application number: 16767152.8
(22) Date of filing: 29.08.2016
(51) Int. Cl.: C12P 7/10, C12P 7/44, C12N 9/02

(54) **MEANS AND METHODS FOR PRODUCTION OF ORGANIC COMPOUNDS**
MITTEL UND VERFAHREN ZUR HERSTELLUNG ORGANISCHER VERBINDUNGEN
MOYENS ET MÉTHODES POUR LA PRODUCTION DE COMPOSÉS ORGANIQUES

(30) Priority: 28.08.2015 EP 15002547; 08.09.2015 LU 92822
(43) Date of publication of application: 04.07.2018
(73) Proprietor: Universität des Saarlandes, 66123 Saarbrücken (DE)
(72) Inventor: VAN DUUREN, Jozef, 68526 Ladenburg (DE); STOLZENBERGER, Jessica, 33813 Oerlinghausen (DE); KOHLSTEDT, Michael, 66119 Saarbruecken (DE); STARCK, Sören, 67435 Neustadt (DE); SELZER, Mirjam, 66113 Saarbrücken (DE); FRITZ, Michel, 57600 Forbach (FR); HOELTZEN, Heike, 66121 Saarbruecken (DE); RICHTER, Rudolf, 66123 Saarbruecken (DE); WITTMANN, Christoph, 66740 Saarlouis (DE)
(74) Representative: Schiweck Weinzierl Koch Patentanwälte Partnerschaft mbB
(86) International application number: PCT/EP2016/070307
(87) International publication number: WO 2017/037013

(56) References cited:
- WO-A1-2013/005202
- WO-A2-2012/075053
- WO-A2-2013/188546
- US-A1- 2013 017 579
- HAN LI ET AL: "Engineering catechol 1, 2-dioxygenase by design for improving the performance of the cis, cis-muconic acid synthetic pathway in Escherichia coli", SCIENTIFIC REPORTS, vol. 5, no. 1, 26 August 2015 (2015-08-26), XP093106702, Retrieved from the Internet <URL:https://www.nature.com/articles/srep13435.pdf> DOI: 10.1038/srep13435
- KOHLSTEDT MICHAEL ET AL: "From lignin to nylon: Cascaded chemical and biochemical conversion using metabolically engineered Pseudomonas putida", METABOLIC ENGINEERING, vol. 47, 1 May 2018 (2018-05-01), AMSTERDAM, NL, pages 279 - 293, XP093262646, ISSN: 1096-7176, Retrieved from the Internet <URL:https://pdf.sciencedirectassets.com/272595/1-s2.0-S1096717618X00032/1-s2.0-S1096717617304615/main.pdf> DOI: 10.1016/j.ymben.2018.03.003
- BEUM JUN KIM ET AL: "Enhancement of cis,cis-muconate productivity by overexpression of catechol 1,2-dioxygenase in Pseudomonas putida BCM114", BIOTECHNOLOGY AND BIOPROCESS ENGINEERING, KOREAN SOCIETY FOR BIOTECHNOLOGY AND BIOENGINEERING, SEOUL, KR, vol. 3, 1 January 1998 (1998-01-01), pages 112 - 114, XP002667138, ISSN: 1226-8372
- HANNA PIKOWSKA ET AL: "Hydrothermal decomposition of alkali lignin in sub- and supercritical water", CHEMICAL ENGINEERING JOURNAL, ELSEVIER SEQUOIA, LAUSANNE, CH, vol. 187, 20 January 2012 (2012-01-20), pages 410 - 414, XP028464906, ISSN: 1385-8947, [retrieved on 20120128], DOI: 10.1016/J.CEJ.2012.01.092
- "SubName: Full=Catechol 1,2-dioxygenase", UNIPROT, 2002, XP002667139
- SZAFRANSKI P ET AL: "Principal transcription sigma factors of Pseudomonas putida strains mt-2 and G1 are significantly different", GENE, ELSEVIER, AMSTERDAM, NL, vol. 204, no. 1-2, 19 December 1997 (1997-12-19), pages 133 - 138, XP004100704, ISSN: 0378-1119, DOI: 10.1016/S0378-1119(97)00533-7
- DATABASE EMBL [online] 15 April 2005 (2005-04-15), SZAFRANSKI P.: "Pseudomonas putida macromolecular synthesis operon: 30S subunit ribosomal protein S21 (rpsU), DNA primase (dnaG), and sigma-70 (rpoD) genes, complete cds.", XP002756238, Database accession no. AF014397
- NAKAI CHIEKO ET AL: "Cloning, DNA Sequencing, and Amino Acid Sequencing of Catechol 1,2-Dioxygenases (Pyrocatechase) from Pseudomonas putida mt-2 and Pseudomonas arvilla C-1", ARCHIVES OF BIOCHEMISTRY AND BIOPHYSICS, vol. 321, no. 2, 1995, pages 353 - 362, XP002756170, ISSN: 0003-9861

## Description

### BACKGROUND

Considering the rapidly growing world population and increasing demand for energy, packaging and building materials with potentially devastating consequences for our environment and living quality due to extensive utilization of fossil fuels and production of waste, the transformation of biomass into fuel and chemicals is becoming increasingly important as a way to mitigate global warming and diversify energy and chemical sources. Biomass, i.e. biological material derived from living, or recently living organisms, is a renewable, carbon-neutral resource. It has been estimated that biomass could provide about 25% of global energy requirements. In addition, biomass can also be a source of valuable and value-added chemicals, pharmaceuticals and food additives.

Lignocellulose describes the main constituents in most plants, namely cellulose, hemicelluloses, and lignin. Lignocellulosic biomass as present in waste from food and paper production and forestry as well as municipal solid waste (MSW), is mostly destroyed as low-grade fuel by burning or used as a low-value products (e.g. as flocculating and dispersing agents). Among the major constituents, cellulose contains large reservoirs of energy and is already used industrially for conversion into biofuels. Lignin constitutes 15-35% of the weight and carries the highest internal energy content of all the three fractions. Efficient conversion of lignin is, however, not trivial due to its complex, irregular structure, which complicates chemical conversion efforts. Thus, lignin valorization technologies are substantially less developed than those for the polysaccharides. (Pinkowska et al. Chem Eng J. 2012, 187: 410-414). However, economic viability of lignocellulosic biorefineries depends, besides the conversion of cellulose and hemicellulose, also on the conversion of lignin to value-added compounds.

There are several different methods by which lignin can be partially separated from lignocellulosic biomass. These processes can be classified into two general groups: (i) processes in which lignin is degraded into soluble fragments and is removed by separating the solid residue from the spent liquor (including pulping processes, such as kraft, sulfite, soda, and organosolv) and (ii) processes that selectively hydrolyze polysaccharides and leave lignin along with some condensed carbohydrate deconstruction products as a solid residue (e.g. dilute acid hydrolysis of lignocellulose to yield sugar monomers, furfural and levulinic acid) (Azadi et al. Renewable and Sustainable Energy Reviews. 2013; 21: 506-523).

Once separated, depolymerization is an important next step for many lignin valorization strategies, in order to generate valuable aromatic chemicals and/or provide a source of low-molecular-mass feedstocks suitable for downstream processing. Considerable amount of research has been done to convert lignin into renewable fuels and chemicals using pyrolysis and gasification methods. Biochemical depolymerization of lignin, such as depolymerisation by fungi, is hampered by its low efficiency. Chemical depolymerization methods, including acid- and base-catalyzed methods and depolymerisation in the presence of transition metal-based catalysts such as Ni and Ct, are also available, but mostly require harsh reaction conditions and are rather complicated to handle due to toxicity and flammability,. (Azadi et al. Renewable and Sustainable Energy Reviews. 2013; 21: 506-523).

Hydrothermal decomposition of lignin in sub- and supercritical water is a comparably unattended technique of lignin biomass treatment used rather in experimental than industrial applications. E.g, Wahyudiono et al. Chem Eng Proc. 2007, 47 (9-10): 1609-1619, 2008, performed hydrothermal decomposition lignin at 300°C and 25-40 MPa, and identified products including mainly catechol (28.37wt.%), phenol (7.53 wt.%), and cresol (11.67 wt.%). Pinkowska et al. Chem Eng J. 2012, 187: 410-414 reported successful hydrothermolysis of alkali lignin with relatively high molecular-weight (Mw = 28,000 and Mn = 5000) resulting in the production of phenolic compounds. The yield (wt%) of guaiacol, catechol, phenol and cresol isomers reached the values of approximately 11.23%, 11.11%, 4.21%, and 7.00% depending on reaction time and temperature.

Organic products from lignin depolymerisation can advantageously be employed as renewable sources of chemicals. E.g., adipic acid can e.g. be obtained from catechol via a variety of organic intermediate products, such as cis-cis-muconic acid, and is a value-added compound used primarily as a precursor for the synthesis of nylon, coatings, and plastics which is today produced mainly in chemical processes from petrochemicals like benzene. Because of the strong environmental impact of the conventional petrochemical production processes due to high energy costs and the dependence on fossil resources, biotechnological production processes would provide an attractive alternative. Lignin valorization into useful chemical compounds is however hampered by the fact that described lignin depolymerization techniques (pyrolysis, gasification, hydrogenolysis, chemical oxidation) typically result in a complex mixture of aromatic compounds in which the individual mass fraction of each compound barely exceeds few percent. In nature, some organisms have evolved metabolic pathways that enable the utilization of lignin-derived aromatic molecules as carbon sources. However, not all aromatics obtained from common lignin depolymerisation techniques are utilizable by said organisms. Consequently, recent efforts to utilize lignin as a renewable source for organic compounds with the help of biotechnological techniques are targeted primarily at the modification on the level of biocatalytic conversion in order to allow funneling of complex mixtures of organic compounds ("biocatalytic funneling"). This approach -as reported by Vardon et al. Energy & Environmental Science. 2015 (8): 617-628- typically requires extensive genetic modification of the biocatalyst, which can be complicated and time-consuming. Further, due to a specific conversion rate for each compound obtained after the depolymerization of lignin with the engineered biocatalyst, intermediates do accumulate and may polymerize leading to a dark coloration of the medium. This effect can even be enhanced, in case not all depolymerized compounds can be biologically converted. In the presence of accumulating compounds, which cannot be converted any further, the biocatalyst experiences increased stress. A major drawback of such an incomplete utilization of the raw material is the obtainment of a mixture of end products that require time-consuming and cost-intensive separation and purification. Hence, setting up a standardized process allowing for high reaction rates and resulting in a high yield of pure product(s) is complicated with biocatalytic funneling.

It was thus the object of the present invention to comply with the needs in the prior art and provide improved means and methods for producing useful compounds from organic (biomass) feedstock, in particular in lignin processing.

### SUMMARY

The present invention provides novel and advantageous approaches for conversion of organic compounds into useful products. As such, the present invention provides a method of producing an organic product, comprising
i) Fluid-assisted decomposition of an organic educt under sub- and/or supercritical conditions
ii) obtaining an intermediate product from step i)
iii) subjecting the intermediate product to biocatalytic conversion

In the method of the invention, steam bath distillation can be employed in obtaining the intermediate product in step (ii). It is envisaged that intermediate product obtained from step ii) has a degree of purity of 70% w/w or more, 75% w/w or more, 80% w/w or more, 85% w/w or more, 90% w/w or more, preferably 95% w/w or more, more preferably of 99% w/w or more. The intermediate product may comprise, e.g., catechol, phenol, m-cresol, p-cresol and/or o-cresol, in particular when the organic educt is selected from lignin, or guaiacol.

Generally, any organic educt is suitable to be processed according to the method of the invention. Particularly envisaged educts comprise lignin, guaiacol, p-coumaryl alcohol, coniferyl alcohol, sinapyl alcohol, catechol, m-cresol, p-cresol, o-cresol, phenol, polysaccharides, cellulose, hemicellulose, xylose, glucose, fructose, proteins, amino acids, triacylglycerides, and/or fatty acids.

In particular when the intermediate product is catechol, the product obtained from the method of the invention may be cis-cis-muconic acid. Advantageously, said cis-cis-muconic acid may be white in color. It is further envisaged that the yield of cis-cis-muconic acid from catechol is greater than 50 % w/w, greater than 60 % w/w, greater than 70% w/w, greater than 80% w/w, greater than 90 % w/w, preferably greater than 95 % w/w, even more preferred greater than 99% w/w. It is further envisaged that the yield of cis-cis-muconic acid from phenol is greater than 50 % w/w, greater than 60 % w/w, greater than 70% w/w, greater than 80% w/w, greater than 90 % w/w, preferably greater than 95 % w/w, preferably even more preferred greater than 99% w/w. It is further envisaged that the yield of cis-cis-muconic acid from cresol is greater than 50 % w/w, greater than 60 % w/w, greater than 70% w/w, greater than 80% w/w, greater than 90 % w/w, preferably greater than 95 % w/w, even more preferred greater than 99% w/w. It is further envisaged that the yield of cis-cis-muconic acid from guaiacol is greater than 50 % w/w, greater than 60 % w/w, greater than 70% w/w, greater than 80% w/w, greater than 90 % w/w, preferably greater than 95 % w/w, even more preferred greater than 99% w/w.

It is further envisaged that in step (iii), subjecting the intermediate product obtained before comprises contacting the intermediate product obtained in step ii) with a biocatalyst, in particular a biocatalyst selected from the group consisting of bacteria, yeast, filamentous fungi, cyanobacteria, algae, and plant cells. Pseudomonas, in particular *Pseudomonas putida,* such as the *Pseudomonas putida* strain KT2440 may be preferred host cells.

The host cell may in general be a non-genetically modified host cell or a genetically modified host cell (recombinant host cell) comprising at least one heterologous gene which may be stably integrated into the host cell's genome. Said at least one heterologous gene, in particular a *catA* gene and/or *catA2* gene, may be derived from *Pseudomonas, preferably Pseudomonas putida, more preferably Pseudomonas putida* strain KT2440.

The host cell may comprise at least two genes encoding a polypeptide having catechol 1,2-dioxygenase activity. Said gene may be endogenous or heterologous to the host cell. More specifically, the host cell may comprise at least one (optionally heterologous) *catA* gene and at least one (optionally heterologous) *catA2* gene. Said *catA* gene is envisaged to encode a catA polypeptide, e.g. a polypeptide comprising a sequence corresponding to SEQ ID No. 1. Said *catA* gene may comprise a sequence corresponding to SEQ ID No. 2. Said *catA2* gene is envisaged to encode a catA2 polypeptide, e.g. a polypeptide comprising a sequence corresponding to SEQ ID No. 3. Said *catA2* gene may comprise a sequence corresponding to SEQ ID No. 4.

In view of the foregoing, the host cell may thus comprise:
i) at least one (optionally heterologous) *catA* gene encoding a catA polypeptide comprising a sequence corresponding to SEQ ID No. 1; and
ii) at least one (optionally heterologous) *catA2* gene encoding a catA2 polypeptide comprising a sequence corresponding to SEQ ID No. 3

Operably linked to, e.g. upstream of, the at least one (optionally heterologous) gene, e.g. operably linked to the *catA* gene and/or operably linked to the *catA2* gene, the host cell may comprise a promoter sequence corresponding to
i) SEQ ID No. 5 [Pem7]; or
ii) SEQ ID No. 6 [Pem7*]; or
iii) SEQ ID No. 7 [Ptuf]; or
iv) SEQ ID No. 8 [PrpoD]; or
v) SEQ ID No. 9 [Plac]; or
vi) SEQ ID No. 10 [PgyrB]; or
vii) SEQ ID No. 11; or
viii) SEQ ID No. 12; or
ix) SEQ ID No. 13; or
x) SEQ ID No. 14; or
xi) SEQ ID No. 15; or
xii) SEQ ID No. 16; or
xiii) SEQ ID No. 88 [Ptuf_1]; or
xiv) SEQ ID No. 89 [Ptuf_short]; or
xv) SEQ ID No. 90 [Ptuf_s_2]; or
xvi) SEQ ID No. 91 [Ptuf_s_3]; or
xvii) SEQ ID No. 92 [Ptuf_s_4]; or
xviii) SEQ ID No. 93 [Ptuf_s_5]; or
xix) SEQ ID No. 94 [Ptuf_s_6]; or
xx) SEQ ID No. 95 [Ptuf_s_7]; or
xxi) SEQ ID No. 96 [Ptuf_s_8]; or
xxii) SEQ ID No. 97 [Ptuf_s_9]; or
xxiii) SEQ ID No. 98 [Ptuf_s_10]; or
xxiv) SEQ ID No. 99 [Ptuf_s_11]; or
xxv) SEQ ID No. 100 [Ptuf_s_12]; or
xxvi) SEQ ID No. 101 [Pgro]; or
xxvii) SEQ ID No. 102 [Pgro_1]; or
xxviii) SEQ ID No. 103 [Pgro_2]; or
xxix) SEQ ID No. 104 [Pgro_4]; or
xxx) SEQ ID No. 105 [Pgro_5].

Promoter sequences corresponding to SEQ ID Nos. 88-100 relate to derivatives of Ptuf with increased activity compared to the original Sequence, created by random mutagenesis as described herein. Promoter sequences corresponding to SEQ ID Nos. 102-105 relate to derivatives of Pgro with increased activity compared to the original Sequence, created by random mutagenesis as described herein.

It is envisaged that the host cell may express the at least one (optionally heterologous) gene, which may be a (optionally heterologous) *catA* gene and a (optionally heterologous) *catA2* gene, constitutively.

The host cell may further be characterized in that it does not express a functional catB polypeptide, a functional catC polypeptide and/or a functional pcaB polypeptide. This may be accomplished by the *catB* gene, *catC* gene and/or *pcaB* gene being for instance silenced, preferably knocked-down or knocked-out, or deleted from the chromosome.

Further provided herein is a host cell for the production of cis,cis-muconic acid from catechol which host cell comprises
i) at least one (optionally heterologous) *catA* gene;
ii) and at least one (optionally heterologous) *catA2* gene

Said at least one (optionally heterologous) *catA* gene is envisaged to encode for a catA polypeptide comprising a sequence corresponding to SEQ ID No. 1. The *catA* gene may comprise a sequence corresponding to SEQ ID No. 2. Said at least one (optionally heterologous) *catA2* gene is envisaged to encode for a catA2 polypeptide comprising a sequence corresponding to SEQ ID No. 3. The *catA2* gene may comprise a sequence corresponding to SEQ ID No. 4.

Said host cell may further comprise, operably linked to, e.g. upstream of, the at least one (optionally heterologous) *catA* gene and/or *catA2* gene a promoter sequence corresponding to
i) SEQ ID No. 5 [Pem7]; or
ii) SEQ ID No. 6 [Pem7*]; or
iii) SEQ ID No. 7 [Ptuf]; or
iv) SEQ ID No. 8 [PrpoD]; or
v) SEQ ID No. 9 [Plac]; or
vi) SEQ ID No. 10 [PgyrB].
vii) SEQ ID No. 11; or
viii) SEQ ID No. 12; or
ix) SEQ ID No. 13; or
x) SEQ ID No. 14; or
xi) SEQ ID No. 15; or
xii) SEQ ID No. 16; or
xiii) SEQ ID No. 88 [Ptuf_1]; or
xiv) SEQ ID No. 89 [Ptuf_short]; or
xv) SEQ ID No. 90 [Ptuf_s_2]; or
xvi) SEQ ID No. 91 [Ptuf_s_3]; or
xvii) SEQ ID No. 92 [Ptuf_s_4]; or
xviii) SEQ ID No. 93 [Ptuf_s_5]; or
xix) SEQ ID No. 94 [Ptuf_s_6]; or
xx) SEQ ID No. 95 [Ptuf_s_7]; or
xxi) SEQ ID No. 96 [Ptuf_s_8]; or
xxii) SEQ ID No. 97 [Ptuf_s_9]; or
xxiii) SEQ ID No. 98 [Ptuf_s_10]; or
xxiv) SEQ ID No. 99 [Ptuf_s_11]; or
xxv) SEQ ID No. 100 [Ptuf_s_12]; or
xxvi) SEQ ID No. 101 [Pgro]; or
xxvii) SEQ ID No. 102 [Pgro_1]; or
xxviii) SEQ ID No. 103 [Pgro_2]; or
xxix) SEQ ID No. 104 [Pgro_4]; or
xxx) SEQ ID No. 105 [Pgro_5].

The host cell may be further characterized in that it does not express a functional *catB* polypeptide; and/or does not express a functional *catC* polypeptide, and/or does not express a functional *pcaB* polypeptide. Thus, the host cell may further not comprise a functional *catB* gene; and/or does a functional *catC* gene, and/or a functional *pcaB* gene.

The host cell may be selected from the group consisting of bacteria, yeast, filamentous fungi, cyanobacteria, algae, and plant cells. In particular, the host cell may be selected from *Pseudomona,* preferably *Pseudomonas putida,* more preferably *Pseudomonas putida* strain KT2440. In case the host cell is selected from another type of cell, and comprises at least one (optionally heterologous) *catA* gene and at least one (optionally heterologous) catA2 gene, said catA gene and/or catA2 gene may be derived from *Pseudomonas,* preferably *Pseudomonas putida,* more preferably *Pseudomonas putida* strain KT2440.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

The term "less than" or "greater than" includes the concrete number. For example, less than 20 means less than or equal to. Similarly, more than or greater than means more than or equal to, or greater than or equal to, respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

### DESCRIPTION OF THE FIGURES

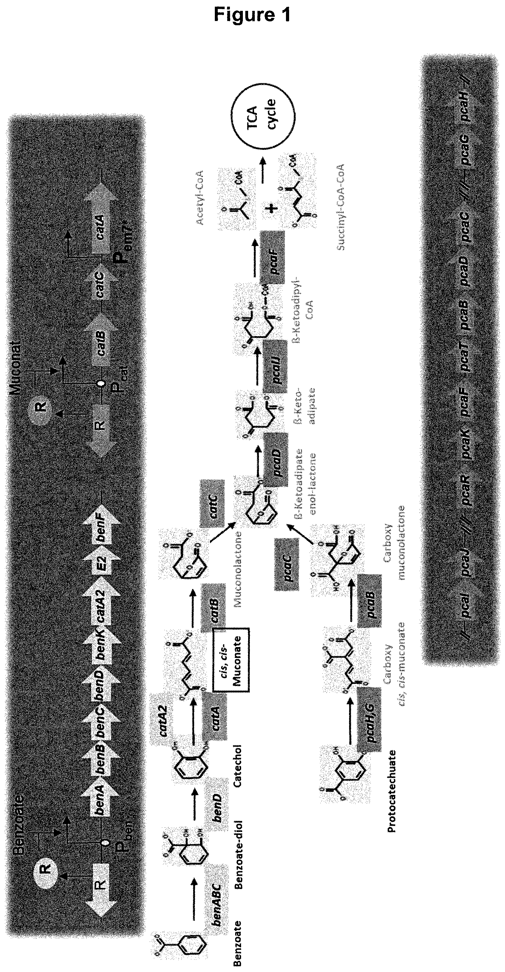

**Figure 1** shows the reaction pathway of catechol conversion in P. putida KT2440.

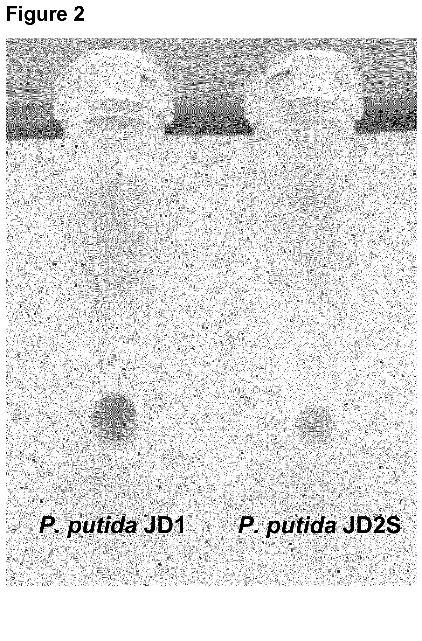

**Figure 2** shows the product of biocatalytic conversion of catechol, cis-cis-muconic acid, obtained by the lignin processing method as described herein. Cultivation of *Pseudomonas putida* strain JD1 in the presence of catechol may result in catechol accumulation and polymerization due to the sole expression of catA2, resulting in yellow and sometimes dark coloration of the medium (left). In contrast, use of *Pseudomonas putida* strains JD2S or BN6 expressing catA and catA2 did not result in accumulation of catechol, thus yielding a product white in color (right).Medium color absorbance at 600 nm (visible light, A600) is up to three times less intense in culture broths using *P. putida* JD2S or BN6 compared to P. putida JD1; e.g. A600,JD1 is 0.063, whereas A600,JD2s is only 0.022.

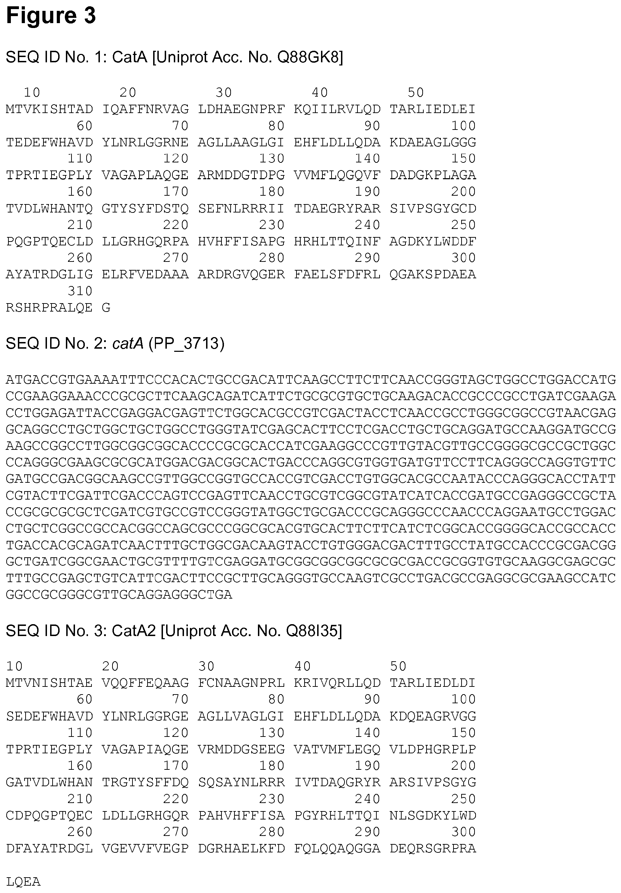

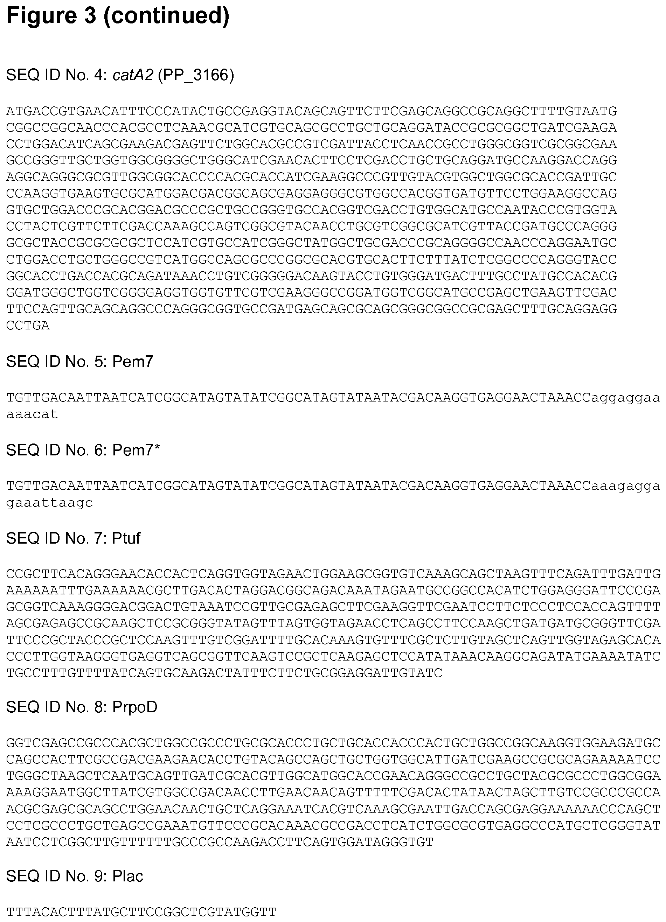

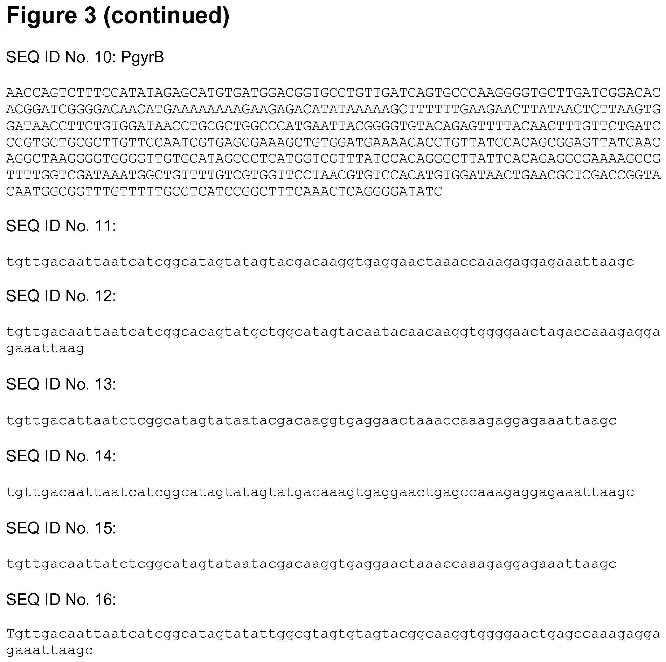

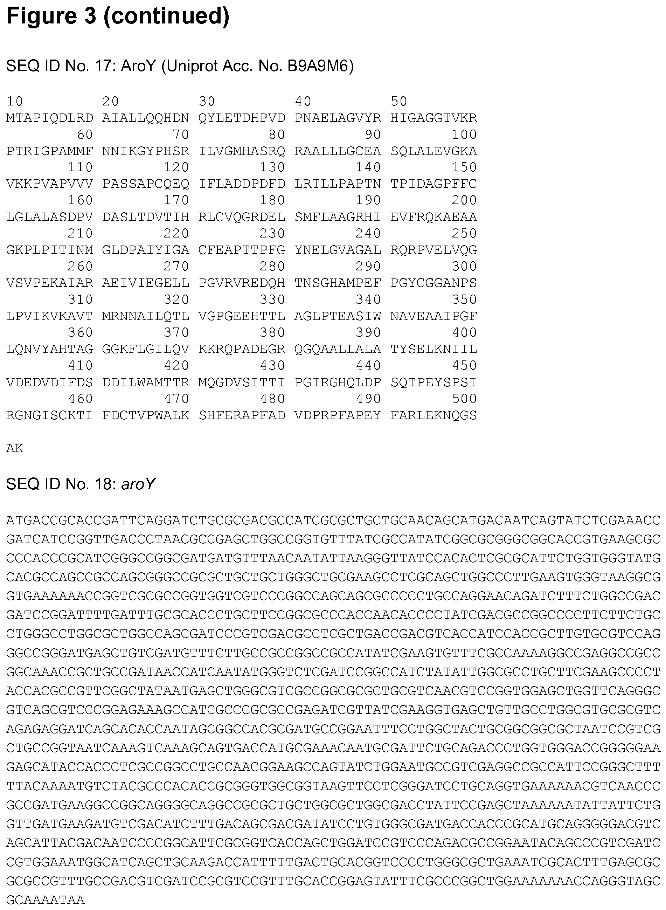

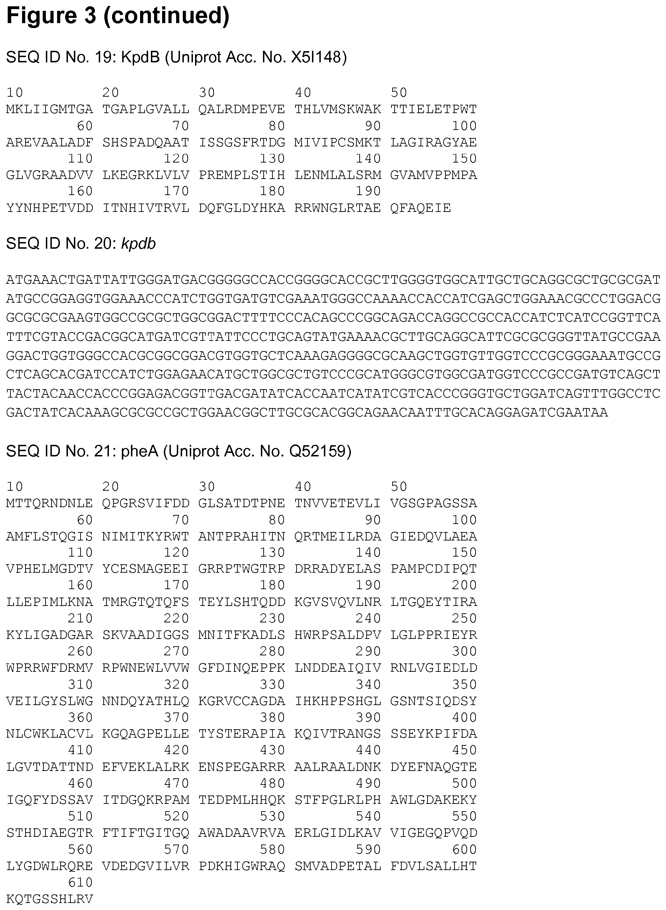

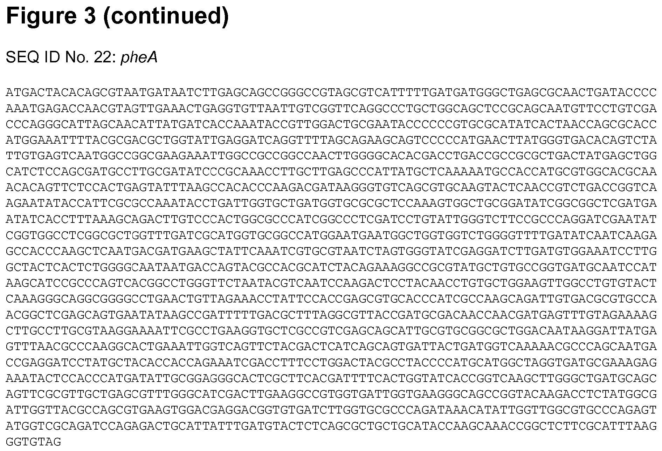

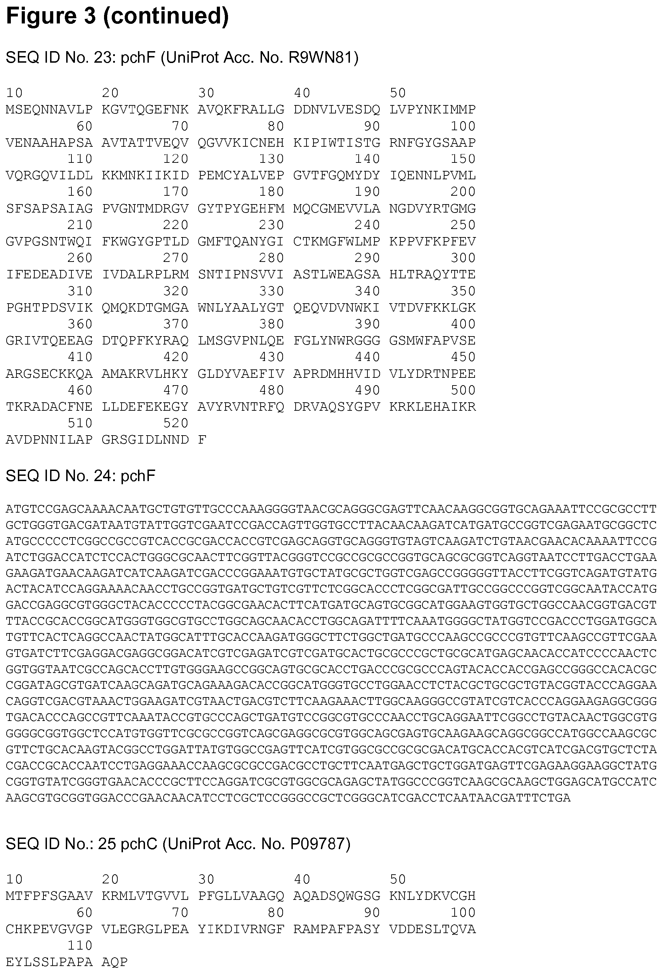

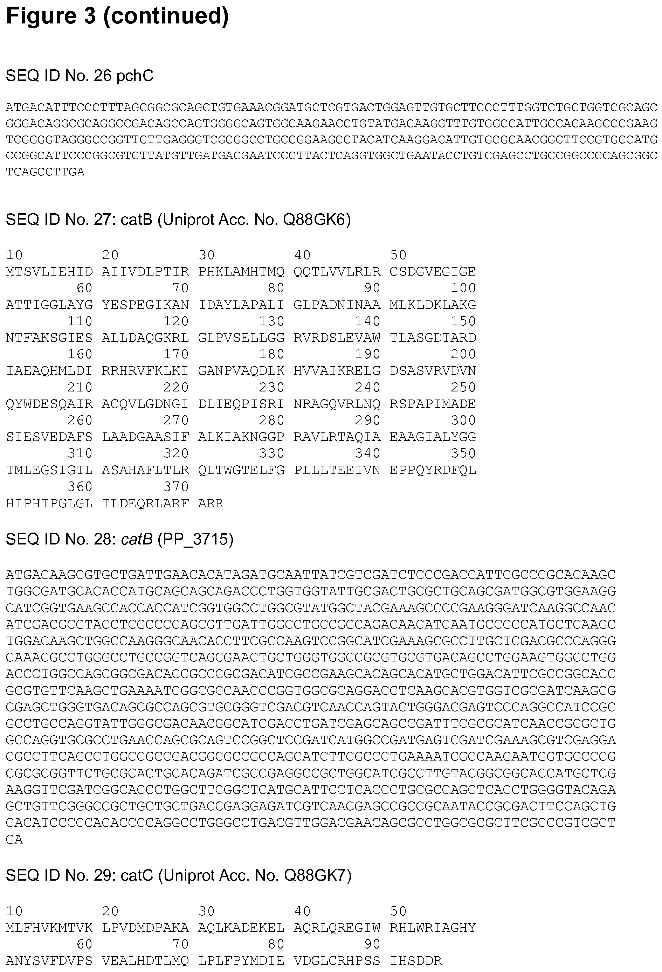

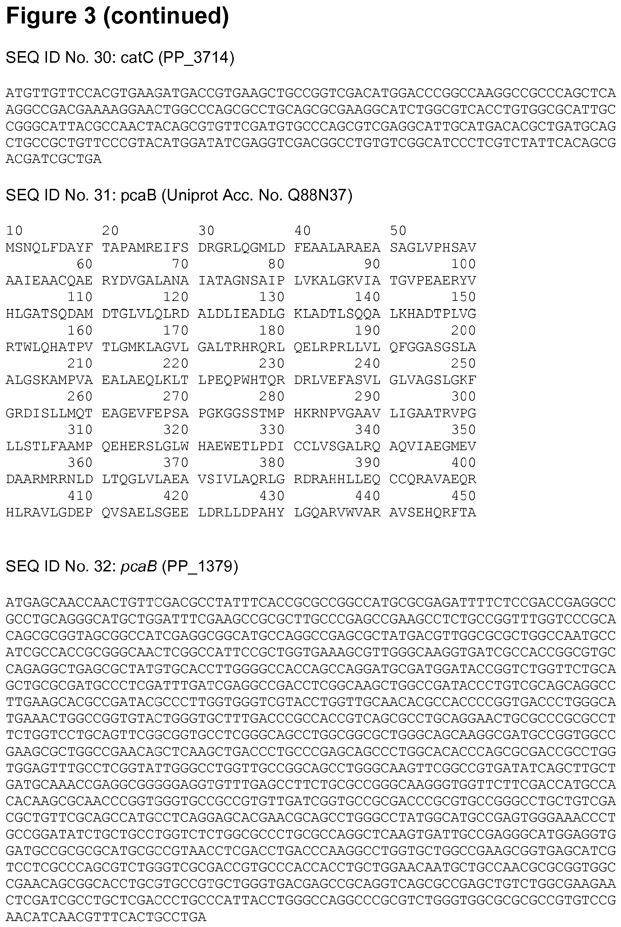

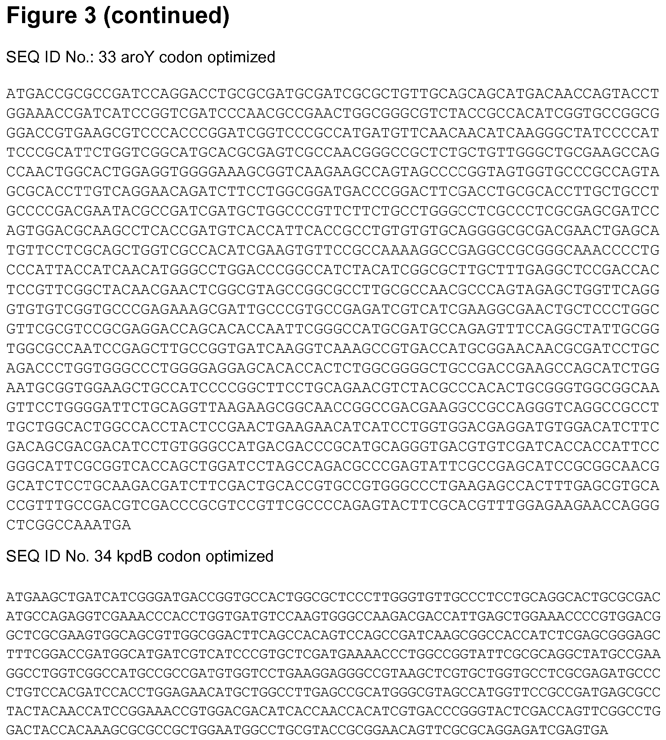

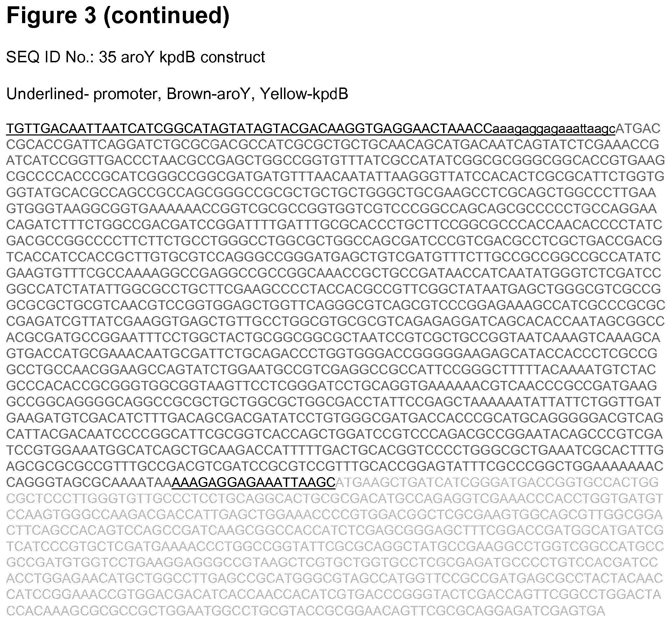

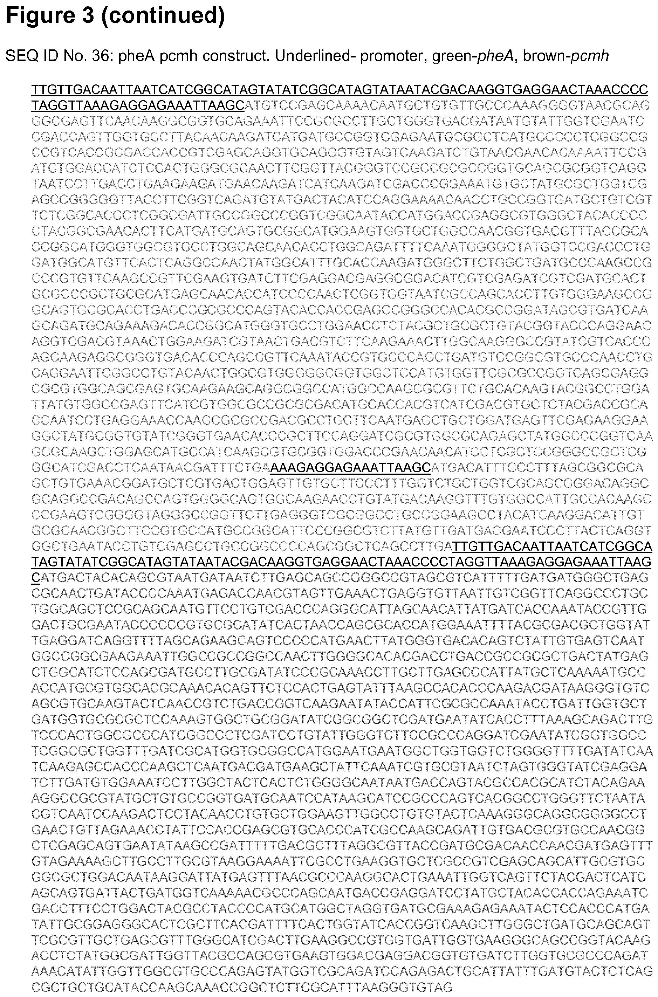

**Figure 3** is a depiction of SEQ ID No.1 to SEQ ID No. 16 referenced herein.
**Figure 4****:** Composition of the HTC liquid phase and the remaining solution after distillation at four different temperatures.
**Figure 5****:** Concentrations of catechol, phenol, guaiacol and o-,p-, m-cresol (cresol) after hydrothermal conversion at different temperatures in °C (first number of labeling on x-axis) and water density in g/cm³ (second number of labeling on x-axis).
**Figure 6****:** Yields of catechol and the sum of catechol, phenol, guaiacol and o, p, m-cresol after hydrothermal conversion at different temperatures in °C (first number of labeling on x-axis) and water density in g/cm³ (second number of labeling on x-axis).
**Figure 7****:** 3D-plot showing the outcome of the DoE experiment, which represents the relation between the yield of catechol in % w/w, and the temperature in °C and the water density in g/cm³ during the hydrothermal conversion of lignin.
**Figure 8****:** Concentrations of catechol, phenol, guaiacol and o-, p, m-cresol (cresol) after hydrothermal conversion at 400 °C and 0.50 g/cm³ water density with various retention times using Kraft lignin from Sigma Aldrich, USA.
**Figure 9****:** Promoter library for constitutive gene expression of Ptuf and Pgro in P. putida. The activity of the promoter is measured as RFU per OD600. The data are the means and standard deviations of results from three independent experiments. The last three balks indicate negative controls (medium control, P. putida Wt KT2440, empty vector pSEVA247R).
**Figure 10****:** Specific catechol conversion rates (mmol g-1 h-1; indicated as bars) and catechol 1,2-dioxygenase activities (U mg-1; indicated as lines) in selected producer strains. In all producer strains, a complete conversion of catechol to the product cis, cis-muconic acid within diverse periods could be detected. The data are the means and standard deviations of results from three independent experiments.

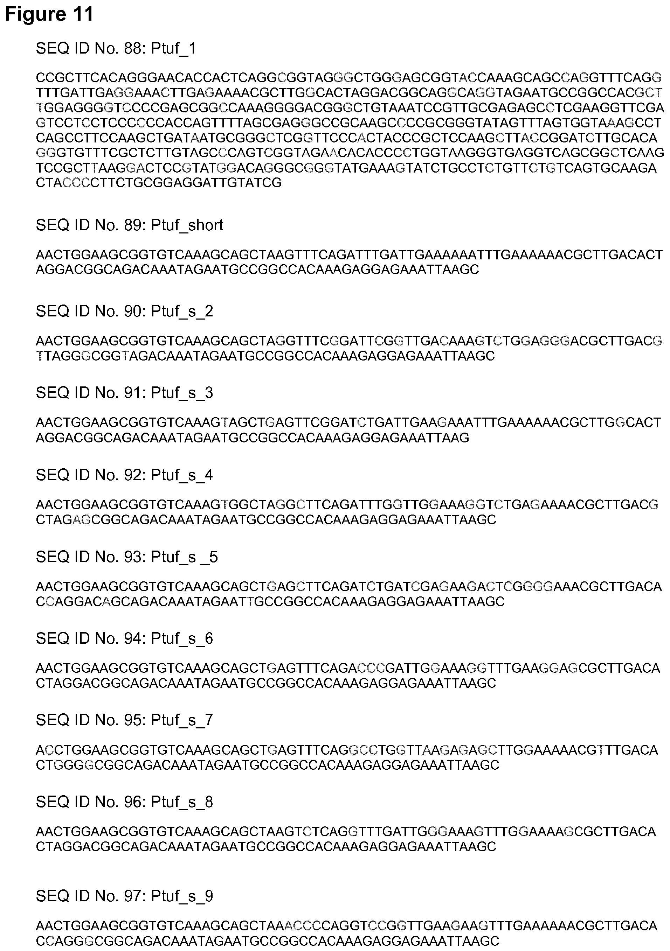

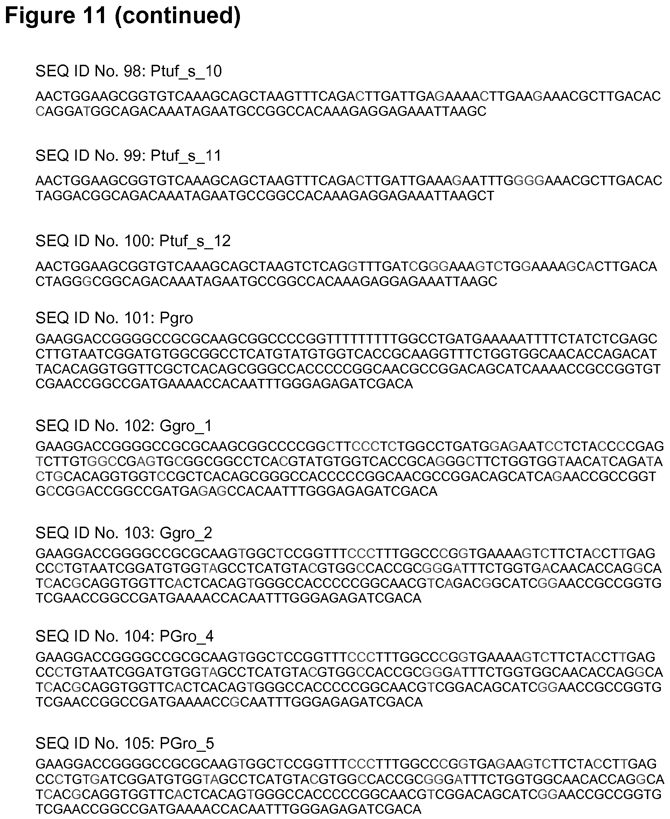

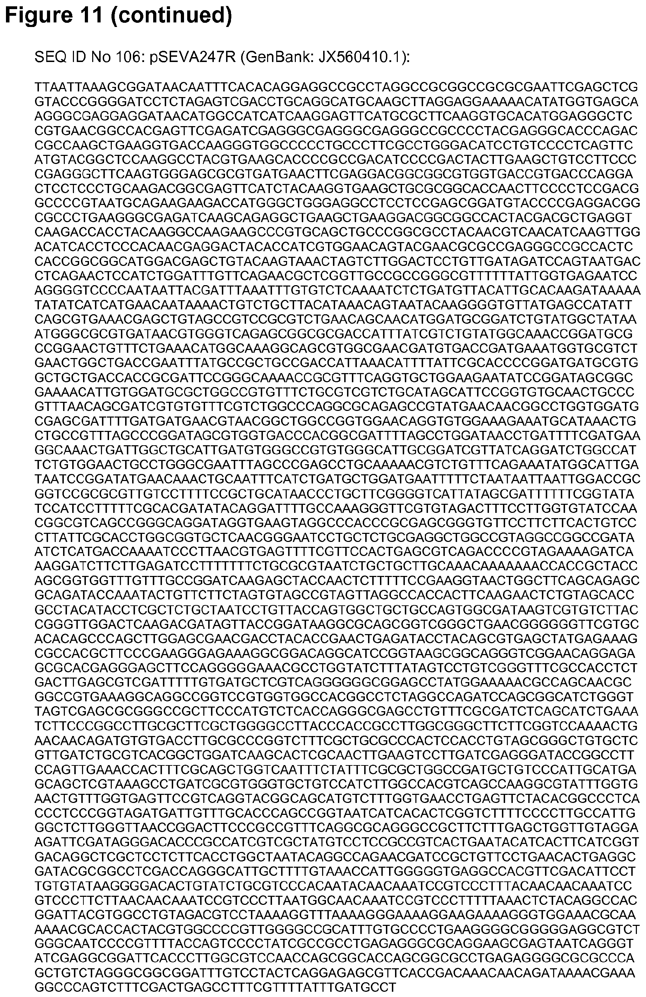

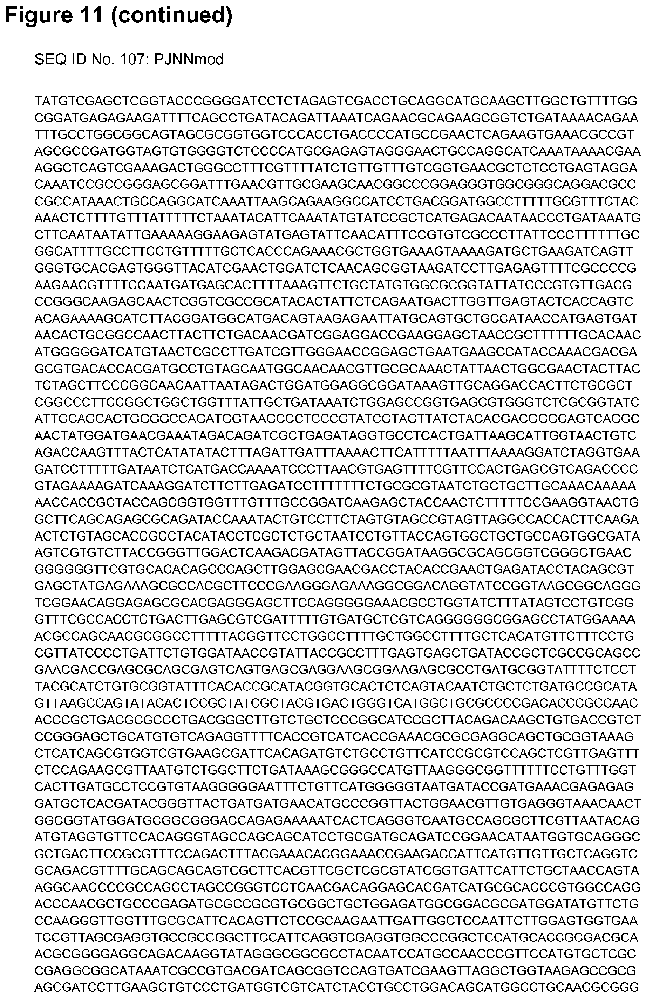

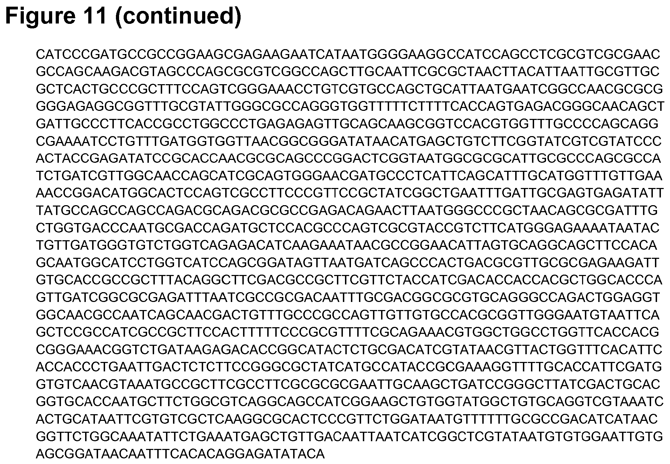

**Figure 11** is a depiction of SEQ ID No.88 to SEQ ID No. 107 referenced herein.

### DETAILED DESCRIPTION

The efficient conversion organic feedstock to useful organic compounds will be critical to address the emerging dilemma for an ever increasing global population while minimizing environmental degradation. Owing to the massive amounts of organic biomass available from a plethora of sources, establishment of organic feedstock conversion opens a new route for the production of useful organic compounds. The present inventors have, for the first time, recognized the potential of coupling a method of decomposition of organic feedstock such as lignin, more specifically by fluid-assisted conversion of the same under sub- and/or supercritical conditions, with biocatalytic conversion of the intermediate products obtained therefrom. This approach is new and advantageous in that it achieves a high yield of useful organic end products from a biomass feedstock on a reproducible basis and can potentially be conducted with suitable genetically modified or non-genetically modified biocatalysts, advantageously resulting in a high yield and purity of organic end product obtainable under high reaction rates, and hence easy and efficient production.

In accordance with the foregoing, the present invention provides a method of producing an organic product, said method comprising the steps of
(i) sub- and/or supercritical fluid-assisted conversion of an organic educt;
(ii) obtaining an intermediate product from step i);
(iii) subjecting the intermediate product from step ii) to biocatalytic conversion.

The present inventors provide a novel method of processing, e.g., complex and/or polymeric organic feedstock that may commonly be seen as waste material or a useless or low-value by-product of processing other organic compounds into useful organic compounds. The method of the present invention advantageously yields extraordinary high concentrations of the final product. By way of example, in case catechol was used as an intermediate product, the present inventors were able to yield cis,cis-muconic acid concentrations of more than 60 g/l.

### Sub- and/or supercritical fluid-assisted decomposition

The term "sub- and/or supercritical fluid-assisted conversion" as used herein to refer to the chemical conversion of organic compounds in sub- und supercritical fluids acting as solvents. Depending on the type of organic feedstock being subjected to sub- and/or supercritical fluid-assisted conversion, and the reaction conditions, the term may also involve decomposition of polymers into their multi- and/or monomeric constituents (also referred to as "depolymerisation" herein). An exemplary protocol for supercritical fluid-assisted conversion employing supercritical water as a solvent is described in the appended examples. "Hydrothermal conversion" refers to conversion of organic compounds with sub- and/or supercritical water as a solvent. Generally, besides water other fluids can be used as sub- and/or supercritical solvents, including: CO₂, methane, ethane, propane, ethylene, propylene, methanol, ethanol, acetone, 2-propanol, acetic acid, formic acid, and nitrous oxide. The skilled person will readily be able to select suitable solvents depending on the organic educt, desired (intermediate) product, reaction conditions, chemical extraction, toxicity, and environmental impact.

"Hydrothermal conversion" in general comprises introducing an organic educt and an effective amount of water into a suitable reaction vessel, operated at a temperature from about 200° C to about 500° C, at a pressure greater than the saturated water vapor pressure within the reaction vessel, and at a suitable residence time (also referred to as "retention time" or "reaction time" herein), thereby resulting in the conversion of the organic educt into one or more intermediate products. Fluid-assisted conversion under sub- and/or supercritical conditions may also involve stirring of the reactor contents.

The critical point for pure water is 374 °C (647,1 K) and 22,1 MPa. Above this temperature and pressure, water is in its supercritical phase. Without wishing to be bound by theory, it is thought that above its critical point, physical properties of water drastically change. The dielectric constant and ion product of water can be changed based on variations in water density and temperature. Above its supercritical point, the dielectric constant of water decreases further as well as the ion product. Water is thought to start behaving like an organic, non-polar solvent which results in poor solubility for inorganics, and complete miscibility with gases and many hydrocarbons. Due to this miscibility, phase boundaries do not exist anymore or are substantially reduced. This absence is thought to lead to fast and complete homogeneous reactions of water with organic compounds, such as the organic educts exemplified herein.

The change in physical properties of water in its supercritical phase is thought to cause water to act as a solvent as well as a catalyst, and, through hydrolysis reactions, also as a reactant.

The use of subcritical fluids, e.g. subcritical water, as a solvent in the fluid-assisted decomposition step of the present invention is also envisaged herein. E.g., for fluid-assisted decomposition of lignin in subcritical water, reaction conditions described in Pinkowska et al. Chem Eng J. 2012, 187: 410-414 can be applied. That is, the reaction temperature may be 250°C and above, such as about 260°C, about 270°C, about 280°C, about 290°C, about 300°C, about 310°C, about 320°C, about 330°C, about 340°C, and about 350°C. The pressure may be 5 Mpa or higher, such as about 10 MPa, about 15 MPa, about 20 MPa, or about 25 MPa. For fluid-assisted decomposition of lignin in supercritical water, reaction temperatures of 350°C and above are envisaged, such as about 360°C, about 370°C, about 380°C, about 390°C, about 400°C, about 410°C, about 420°C, about 430°C, about 440°C, about 450°C, about 460°C, about 470°C, about 480°C, about 490°C, about 500°C, about 510°C, about 520°C, about 530°C, about 540°C, about 550°C, about 560°C, about 570°C, about 580°C, about 590°C, or about 600°C. The pressure may be 25 Mpa or higher, such as about 30 MPa, about 35 MPa, about 40 MPa, about 45 MPa, or about 50 MPa. Other parameters for sub- and supercritical water have been reviewed in Toor SS et al. Energy 2011; 36: 2328-42. Further parameters for sub- and supercritical water for the decomposition of lignin have been disclosed by Wahyudiono et al (Chemical Engineering and Processing; 2008, vol. 47, p. 1609-1619) resulting in the generation of more than 28 wt% catechol. The skilled person will readily be able to select suitable reaction conditions, preferably resulting in a high yield of desired intermediate products.

The skilled artisan will readily understand that the exact reaction conditions will vary depending on the organic educt subjected to sub- and/or supercritical fluid-assisted conversion, the size and properties of the reaction container, and the desired nature and yield of intermediate product that is to be obtained. Reaction conditions can be adjusted, e.g. by the addition of salts, solvents (e.g. methanol, phenol or p-cresol), different concentrations of organic educt (e.g. lignin), and various retention times.

The present invention is considered to be particular advantageous for converting lignin into cis-cis-muconic acid via catechol. This process is also termed "lignin processing" hereinafter. A preferred protocol for the first step in lignin processing, i.e. lignin conversion using sub- and/or supercritical fluids, is described in the following. The present inventors have discovered that in order to convert the organic educt lignin into the intermediate product catechol, reaction temperatures 300°C and above, such as about 320°C, about 340°C, about 360°C, about 380°C, about 400°C, about 420°C, about 440°C about 460°C or about 470°C may be favorable. Reaction temperatures of between about 350°C and about 420°C may be particularly preferred. The reactor contents may be stirred, e.g. at about 150 rpm. The skilled person will acknowledge that reaction conditions, including e.g. the residence time, concentration of organic educt, addition of salts and/or solvents may be adjusted in order to increase catechol yield and decrease the production of unwanted organic by-products. The skilled person will readily be able to adjust the retention time in order to obtain a desired amount of catechol, e.g. depending on the size of the reactor. A steady process is also conceivable. Exemplary retention times at sub and/or supercritical conditions applied in the methods of the invention may in general be between 10 and 160 min. E.g., overall reaction times (including heating and cooling of the reactor) include for instance reaction times between 0,5 hours and 4 hours, such as between 1 hour and 3,5 hours, 1,5 hours and 3 hours, e.g. 2 hours (for instance with heat-up time 1 hour, maintenance of reaction temperature 30 min ("reaction phase"), cooling down 30 min). In general, altered reaction conditions, including altered residence time, reaction time and reaction temperature are also conceivable, for example a reduction in retention time before and after the reaction phase (i.e. shortened heating and cooling time of the reactor before and after the hydrothermal conversion), a reduction or increase of the reaction time itself, an increase in temperature and/or water density, and/or addition of salts and solvents.

The addition of salts to the reactor may shift the reaction equilibrium towards the intermediate compounds. Illustrative examples for useful salts that can be added to the reaction include alkali salts, e.g. Na₂SO₄, NaCl, KCI, CaCl₂, and CaSO₄.

Further, catalysts such as CaO, NaHCO₃, RbOH, CsOH, LiOH, Ca(OH)₂, CaCO₃, Na₂CO₃, K₂CO₃, KOH, Ni, ZrO₂, H₂SO₄, TiO₂, ZrO₂, Ru, Pt, Rh, Pd FeCl₃, and/or NiCl₂, NaOH, HCl can be added. Addition of hydrogen donor solvents such as tetralin, ethyl acetate, coal tar and reducing gas such as and H₂, CO, and Ar can further be applied to increase the liquid reaction product.

### Organic educt

One of the most important benefits of the means and methods of the invention is that they can be applied to a great variety of organic educts. Biomass and its constituents are particularly envisaged for use as feedstock in the methods of the invention and generally include biological material derived from living, or recently living organisms, such as waste from wood processing industry (e.g. sawdust, cut-offs, bark, etc), waste from paper and pulp industry, agricultural waste (palm oil residues, rice husks, sugarcane, coconut shells, coffee & cocoa husks, cotton & maize residues, etc.), organic waste (animal manure, food processing wastes), urban wood waste (wooden pallets, packing material, etc.), wastewater and landfill (municipal sewage, landfill gas, etc.) and other natural resources (plants, meat, straw, peat, bagasse, clover grass, sewage sludge, pinewood, wheat stalk, sorghum stark and other compounds etc.). While it is in general possible and envisaged herein to subject any of the aforementioned biomass resources to the inventive method as described herein, the use constituents isolated from biomass may be advantageous when production of a certain intermediate product with few by-products is desired. Biomass constituents envisaged for use according to the methods of the invention include, without limitation, lignocellulose, lignin, guaiacol, p-coumaryl, coniferyl, sinapyl alcohols, catechol, phenol, m-cresol, p-cresol, o-cresol, cellulose, hemicellulose, starch, glucose, fructose, xylose, triacylglycerides, fatty acids, proteins, amino acids and derivatives thereof.

### Cellulose

Cellulose is a polysaccharide composed of units of glucose. The basic repeating unit of the cellulose polymer consists of two glucose anhydride units, called a cellobiose unit. Unlike starch, the glucose monomers are connected via β-(1/4)-glycosidic bonds, which allows strong intra- and inter-molecular hydrogen bonds to form, and makes them crystalline, resistant to swelling in water, and resistant to attack by enzymes. Cellulose derivatives such as carboxymethylcellulose are also encompassed by the term.

### Hemicellulose

Hemicellulose is a heteropolymer composed of sugar monomers, including xylose, mannose, glucose, galactose and others, which can also have side chains. In comparison to cellulose, hemicellulose consists of various polymerized monosaccharides including five-carbon sugars (usually xylose and arabinose), six-carbon sugars (galactose, glucose, and mannose), and 4-O-methyl glucuronic acid and galacturonic acid residues. The ratios of these monomers can change quite dramatically for different feedstock sources.

Given the lack of repeating β-(1/4)-glycosidic bonds and the random nature of the hemicellulose polymer, it does not form as crystalline and resistant of a structure as cellulose does, and thus is much more susceptible to hydrothermal extraction and hydrdysis.

### Starch

Starch is a polysaccharide consisting of glucose monomers bound with α-(1/4) and α-(1/6) bonds.

### Triacylglycerides

Fats and oils in biological systems are typically in the form of triacylglycerides (TAGs, also termed "triglycerides"), which consist of three fatty acids bound via ester linkages to a glycerol backbone. The term comprises saturated and unsaturated triacylglyerides.

### Lignin

It is particularly envisaged herein to provide means and methods for further processing of lignin. Lignin is a cross-linked amorphous copolymer synthesized from random polymerization of aromatic monomers, in particular the three primary phenylpropane monomers p-coumaryl alcohols, coniferyl alcohols, and sinapyl alcohols containing zero, one, and two methoxyl groups, respectively. An exemplary lignin structure is shown in formula (1)., However, the exact structure may vary depending on the source and pretreatment of the compound.

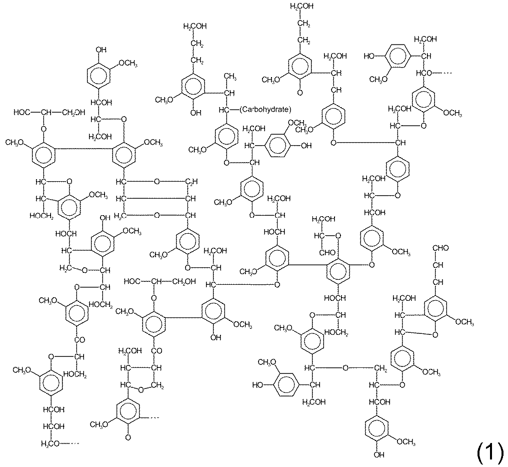

The term "lignin" includes naturally occurring lignin (a water-insoluble macromolecule comprised of three monolignol monomers: p-coumaryl alcohol, coniferyl alcohol, and sinapyl alcohol) and also processed lignin derivatives, for example the following compounds obtainable from Sigma-Aldrich: alkali lignin (CAS Number 8068-05-1), organosolv lignin (CAS Number: 8068-03-9), hydrolytic lignin (CAS Number: 8072-93-3) lignosulfonic acid sodium salt (CAS Number: 8061-51-6) and guaiacol (CAS Number: 90-05-1).

The skilled person will readily understand that when subjecting a complex organic compounds, such as an organic polymer (like lignin) to sub- and/or supercritical fluid-assisted conversion, the compound will decompose during the reaction and release its (e.g. mono- or dimeric) constituents which will also be subjected to conversion in the sub- or supercritical fluid as long as the reaction is not stopped. Hence, the intermediate products described in the following are also envisaged as organic educts being subjected to sub- and/or supercritical fluid-assisted conversion.

### Intermediate product

Sub- and/or supercritical fluid-assisted conversion of an organic educt is envisaged herein to yield a liquid reaction product comprising the desired intermediate product intended for biocatalyzation and optionally further by-products, solvents and remaining organic educt. The relative amount of desired intermediate product in the reaction product may vary depending on the organic educt and the reaction conditions.

E.g., for various lignin compounds, catechol is envisaged to be present in the liquid reaction product in an amount of 5% w/w or more, such as 10% w/w, 15% w/w, 20% w/w, 25% w/w, 30% w/w, 35% w/w, 40% w/w, 45% w/w, 50% w/w, 55 % w/w, 60% w/w, 70% w/w, 80% w/w, 85% w/w, 90% w/w, 95% w/w or 100% w/w. Further components may be phenol, cresol and/or guaiacol. The term "cresol" as used herein generally comprises m-cresol, p-cresol and o-cresol. For instance, supercritical fluid-assisted conversion of guaiacol has been reported to yield up to 90% catechol in the liquid reaction product. It is in principle also conceivable to modify the methods of the invention for biocatalytic conversion of lignin via the intermediate product phenol. Then, phenol is envisaged to be present in the liquid reaction product in an amount of 5% w/w or more, such as 10% w/w, 15% w/w, 20% w/w, 25% w/w, 30% w/w, 35% w/w, 40% w/w, 45% w/w, 50% w/w, 55 % w/w, 60% w/w, 70% w/w, 80% w/w, 85% w/w, 90% w/w, 95% w/w or 100% w/w. Other components may include catechol, guaiacol and/or cresol. Further components may be phenol and/or cresol. It is in principle also conceivable to modify the methods of the invention for biocatalytic conversion of lignin via the intermediate product cresol. Then, cresol is envisaged to be present in the liquid reaction product in an amount of 5% w/w or more, such as 10% w/w, 15% w/w, 20% w/w, 25% w/w, 30% w/w, 35% w/w, 40% w/w, 45% w/w, 50% w/w, 55 % w/w, 60% w/w, 70% w/w, 80% w/w, 85% w/w, 90% w/w, 95% w/w or 100% w/w. Other components may include catechol, guaiacol and/or phenol. It is in principle also conceivable to modify the methods of the invention for biocatalytic conversion of lignin via the intermediate product guaiacol. Then, guaiacol is envisaged to be present in the liquid reaction product in an amount of 5% w/w or more, such as 10% w/w, 15% w/w, 20% w/w, 25% w/w, 30% w/w, 35% w/w, 40% w/w, 45% w/w, 50% w/w, 55 % w/w, 60% w/w, 70% w/w, 80% w/w, 85% w/w, 90% w/w, 95% w/w or 100% w/w. Other components may include catechol, cresol and/or phenol.

It is envisaged that the step of obtaining an intermediate product from step (i) of the method of the invention may involve separating said intermediate product from the liquid reaction product of sub- and/or supercritical fluid-assisted conversion. Separation of said intermediate product includes complete separation (i.e. purification), and partial separation of said product. "Complete separation" means that a product is yielded in essentially pure form (i.e. without the presence of other by-products or solvents). "Partial separation" means that other by-products or solvents are present.

The step of ii) obtaining an intermediate product from sub- and/or supercritical fluid-assisted conversion of the organic educt may involve a variety of process steps depending on the characteristics of the intermediate product to be recovered, the presence and nature of potential by-products and the desired purity of the intermediate product. E.g., obtaining the intermediate product may involve distillation of the reaction product obtained after sub- and/or supercritical fluid-assisted conversion. As it is well-known in the art, distillation is a process of separating components from a liquid mixture by selective evaporation and condensation. Distillation includes e.g. simple distillation, fractional distillation, steam distillation (also referred to as "steam bath distillation" herein). Steam bath distillation may be accomplished as set out in the appended examples. Other methods for separating the intermediate product and/or by-product and/or salts from other components of the reaction (e.g. catalyst, solvent, and/or remaining educt) are also conceivable, and include, e.g., filtration (such as vacuum filtration, for instance using PTFE membranes), affinity chromatography, ion exchange chromatography, solvent extraction, filtration, centrifugation, electrophoresis, hydrophobic interaction chromatography, gel filtration chromatography, chromatofocusing, differential solubilization, preparative disc-gel electrophoresis, isoelectric focusing, HPLC, reverse-phase HPLC, and countercurrent distribution.

Intermediate products obtained in step (ii) of the inventive method may vary depending on the organic educt and reaction parameters. Some exemplary intermediate products envisaged for further biocatalyzation according to the inventive method are listed in the following.

When using cellulose as a feedstock for sub- and/or supercritical fluid-assisted decomposition, the intermediate product may be selected from one or more of the following: Glucose, Fructose, 5-(Hydroxymethyl)furfural (5-HMF), Glycolaldehyde, Glyceraldehyde, Dihydroxyacetone, 1,6-Anhydroglucose,Erythrose, Pyruvaldehyde, 2-furaldehyde, Acetic acid, Formic acid, Lactic acid, Acrylic acid, 1,2,4-Benzenetriol, 4-oxopentanoic acid, o-, m-, or p-xylene, ethylbenzene, n-propylbenzene, 1-methyl-2-ethylbenzene, 3-ethylbenzene, Phenol, o-,m-,p-cresol, 2-phenoxyethanol, Oligomers (cellobiose. cellotriose, cellotetraose, cellohexaose, etc.). When using hemicellulose as a feedstock for sub- and/or supercritical fluid-assisted decomposition, the intermediate product may be selected from one or more of the following: xylose, glucose, fructose, arabinose.

When using starch as a feedstock for sub- and/or supercritical fluid-assisted decomposition, the intermediate product may be selected from one or more of the following: Glucose, Fructose, 5-(Hydroxymethyl)furfural (5-HMF), Glycolaldehyde, Glyceraldehyde, Dihydroxyacetone, 1,6-Anhydroglucose,Erythrose, Pyruvaldehyde, 2-furaldehyde, Acetic acid, Formic acid, Lactic acid, Acrylic acid, 1,2,4-Benzenetriol, 4-oxopentanoic acid, o-, m-, or p-xylene, ethylbenzene, n-propylbenzene, 1-methyl-2-ethylbenzene, 3-ethylbenzene, Phenol, o-,m-,p-cresol, 2-phenoxyethanol, Oligomers (cellobiose. cellotriose, cellotetraose, cellohexaose, etc.).

When using glucose as a feedstock for sub- and/or supercritical fluid-assisted decomposition, the intermediate product may be selected from glucose, fructose, Dihydroxyacetone, Glyceraldehyde, Erythrose, Glycolaldehyde, Pyruvaldehyde, Lactic acid, 1,6-Anhydroglucose, Acetic acid, formic acid, 5-HMF, 2-furaldehyde, Acrylic acid, 1,2,4-Benzenetriol, Levulinic acid, 4-oxopentanoic acid .

When using fructose as a feedstock for sub- and/or supercritical fluid-assisted decomposition, the intermediate product may be selected from glucose, fructose, Dihydroxyacetone, Glyceraldehyde, Erythrose, Glycolaldehyde, Pyruvaldehyde, Lactic acid, 1,6-Anhydroglucose, Acetic acid, formic acid, 5-HMF, 2-furaldehyde, Acrylic acid, 1,2,4-Benzenetriol, Levulinic acid, 4-oxopentanoic acid.

When using Hemicellulose, Xylan or Xylose as a feedstock for sub- and/or supercritical fluid-assisted decomposition, the intermediate product may be selected from Xylose, Furfural, Formic acid, Glucolaldehyde, Glyceraldehyde, Dihydroxyacetone, Pyruvaldehyde, Hydroxyacetone, Lactic acid.

When using triacylglycerides as a feedstock for sub- and/or supercritical fluid-assisted decomposition, the intermediate product may be selected from Acrolein, Methanol, Acetaldehyde, Propionaldehyde, Acrolein, Allyl Alkohol, Ethanol, Formaldehyde, CO, CO2, H2, Alkanes.

When using fatty acids as a feedstock for sub- and/or supercritical fluid-assisted decomposition, the intermediate product may be selected from Alkanes.

When using proteins as a feedstock for sub- and/or supercritical fluid-assisted decomposition, the intermediate product may be selected from CO₂, CO, H₂, CH₄, Acetic acid, Propanoic acid, n-butyric acid, iso-butyric acid, iso-valeric acid.

When using amino acids or Bovine serum albumin as a feedstock for sub- and/or supercritical fluid-assisted decomposition, the intermediate product may be selected from CO₂, CO, H₂, CH₄, Acetic acid, Propanoic acid, n-butyric acid, iso-butyric acid, iso-valeric acid.

When using Valine, Leucine or Isoleucine as a feedstock for sub- and/or supercritical fluid-assisted decomposition, the intermediate product may be selected from NH3, CO2, CO, Propane, Butane, Isobutene, Isopentane, 3-methyl-1-butane, 2-methyl-1-butane, Propane, Butene, Isobutylene, Acetone, Iso-butylamine.

When using Glycine or Alanine as a feedstock for sub- and/or supercritical fluid-assisted decomposition, the intermediate product may be selected from Acetaldehyde, Acetaldehyde-hydrate, Diketopiperazine, Ethylamine, Methylamine, Formaldehydes, Lactic acid, Propionic acid.

When using Alanine as a feedstock for sub- and/or supercritical fluid-assisted decomposition, the intermediate product may be selected from NH3, Carbonic acid, Lactic acid, Pyruvic acid, Acrylic acid, Acetic acid, Propionic acid, Formic acid.

When using amino acids as a feedstock for sub- and/or supercritical fluid-assisted decomposition, the intermediate products may be selected from acid intermediates,amine compounds, acrolein, methanol, acetaldehyde, propionaldehyde, acrolein, allyl alcohol, ethanol, formaldehyde, carbon CO, CO₂, H₂, C-17 alkane, NH₃, propane, butane, isobutane, isopentane, 3-methyl-1-butene, 2-methyl-1-butene, propene, butene, isobutylene, acetone, iso-butylamine, Acetaldehyde, acetaldehyde-hydrate, diketopiperazine, ethylamine, methylamine, formaldehydes, lactic acid, propionic acid, carbonic acid, lactic acid, pyruvic acid, acrylic acid, acetic acid, propionic acid, formic acid, CH₂, and CH₄, propanoic acid, n-butyric acid, iso-butyric acid, and iso-valeric acid

When using lignin as a feedstock for sub- and/or supercritical fluid-assisted conversion, the intermediate products may be selected from guaiacol, catechol, phenol, m,p-cresol and o-cresol. Processing of lignin in sub- or supercritical fluid (e.g. water) is thought to produce smaller fragments (intermediate products) through breakage of the (ether) linkages and produce larger fragments through cross linking between the reactive fragments, predominantly by Friedel-Craft mechanism (repolymerization). Dealkylation and demethoxylation may also occur when processing lignin in a hydrothermal medium.

Notably, as mentioned previously many of the intermediate products exemplified herein also themselves constitute potential organic educts susceptible to further conversion or re-polymerization in sub- or supercritical fluids. As set out elsewhere herein, reaction parameters such as reaction temperature, pressure and reaction time, can be readily adjusted in order to shift the reaction towards favorable intermediate products.

### Biocatalyst

The intermediate product obtained in step ii) of the inventive method is subjected to biocatalytic conversion, i.e. contacted with a biocatalyst, in particular a host cell that produces the desired organic product. Contacting the intermediate product will, as will be well understood by the person skilled in the art, be conducted under conditions that allow the biocatalyst to catalyze production of the desired organic product from the intermediate product. The exact conditions, including concentration of the intermediate product, concentration and growth state of the biocatalyst, culture conditions including culture medium composition, pH, temperature, aeration, agitation and container, will depend greatly on the biocatalyst, the intermediate product and the organic product to be obtained and will be readily ascertainable by the skilled person in the art.

A host cell of the present invention includes any suitable host cell that is capable of producing the desired organic product from the intermediate product it is supplied with. The skilled person will readily acknowledge that feasibility of using a given host cell as a biocatalyst in the methods of the invention primarily depends on whether the host cell comprises the genetic constitution required to catalyze production of the desired end product. E.g., the host cell preferably expresses enzymes capable of converting the intermediate product (e.g., catechol) obtained in step (ii) of the invention into the desired organic end product (e.g., cis-cis-muconic acid). Polypeptides required for production of the desired organic end product (which may include, e.g., enzymes catalyzing the conversion and proteins required for import and/or export of the reactants into or out of the cell, respectively), will also be referred to as "polypeptides of interest" or "POI" herein. Genes encoding said polypeptides of interest are also termed "genes of interest" or "GOI" herein.

The host cell may be a prokaryotic or eukaryotic host cell and may be selected from bacteria, yeast, filamentous fungi, cyanobacteria, algae, and plant cells.

The host cell is envisaged to be a single cell organism, which is typically capable of dividing and proliferating. A host cell can include one or more of the following features: aerobe, anaerobe, filamentous, non-filamentous, monoploid, dipoid, auxotrophic and/or non-auxotrophic.

Suitable prokaryotic host cells include Gram negative or Gram positive bacteria and may be selected from, e.g., Bacillus bacteria (e.g., B. subtilis, B. megaterium), Acinetobacter bacteria, Norcardia baceteria, Xanthobacter bacteria, Escherichia bacteria (e.g., E. coli (e.g., strains DH10B, StbI2, DH5-alpha, DB3, DB3.1 ), DB4, DB5, JDP682 and ccdA-over (e.g., U.S. Application No. 09/518,188), Streptomyces bacteria, Erwinia bacteria, Klebsiella bacteria, Serratia bacteria (e.g., S. marcescens), Pseudomonas bacteria (e.g., P. aeruginosa, P. putida), Salmonella bacteria (e.g., S. typhimurium, S. typhi), Megasphaera bacteria (e.g., Megasphaera elsdenii).

Bacteria also include, but are not limited to, photosynthetic bacteria (e.g., green non-sulfur bacteria (e.g., Choroflexus bacteria (e.g., C. aurantiacus), Chloronema bacteria (e.g., C. gigateum)), green sulfur bacteria (e.g., Chlorobium bacteria (e.g., C. limicola), Pelodictyon bacteria (e.g., P. luteolum), purple sulfur bacteria (e.g., Chromatium bacteria (e.g., C. okenii))), and purple non-sulfur bacteria (e.g., Rhodospirillum bacteria (e.g., R. rubrum), Rhodobacter bacteria (e.g., R. sphaeroides, R. capsulatus), and Rhodomicrobium bacteria (e.g., R. vanellii)).

Any suitable yeast may be selected as a host cell, including without limitation Yarrowia yeast (e.g., Y. lipolytica (formerly classified as Candida lipolytica)), Candida yeast (e.g., C. revkaufi, C. pulcherrima, C. tropicalis, C. utilis), Rhodotorula yeast (e.g., R. glutinus, R. graminis), Rhodosporidium yeast (e.g., R. toruloides), Saccharomyces yeast (e.g., S. cerevisiae, S. bayanus, S. pastorianus, S. carlsbergensis), Cryptococcus yeast, Trichosporon yeast (e.g., T. pullans, T. cutaneum), Pichia yeast (e.g., P. pastoris) Kluyveromyces yeast (e.g. K. marxianus), and Lipomyces yeast (e.g., L. starkeyii, L. lipoferus).

Any suitable fungus may be selected as a host cell, including without limitation Aspergillus fungi (e.g., A. parasiticus, A. nidulans), Thraustochytrium fungi, Schizochytrium fungi and Rhizopus fungi (e.g., R. arrhizus, R. oryzae, R. nigricans). The fungus may for example be an A. parasiticus strain such as strain ATCC24690, or an A. nidulans strain such as strain ATCC38163.

Eukaryotic host cells from non-microbial organisms can also be utilized as host cells in accordance with the present invention. Examples of such cells, include, without limitation, insect cells (e.g., Drosophila (e.g., D. melanogaster), Spodoptera (e.g., S. frugiperda Sf9 or Sf21 cells) and Trichoplusa (e.g., High-Five cells); nematode cells (e.g., C. elegans cells); avian cells; amphibian cells (e.g., Xenopus laevis cells); reptilian cells; and mammalian cells (e.g., NIH3T3, 293, CHO, COS, VERO, C127, BHK, Per-C6, Bowes melanoma and HeLa cells).

The aforementioned host cells are commercially available, for example, from Invitrogen Corporation, (Carlsbad, CA), American Type Culture Collection (Manassas, Virginia), and Agricultural Research Culture Collection (NRRL; Peoria, Illinois).

.Suitable host cells are selected for their capability of converting the provided substrate (i.e. intermediate product) into the desired organic end product. Therefore, the host cell must be capable of channeling the substrate in and preferably of channeling the product out of the cell. In addition, the host cell should be tolerant to both the substrate and especially the accumulated product. Advantageously, the host cell can cope with the pH and temperature changes occurring during cultivation in the presence of the substrate. Thus, the host cell preferably provides for a high reaction rate, high yield and purity of the end product.

Depending on the organic product obtained from the host cell, it may be beneficial to use a host cell which is non-pathogenic, in particular non-pathogenic for humans, for example when the organic end product obtained from said host cell is intended for further processing in the pharmaceutical, cosmetic or food industry.

It is equally conceivable that the host cell is a genetically modified (i.e. recombinant) or a non-genetically modified host cell.

### Non-genetically modified host cells

Non-genetically modified host cells (also referred to as non-genetically modified organism or non-GMO herein) are host cells whose genetic material has not been altered using recombinant DNA technology techniques in contrast to genetically modified host cells (also termed "GMO" herein). The term "non-GMO" includes both wild-type host cells and host cells comprising mutations.

The use and creation of GMOs is governed by varying national regulations and guidelines. In particular when producing food products, use of non-GMOs is typically preferable.

Said non-GMO is preferably capable of catalyzing the conversion of the intermediate product obtained from step (ii) of the inventive method to the desired organic end product. That is, said non-GMO preferably comprises endogenous genes encoding for the polypeptide(s) of interest required for biocatalytic conversion of the intermediate products into the desired organic end products according to the methods of the invention.

As regards "lignin processing" as described herein, host cells comprising endogenous genes encoding for polypeptides having catechob-1,2-dioxygenase activity and are thus conceivable for use as non-genetically modified host cells include without limitation Acinetobacter sp. (e.g. A. calcoaceticus PHEA-2, A. gyllenbergii NIPH 230, A. junii CIP 64.5, A. Iwoffii NCTC 5866 = CIP 64.10, A. oleivorans DR1, A. radioresistens DSM 6976 = NBRC 102413 = CIP 103788, A. schindleri CIP 107287, A. schindleri CIP 107287), Amycolatopsis mediterranei U32, Arthrobacter sp., Aspergillus sp. (e.g. A. niger CBS 513.88, A. oryzae RIB40), Bordetella holmesii ATCC 51541, Bradyrhizobium sp. (e.g. B. diazoefficiens USDA 110, B. genosp. SA-4 str. CB756), Burkholderia sp. (e.g. B. cenocepacia J2315, B. glumae BGR1, B. mallei ATCC 23344, B. multivorans, B. pseudomallei K96243, B. xenovorans LB400), Candida dubliniensis CD36, Corynebacterium glutamicum ATCC 13032, Cupriavidus metallidurans CH34, Delftia acidovorans SPH-1, Enterobacter aerogenes KCTC 2190, Herbaspirillum seropedicae SmR1, Klebsiella pneumoniae subsp. pneumoniae HS11286, Mycobacterium smegmatis str. MC2 155, Neorhizobium galegae bv. orientalis str. HAMBI 540, Neurospora crassa OR74A, Pseudomonas sp. (e.g. P. aeruginosa PAO1, P. fluorescens SBW25, P. fragi B25, P. putida KT2440, P. stutzeri A1501), Ralstonia sp. (e.g. R. eutropha H16, R. pickettii 12J), Rhizobium sp. (e.g. R. etli CFN 42, R. leguminosarum bv. trifolii CB782), Rhodococcus sp. (e.g. R. erythropolis PR4), R. fascians NBRC 12155 = LMG 3623, R. jostii RHA1), Sinorhizobium sp. (e.g. S. fredii NGR234, S. meliloti 1021, S. wenxiniae), Sphingomonas sp. KA1, Thermus thermophilus HB8, Verticillium albo-atrum VaMs.102.

As set out elsewhere herein, host cells will typically be selected for ease of handling, tolerance to culture conditions, and (high) substrate concentrations, insensitivity towards accumulated end product and capability of producing the end product at high reaction rates in high yields and purity. Particularly envisaged as non-GMO host cells for use in lignin processing as described herein are host cells selected from *Pseudomonas,* preferably P. *putida,* and more preferably from *P. putida* strain KT2440.

### Recombinant host cell

Recombinant host cells (also referred to as genetically modified organisms or GMOs) are also envisaged for use in accordance with the methods of the invention. Recombinant host cells are host cells whose genetic material has been altered using recombinant DNA technologies. It is in particular envisaged that recombinant host cells comprise at least one heterologous nucleic acid sequence.

The heterologous nucleic acid sequence may e.g. be a heterologous gene regulation element, or a heterologous gene. Useful heterologous genes in the context of the present invention encode polypeptides of interest, i.e. polypeptides aiding in the production of the desired organic end product from the intermediate product obtained in step (ii) of the method of the invention. E.g., in the lignin processing method as contemplated herein, the intermediate product is envisaged to be catechol, and a host cell comprising at least one (optionally heterologous) gene encoding a polypeptide having catechob-1,2-dioxygenase activity is envisaged, in particular a catA gene and/or a *catA2* gene. Further heterologous genes that may advantageously be present in the host cell include genes for the metabolic funneling of aromats, e.g. phenol and cresol.

### Heterologous nucleic acid sequence

The term "heterologous" or "exogenous" nucleic acid sequence is used herein to refer to a nucleic acid sequence not naturally occurring in, i.e. foreign to, the host cell. In other words, a heterologous nucleic acid sequence is not found in wild-type host cells. The term includes nucleic acid sequences such as heterologous regulatory sequences (e.g. promoters) and heterologous genes. The heterologous nucleic acid sequences may be derived from another "donor" cell, or be a synthetic or artificial nucleic acid sequences. Heterologous gene(s) in the context of the present invention are in particular envisaged to encode for the polypeptide(s) of interest required for catalyzing conversion of said compound into a product (i.e. the desired organic end product), and optionally also for channeling in of the substrate (i.e. the intermediate product), and exporting the product from the host cell.

### Preparation

Recombinant host cells can be prepared using genetic engineering methods known in the art. The process of introducing nucleic acids into a recipient host cell is also termed "transformation" or "transfection" hereinafter. The terms are used interchangeably herein.

Host cell transformation typically involves opening transient pores or "holes" in the cell wall and/or cell membrane to allow the uptake of material. Illustrative examples of transformation protocols involve the use of calcium phosphate, electroporation, cell squeezing, dendrimers, liposomes, cationic polymers such as DEAE-dextran or polyethylenimine, sonoporation, optical transfection, impalefection, nanoparticles (gene gun), magnetofection, particle bombardement, alkali cations (cesium, lithium), enzymatic digestion, agitation with glass beads, viral vectors, or others. The choice of method is generally dependent on the type of cell being transformed, the nucleic acid to be introduced into the cell and the conditions under which the transformation is taking place.

### Transient expression

A nucleic acid molecule encoding a polypeptide of interest (for instance a polypeptide having catechol-1,2-dioxygenase activity) and an operably linked regulatory sequence such as a promoter may be introduced into a recipient host cell either as a non-replicating DNA or RNA molecule, which may be a linear molecule or a closed covalent circular molecule. Such molecules are incapable of autonomous replication, and the expression of the gene occurs through the transient expression of the introduced sequence.

### Stable expression

The heterologous nucleic acid sequence, in particular gene (for instance a gene encoding for a polypeptide having catechol-1,2-dioxygenase activity such as a catA or *catA2* gene) may be stably integrated into the host cell's genome. Permanent (stable) expression of the gene encoding the polypeptide of interest may be achieved by integration of the introduced DNA sequence into the host cell chromosome. Stable expression may also be achieved by providing the gene of interest in a vector capable of autonomously replicating in the host cell.

The vector employed for delivery of the heterologous nucleic acid sequence to be expressed stably is envisaged to be capable of integrating the desired gene sequences into the host cell chromosome, or of autonomously replicating within the host cell; thereby ensuring maintenance of the heterologous nucleic acid sequence in the host cell and stable integration into the host cell's genome. Cells with DNA stably integrated into their genomes can be selected by also introducing one or more markers into the vector, e.g. providing for prototrophy to an auxotrophic host, biocide (e.g. antibiotics or heavy metal) resistance, or the like. The selectable marker gene sequence can either be directly linked to the DNA gene sequences to be expressed, or introduced into the same cell by co-transfection. Additional elements may also be needed for optimal synthesis of mRNA. These elements may include splice signals, as well as transcription promoters, enhancers, and termination signals.

### Vector

The heterologous nucleic acid molecule of interest can be delivered to the host cell in the form of a vector, e.g. a plasmid or viral vector. If said heterologous nucleic acid molecule is e.g. a DNA molecule and comprises a gene of interest, it is envisaged that said vector comprises regulatory sequences that allow for the expression of said gene of interest. The vector may or may not comprise sequences enabling autonomous replication of said vector in the host cell, depending on whether transient or stable expression of the gene is intended, as explained above.

Any of a wide variety of vectors may be employed for this purpose. The person skilled in the art will readily understand that selection of a particular vector include depends, e.g., on the nature of the host cell, the intended number of copies of the vector, whether transient or stable expression of the gene of interest is envisaged, and so on.

Illustrative examples of vectors conceivable for use in accordance with the invention include, without limitation, viral origin vectors (M13 vectors, bacterial phage λ vectors, baculovirus vectors, adenovirus vectors, and retrovirus vectors), high, low and adjustable copy number vectors, eukaryotic episomal replication vectors (pCDM8), and prokaryotic expression vectors such as pcDNA II, pSL301, pSE280, pSE380, pSE420, pTrcHisA, B, and C, pRSET A, B, and C (Invitrogen, Inc.), pGEMEX-1, and pGEMEX-2 (Promega, Inc.), the pET vectors (Novagen, Inc.), pTrc99A, pKK223-3, the pGEX vectors, pEZZ18, pRIT2T, and pMC1871 (Pharmacia, Inc.), pKK233-2 and pKK388-1 (Clontech, Inc.), and pProEx-HT (Life Technologies, Inc.) and variants and derivatives thereof. Vectors can also be eukaryotic expression vectors such as pFastBac, pFastBac HT, pFastBac DUAL, pSFV, and pTet-Splice (Life Technologies, Inc.), pEUK-Cl, pPUR, pMAM, pMAMneo, pBI101, pBI121, pDR2, pCMVEBNA, and pYACneo (Clontech), pSVK3, pSVL, pMSG, pCH110, and pKK232-8 (Pharmacia, Inc.), p3'SS, pXTl, pSG5, pPbac, pMbac, pMC1neo, and pOG44 (Stratagene, Inc.), and pYES2, pAC360, pBlueBacHis A, B, and C, pVL1392, pBsueBaclll, pCDM8, pcDNAI, pZeoSV, pcDNA3 pREP4, pCEP4, and pEBVHis (Invitrogen, Inc.) and variants or derivatives thereof are also conceivable.

Further vectors of interest include pUC 18, pUC 19, pBlueScript, pSPORT, cosmids, phagemids, fosmids (pFOSI), YAC's (yeast artificial chromosomes), BAC's (bacterial artificial chromosomes), pBAC 108L, pBACe3.6, pBeloBACI1 (Research Genetics), PACs, P1 (E. coli phage), pQE70, pQE60, pQE9 (Qiagen), pBS vectors, PhageScript vectors, BlueScript vectors, pNH8A, pNH16A, pNH18A, pNH46A (Stratagene), pcDNA3 (InVitrogen), pGEX, pTrsfus, pTrc99A, pET-5, pET-9, pKK223-3, pKK233-3, pDR540, pRIT5 (Pharmacia), pSPORT1, pSPORT2, pCMVSPORT2.0, pSV-SPORT1 (Life Technologies, Inc.), and the vectors described in Provisional Patent Application No. 60/065,930, filed Oct. 24, 1997.

It will be acknowledged that the vector may comprise regulatory sequences as exemplified elsewhere herein, preferably operably linked to, e.g. upstream of, the gene of interest.

After the introduction of the vector, recipient cells can be grown in a selective medium, which selects for the growth of vector-containing cells. Expression of the heterologous gene(s) of interest (e.g. a *catA* or *catA2* gene) is envisaged to result in the production of a polypeptide of interest (e.g. a polypeptide having catechol-1,2-dioxygenase activity).

### Catechol-1,2-dioxygenase activity

As described in the foregoing, the (optionally heterologous) gene of interest preferably encodes a polypeptide of interest having a desired capability that beneficially enables biocatalytic conversion of the intermediate product into the organic end product according to the methods of the invention. In particular as regards the "lignin processing" method as described herein, the host cell preferably expresses at least one (optionally heterologous) gene encoding a polypeptide having catechol-1,2-dioxygenase activity. Said (optionally heterologous) gene may be a *catA* gene or a *catA2* gene. It is also envisioned herein to use host cells comprising both at least one *catA* gene and at least one *catA2* gene.

Catechol-1,2-dioxygenase (EC 1.13.11.1) catalyzes intradiol (or ortho-) cleavage of catechol as, thereby producing cis-cis muconic acid. Catechol-1,2-dioxygenase activity can be easily assessed spectrophotometrically by measurement of the increase in absorbance at λ = 260 nm, corresponding to the formation of cis,cis-muconic acid as reported by Silva et al. Braz J Microbiol. 2013; 44(1): 291-297.

### CatA and CatA2

Said gene of interest encoding a polypeptide having catechol-1,2-dioxygenase activity is envisaged to be a *catA* or *catA2* gene. The protein encoded by a *catA* gene or a *catA2* gene may be identified in a database as a catechol-1,2-dioxygenase.

The term "gene of interest" and "polypeptide of interest", in particular "catA" and/or "catA2", includes variants. The term "variant" or with reference to a nucleic acid or polypeptide refers to polymorphisms, i.e. the exchange, deletion, or insertion of one or more nucleotides or amino acids, respectively, compared to the predominant form of the respective nucleic acid or polypeptide. In the context of the present invention, a "variant" may refer to a contiguous sequence of at least about 50, such as about 100, about 200, or about 300 amino acids set forth in the amino acid sequence of a protein named herein (cf. e.g. below), or the corresponding full-length amino acid sequence, with the proviso that said alteration is included in the respective amino acid sequence. In case the mutation leads to a premature stop codon in the nucleotide sequence encoding the protein, the sequence may even be shorter than the corresponding wild type protein.

Variants of genes of interest as described herein, in particular catA and/or catA2, may be orthologs. An ortholog, or orthologous gene, is a gene with a sequence that has a portion with similarity to a portion of the sequence of a known gene, but found in a different species than the known gene. An ortholog and the known gene originated by vertical descentfrom a single gene of a common ancestor.

As used herein a variant or ortholog of the *catA* or *catA2* gene is envisaged to encode a protein having catechol-1,2-dioxygenase activity and having at least about 60 %, at least about 65 %, at least about 70 %, at least about 75 %, at least about 80 % or at least about 90 %, including at least 95%, at least 97%, at least 98%, at least 99%, or at least 99.5% identity or 100% sequence identity with a known *catA* gene, in particular PP_3713 of P. *putida* KT2440, or a known *catA2* gene, in particular PP_3166 of *P. putida* KT2440, respectively.

Variants substantially similar to known POls, in particular catA and/or catA2 polypeptides, are preferred. A sequence that is substantially similar to a catA or catA2 polypeptide may have at least about 60 %, at least about 65 %, at least about 70 %, at least about 75 %, at least about 80 % or at least about 90 %, including at least 95%, at least 97%, at least 98%, at least 99%, or at least 99.5% identity or 100% sequence identity with the sequence of a known catA or catA2 or polypeptide, respectively.

By "% identity" is meant a property of sequences that measures their similarity or relationship. Identity is measured by dividing the number of identical residues by the total number of residues and gaps and multiplying the product by 100. Preferably, identity is determined over the entire length of the sequences being compared. "Gaps" are spaces in an alignment that are the result of additions or deletions of amino acids. Thus, two copies of exactly the same sequence have 100% identity, but sequences that are less highly conserved, and have deletions, additions, or replacements, may have a lower degree of identity. Those skilled in the art will recognize that several computer programs are available for determining sequence identity using standard parameters, for example Blast (Altschul, et al. (1997) Nucleic Acids Res. 25:3389-3402), Blast2 (Altschul, et al. (1990) J. Mol. Biol. 215:403-410), and Smith-Waterman (Smith, et al. (1981) J. Mol. Biol. 147:195-197). The term "mutated" or "mutant" in reference to a nucleic acid or a polypeptide refers to the exchange, deletion, or insertion of one or more nucleotides or amino acids, respectively, compared to the naturally occurring nucleic acid or polypeptide.

"Sequence identity" or "% identity" refers to the percentage of residue matches between at least two polypeptide or polynucleotide sequences aligned using a standardized algorithm. Such an algorithm may insert, in a standardized and reproducible way, gaps in the sequences being compared in order to optimize alignment between two sequences, and therefore achieve a more meaningful comparison of the two sequences. Sequence comparisons can be performed using standard software programs such as the NCBI BLAST program.

In the context of the invention, the expression "position corresponding to another position" (e.g., regions, fragments, nucleotide or amino acid positions, or the like) is based on the convention of numbering according to nucleotide or amino acid position number and then aligning the sequences in a manner that maximizes the percentage of sequence identity. Because not all positions within a given "corresponding region" need be identical, non-matching positions within a corresponding region may be regarded as "corresponding positions." Accordingly, as used herein, referral to an "amino acid position corresponding to amino acid position [X]" of a specified protein sequence represents, in addition to referral to amino acid positions of the specified protein sequence, referral to a collection of equivalent positions in other recognized protein and structural homologues and families.

Thus, when a position is referred to as a "corresponding position" in accordance with the disclosure it is understood that nucleotides/amino acids may differ in terms of the specified numeral but may still have similar neighbouring nucleotides/amino acids. Such nucleotides/amino acids which may be exchanged, deleted or added are also included in the term "corresponding position".

Specifically, in order to determine whether an amino acid residue of the amino acid sequence of a polypeptide of interest, e.g. a catA or catA2 polypeptide different from a known host cell, corresponds to a certain position in the amino acid sequence of the known host cell, a skilled artisan can use means and methods well-known in the art, e.g., alignments, either manually or by using computer programs such as BLAST2.0, which stands for Basic Local Alignment Search Tool or ClustalW or any other suitable program which is suitable to generate sequence alignments. Accordingly, a known wild-type catA or catA2 polypeptide (or nucleic acid encoding the same) may serve as "subject sequence" or "reference sequence", while the amino acid sequence of a catA or catA2 polypeptide (or nucleic acid sequence encoding the same) different from the wild-type can serve as "query sequence". The terms "reference sequence" and "wild type sequence" are used interchangeably herein.

As set out above, a host cell employed in the methods of the invention -in particular in the lignin processing method as described herein- may comprise a catA gene. The *catA* gene may be an endogenous gene or a heterologous gene. Said *catA* gene may be under the control of an endogenous promoter, either a wild-type promoter or a mutated promoter, or a heterologous promoter. Additionally or alternatively, the host cell may comprise a catA2 gene. The *catA2* gene may be an endogenous gene or a heterologous gene. Said *catA2* gene may be under the control of an endogenous promoter, either a wild-type promoter or a mutated promoter, or a heterologous promoter. Said promoter may be different from the promoter that controls expression of the *catA* gene. The *catA* gene may be under the control of a promoter that is similar or identical to the promoter that controls the *catA2* gene.

It is in particular envisaged that the host cell may comprise a (optionally heterologous) *catA* gene and a (optionally heterologous) *catA2* gene. Thus, the host cell may comprise an endogenous *catA* gene and an endogenous *catA2* gene. A host cell comprising a heterologous *catA* gene and a heterologous *catA2* gene is also conceivable. Also envisaged herein are host cells comprising an endogenous *catA* gene and a heterologous *catA2* gene, and *vice versa.*

Host cells comprising at least one endogenous gene encoding a polypeptide having catechol-1,2-dioxygenase activity, such as a *catA* and/or *catA2* gene as described herein, include, without limitation, Acinetobacter sp. (e.g. A. calcoaceticus PHEA-2, A. gyllenbergii NIPH 230, A. junii CIP 64.5, A. Iwoffii NCTC 5866 = CIP 64.10, A. oleivorans DR1, A. radioresistens DSM 6976 = NBRC 102413 = CIP 103788, A. schindleri CIP 107287, A. schindleri CIP 107287), Amycolatopsis mediterranei U32, Arthrobacter sp., Aspergillus sp. (e.g. A. niger CBS 513.88, A. oryzae RIB40), Bordetella holmesii ATCC 51541, Bradyrhizobium sp. (e.g. B. diazoefficiens USDA 110, B. genosp. SA-4 str. CB756), Burkholderia sp. (e.g. B. cenocepacia J2315, B. glumae BGR1, B. mallei ATCC 23344, B. multivorans, B. pseudomallei K96243, B. xenovorans LB400), Candida dubliniensis CD36, Corynebacterium glutamicum ATCC 13032, Cupriavidus metallidurans CH34, Delftia acidovorans SPH-1, Enterobacter aerogenes KCTC 2190, Herbaspirillum seropedicae SmR1, Klebsiella pneumoniae subsp. pneumoniae HS11286, Mycobacterium smegmatis str. MC2 155, Neorhizobium galegae bv. orientalis str. HAMBI 540, Neurospora crassa OR74A, Pseudomonas sp. (e.g. P. aeruginosa PAO1, P. fluorescens SBW25, P. fragi B25, P. putida KT2440, P. stutzeri A1501), Ralstonia sp. (e.g. R. eutropha H16, R. pickettii 12J), Rhizobium sp. (e.g. R. etli CFN 42, R. leguminosarum bv. trifolii CB782), Rhodococcus sp. (e.g. R erythropolis PR4), R. fascians NBRC 12155 = LMG 3623, R. jostii RHA1), Sinorhizobium sp. (e.g. S. fredii NGR234, S. meliloti 1021, S. wenxiniae), Sphingomonas sp. KA1, Thermus thermophilus HB8, Verticillium albo-atrum VaMs.102.

Particularly preferred host cells for use in lignin processing as described herein are selected from Pseudomonas, preferably P. putida, and more preferably from P. putida strain KT2440..

As set out herein, the GOI encoding a polypeptide of interest, e.g. a polypeptide having catechol-1,2-dioxygenase activity, may be encoded by a heterologous gene. Said heterologous gene may be a catA gene or a *catA2* gene. The host cell comprising the heterologous gene is also termed "recipient host cell" herein, whereas the host cell from which the heterologous gene is obtained is also referred to as "donor host cell": Suitable donor host cells include host cells comprising an endogenous gene encoding for a polypeptide of interest, e.g. with regards to the lignin processing method described herein, a polypeptide having catechol-1,2-dioxygenase activity such as a catA polypeptide and/or a catA2 polypeptide. The skilled person will readily acknowledge that heterologous genes encoding for polypeptides of interest, e.g. polypeptides having catechol-1,2-dioxygenase activity, can advantageously be obtained from host cells expressing said gene endogenously, e.g. host cells expressing an endogenous catechol-1,2-dioxygenase as listed above. Exemplary donor cells can be selected from *Pseudomonas,* preferably *P. putida,* more preferably *P. putida* strain KT2440. The heterologous gene encoding the polypeptide of interest (for instance a polypeptide having catechol-1,2-dioxygenase activity) can be introduced using into the recipient host cell using recombinant DNA technology as described elsewhere herein. Any of the various host cells specified herein is in principle suitable as a recipient host cell. E.g., host cells not expressing an endogenous *catA* and/or *catA2* gene may be selected as recipient host cells.

The *catA* gene is in particular envisaged to be the gene PP_3713 encoding the catA polypeptide of *Pseudomonas putida,* strain KT2240, with Uniprot accession No. Q88GK8 (Version 79 of 04 Feb 2015), and may also be referred to as PP_3713. Variants of PP_3713 may also be used. It is further envisaged that said the *catA* gene may encode for a polypeptide comprising a sequence corresponding to SEQ ID No. 1. Said *catA* gene may comprise a sequence corresponding to SEQ ID No. 2.

The *catA2* gene is in particular envisaged to be the gene PP_3166 encoding the catA2 polypeptide of *Pseudomonas putida,* strain KT2240, with Uniprot accession No. Q88I35 (Version 70 of 22 Jul 2015). Variants of PP_3166 may also be used. It is envisaged that said the *catA2* gene may encode for a polypeptide comprising a sequence corresponding to SEQ ID No. 3. Said *catA2* gene may comprise a sequence corresponding to SEQ ID No. 4.

In accordance with the foregoing, it is envisaged that the host cell may comprise at least one (optionally heterologous) *catA* gene, optionally comprising a sequence corresponding to SEQ ID No. 2; and/or at least one (optionally heterologous) *catA2* gene, optionally comprising a sequence corresponding to SEQ ID No. 4. Said host cell may thus express a (optionally heterologous) catA polypeptide comprising a sequence corresponding to SEQ ID No. 1; and/or a (optionally heterologous) catA2 polypeptide comprising a sequence corresponding to SEQ ID No. 3.

### Further genes

It is further envisaged that the host cell may be equipped with further (optionally heterologous) genes which advantageously aid in converting (by-)products obtained during sub- and/or supercritical fluid-assisted conversion. An exemplary (by-)product would be protocatechuate, a key intermediate in degradation of the lignin and other aromic compounds, such as vanillate, benzoate, coumarate and ferulate. Protocatechuate decarboxylase (AroY, EC 4.1.1.63) is an enzyme which catalyzes the conversion of protocatechuate to catechol and therefore enables the use of multiple aromatic compounds as carbon sources. Furthermore the presence of 4 hydroxybenzoate decarboxylase subunit B (KpdB, EC 4.1.1.61) was shown to increase the activity of AroY (T. Sonoki et al. J Biotechnol. 2014 Dec 20;192 Pt A:71-7.)

### AroY

In particular with regards to lignin processing, it is thus envisioned that the host cell may further comprise an (optionally heterologous) gene encoding for a polypeptide having protocatechuate decarboxylase (EC 4.1.1.63) activity. An illustrative example is the AroY polypeptide of *Klebsiella pneunomia,* strain A170-10, with Uniprot accession No. B9A9M6 (version 14 of 24 July 2015) or a variant thereof. Said polypeptide may comprise a sequence corresponding to SEQ ID No. 17. The gene may be an *AroY* gene of *K. pneunomia,* strain A170-10 comprising a sequence corresponding to SEQ ID No. 18 or a variant thereof. E.g., an exemplary codon-optimized version of the *AroY* gene according to SEQ ID No. 33 may also be used.

### KpdB

Additionally, the host cell may comprise an (optionally heterologous) gene encoding for a polypeptide having 4 hydroxybenzoate decarboxylase subunit B activity (KpdB, EC 4.1.1.61). An illustrative example is the KpdB polypeptide of *Klebsiella pneunomia* NBRC 114940, with Uniprot accession No. X5I148 (version 5 of 22 July 2015) or a variant thereof. Said polypeptide may comprise a sequence corresponding to SEQ ID No. 19. The gene may be the *kdpb* gene of *K. pneunomia* NBRC 114940 comprising a sequence corresponding to SEQ ID No. 20 or a variant thereof. E.g., an exemplary codon-optimized version of the *kpdB* gene according to SEQ ID No. 34 may be used.

### pheA

Phenol and cresol are the two main by-products that accumulate during sub- and/or supercritical fluid-assisted conversion of lignin.

Thus, for the conversion of phenol, in particular with regards to lignin processing as described herein, it is envisioned that the host cell may further comprise an (optionally heterologous) gene encoding for a polypeptide having phenol 2-monooxygenase activity. An illustrative example is the pheA polypeptide of *P. putida* sp. EST1001 with Uniprot Acc. No. Q52159 (version 54 of 24 June 2015) comprising a sequence corresponding to SEQ ID No. 21. Said polypeptide may be encoded by a gene comprising a sequence corresponding to SEQ ID No. 22 or a variant thereof.

### pcmh

For the conversion of cresol, in particular with regards to lignin processing as described herein, it is envisioned that the host cell may further comprise an (optionally heterologous) gene encoding for a polypeptide having p-cresol methylhydroxylase activity. An illustrative example is the pcmh polypeptide comprising the pchF subunit of *Pseudomonas putida* (*Arthrobacter siderocapsulatus*) with Uniprot Acc. R9WN81 (version 12 of May 27, 2015) and the pchC subunit of *Pseudomonas putida* (*Arthrobacter siderocapsulatus*) with Uniprot Acc. No. P09787 (version 94 of April 1, 2015). Said pchF subunit may comprise a sequence corresponding to SEQ ID No. 23. Said pchC subunit may comprise a sequence corresponding to SEQ ID No. 25. The pchF subunit may be encoded by a gene comprising a sequence corresponding to SEQ ID No. 24 or a variant thereof. The pchC subunit may be encoded by a gene comprising a sequence corresponding to SEQ ID No. 26 or a variant thereof.

Other genes useful for processing of (by-)products are also conceivable and can be selected by the skilled person in the art depending on the intermediate product(s) obtained after sub- and/or supercritical fluid assisted conversion and the desired organic products to be obtained.

The genes described herein may be operable linked to an (optionally heterologous) promoter, said promoter may comprise a sequence corresponding to SEQ ID No. 5, 6, 7, 8, 9, 10, 11, 12, 13, 14, 15 or 16. An optional construct comprising aroY and kdpb operably linked to, e.g. upstream of, suitable promoter sequences is disclosed in SEQ ID No. 35. An optional construct comprising pheA and pcmh operably linked to, e.g. upstream of, suitable promoter sequences is disclosed in SEQ ID No. 36.

### Regulatory sequences

The terms "expression" and "expressed", as used herein, are used in their broadest meaning, to signify that a sequence included in a nucleic acid molecule and encoding a peptide/protein is converted into its peptide/protein product. Thus, where the nucleic acid is DNA, expression refers to the transcription of a sequence of the DNA into RNA and the translation of the RNA into protein. A nucleic acid molecule, such as DNA, is said to be "capable of expressing" a peptide/protein if it contains nucleotide sequences which contain transcriptional and translational regulatory information and such sequences are operably linked to nucleotide sequences which encode the polypeptide. An operable linkage is a linkage in which a nucleotide sequence encoding a polypeptide of interest is linked to one or more regulatory sequence(s) such that expression of said nucleotide sequence can take place. Thus, a regulatory sequence operably linked to a coding sequence is capable of effecting the expression of the coding sequence, for instance in an *in vitro* transcription/ translation system or in a cell when the vector is introduced into the cell. A respective regulatory sequence need not be contiguous with the coding sequence, as long as it functions to direct the expression thereof. Thus, for example, intervening untranslated yet transcribed sequences may be present between a promoter sequence and the coding sequence and the promoter sequence can still be considered "operably linked" to the coding sequence.

The term "regulatory sequence" includes controllable transcriptional promoters, operators, enhancers, silencers, transcriptional terminators, 5' and 3' untranslated regions which interact with host cellular proteins to carry out transcription and translation and other elements that may control gene expression including initiation and termination codons. The regulatory sequences can be native (endogenous), or can be foreign (heterologous) to the cell. The precise nature of the regulatory regions needed for gene sequence expression may vary from organism to organism, but shall in general include a promoter region which, in prokaryotes, contains both the promoter (which directs the initiation of RNA transcription) as well as the DNA sequences which, when transcribed into RNA, will signal synthesis initiation. The term "promoter" as used herein, refers to a nucleic acid sequence that operates gene expression. For example, in prokaryotes, the promoter region contains both the promoter (which directs the initiation of RNA transcription) as well as the DNA sequences which, when transcribed into RNA, will signal synthesis initiation. Such regions will normally include those 5'-non-coding sequences involved with initiation of transcription and translation, such as the TATA box, capping sequence or the CAAT sequence. Promoter regions vary from organism to organism, but are well known to persons skilled in the art.

The promoter operably linked to and thus driving the expression of the gene of interest in the host cell may be an endogenous, i.e. wild-type or mutated, promoter, or a heterologous promoter. Two nucleic acid sequences (such as a promoter region sequence and a sequence encoding a *catA* or *catA2* polypeptide) are said to be operably linked if the nature of the linkage between the two DNA sequences does not (1) result in the introduction of a frame-shift mutation, (2) interfere with the ability of the promoter region sequence to direct the transcription of a gene sequence encoding the polypeptide of interest, in particular *a catA* or *catA2* polypeptide, or (3) interfere with the ability of the gene sequence of a polypeptide of interest, in particular a *catA* or *catA2* polypeptide to be transcribed by the promoter region sequence.

Thus, a promoter region is operably linked to a gene if the promoter is capable of driving transcription of said gene.

### Promoters

Promoters are regarded as molecular tools that enable the modulation and regulation of expression of genes of interest in homologous organisms as well as in heterologous organisms. In order to allow for fast and efficient conversion of the intermediate product, advantageously promoters allowing for standardized and constitutive expression (i.e. continuous gene transcription) control the expression of the genes of interest (i.e. genes involved in intermediate product processing). Said promoters can be heterologous promoters or endogenous promoters. It is in particular envisaged that such promoters enable constitutive and/or standardized expression of the downstream genes, and preferably abolish the need for induction of the genes of interest. The promoter may also be equipped with a regulatory sequence/element that makes the promoter inducible and/or repressible.

E.g., with regards to lignin processing, constitutive promoters are envisaged to operably linked to, e.g. upstream of, the (optionally heterologous) *catA* gene and/or the (optionally heterologous) *catA2* gene. It is envisioned that constitutive expression of preferably *catA* and *catA2* under the control of said promoters allows for efficient conversion of catechol into the desired end product cis-cis-muconic acid, thereby preventing accumulation of the (toxic) intermediate product. If further (optionally heterologous) genes are present in the host cell that allow for conversion of other (by-)products of sub- and/or supercritical fluid-assisted conversion, a constitutive promoter may also be pesent or introduced operably linked to, e.g. upstream of, said genes.

Suitable promoters may be strong constitutive promoters, such as promoters naturally controlling the expression of housekeeping genes which typically constitutive genes that are transcribed at a relatively constant level as their products are typically needed for maintenance of the cell and their expression (PrpoD, PgyrB, Ptuf and PgroES) is usually unaffected by experimental conditions.

Promoter strength may be tuned to be appropriately responsive to activation or inactivation. The promoter strength may also be tuned to constitutively allow an optimal level of expression of a gene of interest or of a plurality of gene of interest. Strength of expression can, for example, be determined by the amount/yield of organic end product production and/or by quantitative reverse transcriptase PCR (qRT-PCR).

Illustrative examples of a strong constitutive promoter include, but are not limited to, the T7 promoter, the T5 promoter, the *Escherichia coli* lac promoter, the trc promoter, the tac promoter, the recA promoter, the adenyl methyltransferase (AMT) promoters AMT-1 and AMT-2, and synthetic promoters derived from the foregoing promoters or e.g. Pcp7 as disclosed in Spexard et al (Biotechnol Lett (2010) 32, 243-248).

The present inventors further provide a promoter library comprising particulary suitable promoters for regulating the expression of the genes of interest, especially *catA* and/or *catA2.* Said promoters favorably allow constitutive expression, and preferably strong and standardized expression, of *catA* and/or *catA2,* and are envisaged to comprise a sequence corresponding to
(i) SEQ ID No. 5 [Pem7]; or
(ii) SEQ ID No. 6 [Pem7*]; or
(iii) SEQ ID No. 7 [Ptuf]; or
(iv) SEQ ID No. 8 [PrpoD]; or
(v) SEQ ID No. 9 [Plac]; or
(vi) SEQ ID No. 10 [PgyrB]; or
(vii) SEQ ID No. 11; or
(viii) SEQ ID No. 12; or
(ix) SEQ ID No. 13; or
(x) SEQ ID No. 14; or
(xi) SEQ ID No. 15; or
(xii) SEQ ID No. 16, or
(xiii) SEQ ID No. 88 [Ptuf_1]; or
(xiv) SEQ ID No. 89 [Ptuf_short]; or
(xv) SEQ ID No. 90 [Ptuf_s_2]; or
(xvi) SEQ ID No. 91 [Ptuf_s_3]; or
(xvii) SEQ ID No. 92 [Ptuf_s_4]; or
(xviii) SEQ ID No. 93 [Ptuf_s_5]; or
(xix) SEQ ID No. 94 [Ptuf_s_6]; or
(xx) SEQ ID No. 95 [Ptuf_s_7]; or
(xxi) SEQ ID No. 96 [Ptuf_s_8]; or
(xxii) SEQ ID No. 97 [Ptuf_s_9]; or
(xxiii) SEQ ID No. 98 [Ptuf_s_10]; or
(xxiv) SEQ ID No. 99 [Ptuf_s_11]; or
(xxv) SEQ ID No. 100 [Ptuf_s_12]; or
(xxvi) SEQ ID No. 101 [Pgro]; or
(xxvii) SEQ ID No. 102 [Pgro_1]; or
(xxviii) SEQ ID No. 103 [Pgro_2]; or
(xxix) SEQ ID No. 104 [Pgro_4]; or
(xxx) SEQ ID No. 105 [Pgro_5].

In particular with regards to lignin processing, it is thus envisaged to employ a host cell such as *P. putida* comprising at least one (optionally heterologous) *catA* gene and/or at least one (optionally heterologous) *catA2* gene as described elsewhere herein, each or any of said genes under the control of an (optionally heterologous) promoter comprising a sequence corresponding to SEQ ID No. 5 [Pem7]; or SEQ ID No. 6 [Pem7*]; or SEQ ID No. 7 [Ptuf]; or SEQ ID No. 8 [PrpoD]; or SEQ ID No. 9 [Plac]; SEQ ID No. 10 [PgyrB], or SEQ ID No. 11; or SEQ ID No. 12; or SEQ ID No. 13; or SEQ ID No. 14; or SEQ ID No. 15; or SEQ ID No. 16; SEQ ID No. 88 [Ptuf_1]; or SEQ ID No. 89 [Ptuf_short]; or SEQ ID No. 90 [Ptuf_s_2]; or SEQ ID No. 91 [Ptuf_s_3]; or SEQ ID No. 92 [Ptuf_s_4]; or SEQ ID No. 93 [Ptuf_s_5]; or SEQ ID No. 94 [Ptuf_s_6]; or SEQ ID No. 95 [Ptuf_s_7]; or SEQ ID No. 96 [Ptuf_s_8]; or SEQ ID No. 97 [Ptuf_s_9]; or SEQ ID No. 98 [Ptuf_s_10]; or SEQ ID No. 99 [Ptuf_s_11]; or SEQ ID No. 100 [Ptuf_s_12]; or SEQ ID No. 101 [Pgro]; or SEQ ID No. 102 [Pgro_1]; or SEQ ID No. 103 [Pgro_2]; or SEQ ID No. 104 [Pgro_4]; or SEQ ID No. 105 [Pgro_5].

### Endogenous promoters

Host cells comprising endogenous genes of interest and endogenous promoters (i.e., non-genetically modified host cells) are thus easy to work with and may be advantageous in a variety of applications. For example, in the lignin processing method described herein, *Pseudomonas sp.,* e.g., *Pseudomonas putida* comprising an endogenous *catA* gene and an endogenous *catA2* gene, both under the control of endogenous promoters, can be utilized.

### Heterologous promoters

The promoter may, however, also be heterologous to the host cell. A heterologous promoter can be introduced operably linked to, e.g. upstream of, the gene(s) of interest into the genome of a host cell which naturally harbors said genes using common genetic engineering techniques. The heterologous promoter may also be introduced operably linked to, e.g. upstream of, heterologous genes of interest and inserted as an expression cassette/unit into the genome of a host cell. The expression cassette may also be present in an extrachromosomal element such as a vector, e.g. a plasmid. Culture conditions

Host cells and recombinant host cells may be provided in any suitable form. For example, such host cells may be provided in liquid culture or solid culture (e.g., agar-based medium), which may be a primary culture or may have been passaged (e.g., diluted and cultured) one or more times. Host cells also may be provided in frozen form or dry form (e.g., lyophilized). Host cells may be provided at any suitable concentration.

Host cells are preferably cultured under conditions that allow production of the desired organic end product, e.g. cis-cis-muconic acid in the lignin processing method as described herein. Suitable conditions are within the routine knowledge of the skilled artisan. The term "cultivation of cells" or "culturing of cells" in medium in the context of the host cells of the present invention generally refers to the seeding of the cells into a culture vessel, to the growing of the cells in medium in the logarithmic phase until a sufficient cell density is established and/or to the maintenance of the cells in medium, respectively. Culturing can be performed in any container suitable for culturing cells.

The skilled person will readily understand that culture conditions will vary depending on the host cell, and the characteristics of the intermediate product and organic end product. Suitable conditions for culturing the host cell typically include culturing the same in an aqueous medium that is suitable for sustaining cell viability and cell growth and allows the host cell to produce the desired organic product. For instance, in the lignin processing method provided herein, suitable culture conditions that enable the biocatalyst, in particular *Pseudomonas sp.,* preferably *P. putida* and more preferably P. putida KT2440, to convert the substrate catechol into the desired organic product cis-cis-muconic acid, may comprise E-2 minimal medium with glucose as a carbon source (pH 7) and a reaction temperature of about 30°C as described in the appended examples. Also, in order to express the necessary enzymes, in particular *catA2* situated in the ben operon, expression of the catA2 polypeptide may require induction. Thus, addition of an agent for induction, e.g. benzoic acid, may be required.

### Cell culture medium

Illustrative examples of a suitable cell culture medium, for example for culturing a bacterial host such as a *Pseudomonas* sp. host or a *Burkholderia sp.* host, include, but are not limited to, Luria-Bertani (LB) complex medium, Inkas-medium, phosphate-limited protease peptone-glucose-ammonium salt medium (PPGAS), Minimal medium E (MME), nitrogen-limited minimal medium or mineral salt medium. The media used may include a factor selected from growth factors and/or attachment factors or may be void of such a factor. It may be sufficient to add such a factor only to the media used for the seeding of the cells and/or the growing of the cells, for example under logarithmic conditions. The media may contain serum or be serum-free. A variety of carbon sources may be used such as a monosaccharide, e.g. glucose, a disaccharide, e.g. sucrose, an alcohol, e.g. glycerol, an alkane, e.g. n-hexane, a fatty acid such as caprylic acid (also termed octanoate), or mixtures thereof. The bacterial host cell may for instance be in the logarithmic growth phase or in the stationary phase.

Suitable cell culture media may further include salts, vitamins, buffers, energy sources, amino acids and other substances. Any medium may be used that is suitable to sustain cell viability and in which the selected host cell is capable of producing the desired organic end product (e.g. cis-cis-muconat), as explained above.

### Recovery of organic end product

The host cells may be removed, for example by way of centrifugation or filtration, before recovering the one or more organic end products produced in a method according to the invention. E.g., host cells may be recovered, e.g. concentrated, captured, harvested and/or enriched in/on a separation or filter unit. For example, host cells as employed in the present invention may be enriched before they are collected and/or are concentrated before they are collected and/or are captured before they are collected. Enriching may, for example, be achieved by batch centrifugation, flow through centrifugation and/or tangential flow filtration.

The organic end product, e.g. cis-cis-muconic acid, may be advantageously secreted from the host cell, so that its formation can be easily analysed and/or monitored by standard techniques of cell culture broth analysis, including chromatographic techniques such as HPLC.

### Downstream metabolization

The host cells used in accordance with the present invention may further be characterized in that they do not express genes that catalyze downstream metabolization of the desired organic end product. As the host cells are employed for production of a specific desired target compound, further processing and degradation of the same should advantageously be avoided.

As will be acknowledged by the skilled artisan, genes encoding downstream processing factors for a given organic product will typically be present in cells that are capable of processing said organic product. E.g., in *Pseudomonas putida,* the *catB* and *catC* genes encode enzymes that catalyze consecutive reactions in the catechol branch of the beta-ketoadipate pathway synthesis of 5-oxo-4,5-dihydro-2-furylacetate from catechol. Another *P. putida* gene catalyzing downstream metabolization of catechol is *pcaB* which converts cis, cis-muconic acid to carboxy muconolactone in the structurally related protocatechuate branch. Other host cells capable of processing catechol may also comprise functional *catB* and/or *catC* and/or *pcaB* genes that may advantageously be removed or "turned off" in order to allow for accumulation of cis-cis-muconate.

Particularly in the lignin processing method according to the invention, and in order to avoid further processing of the desired end product cis-cis-muconate, it is thus envisaged that the host cell does not express a functional *catB* polypeptide and/or that the host cell does not express a functional *catC* polypeptide and/or that the host cell does not express a functional *pcaB* polypeptide. It is therefore envisioned that said host cell does not comprise a functional *catB* gene and/or a functional *catC* gene and/or a functional *pcaB* gene, respectively.

The *catB* gene is in particular envisaged to encode a catB polypeptide having muconate cycloisomerase activity (EC 5.5.1.1), i.e. which is capable of synthesizing (S)-5-oxo-2,5-dihydro-2-furylacetate from cis-cis-muconic acid. An illustrative example of a catB polypeptide is the catB polypeptide of *Pseudomonas putida,* strain KT2240, with Uniprot accession No. Q88GK6 (version 67 of 22 July 2015). Said catB polypeptide may comprise a sequence corresponding to SEQ ID No. 27. An illustrative example of a *catB* gene is PP_3715 (SEQ ID No. 28). The term *catB* gene and *catB* polypeptide also comprises variants as defined elsewhere herein.

The *catC* gene is in particular envisaged to be encode a catC polypeptide having muconolactone Delta-isomerase activity (E.C. 5.3.3.4), i.e. which is capable of synthesizing 5-oxo-4,5-dihydrofuran-2-acetate from (S)-5-oxo-2,5-dihydrofuran-2-acetate. An illustrative example is the catC polypeptide of *Pseudomonas putida,* strain KT2240, with Uniprot accession No. Q88GK7 (version 67 of 22 July 2015). Said catC polypeptide may comprise a sequence corresponding to SEQ ID No. 29. An illustrative example of a *catC* gene is PP_3714 (SEQ ID No. 30). The term *catC* gene and catC polypeptide also comprises variants as defined elsewhere herein.

The *pcaB* gene is in particular envisaged to encode a pcaB polypeptide having 3-carboxy-cis,cis-muconate cycloisomerase activity, i.e. which is capable of synthesizing carboxy muconolactone from cis-cis-muconic acid. An illustrative example is the pcaB polypeptide of *Pseudomonas putida,* strain KT2240, with Uniprot accession No. Q88N37 (version 89 of 22 July 2015). Said pcaB polypeptide may comprise a sequence corresponding to SEQ ID No. 31. An illustrative example of a *pcaB* gene is PP_1379 (SEQ ID No. 32). The terms *"pcaB* gene" and "pcaB polypeptide" also comprises variants as defined elsewhere herein. A host cell "not comprising a functional *catB*/*catC*/*pcaBgene"* may either lack an endogenous catB/catC/pcaB gene, or it naturally comprises an endogenous *catB*/*catC*/*pcaB* gene, which is however silenced, preferably knocked-down or knocked-out, or deleted from the host cell chromosome. The skilled person is well aware of suitable methods for silencing endogenous genes, e.g. by manipulating the promoter region at a gene. It is preferred that the endogenous gene is knocked-down or knocked-out using by way of known methods, e.g. by recombinase techniques. Alternatively, the endogenous gene may be deleted from the chromosome by allelic substitution etc.

The term "silenced" is used herein to generally indicate that the expression of a gene is suppressed or inhibited as ascertainable e.g. by a reduced level of production or accumulation of the transcript or a processed product, for example of an mRNA, or of a translation product of the mRNA.

The level of expression of *catB*/*catC*/*pcaB* may be reduced by at least about 10%, by at least about 15%, by at least about 20%, by at least about 25%, at least about 30%, at least about 35%, at least about 40%, at least about 45%, at least about 50%, at least about 55%, at least about 60%, at least about 65%, at least about 70%, at least about 75%, at least about 80%, at least about 85% or more, including about 90% or more, about 95% or more including about 100%.

Genes encoding for downstream metaboliation of the desired organic products may for instance be knocked-out, i.e. made inoperable, resulting in an inhibition of gene expression, or translation of a non-functional protein product. Knock-out techniques are well-known in the art and include, e.g., introduction of one or more mutations into the *catB*/*catC*/*pcaB* gene, or into a regulatory sequence to which the respective gene is operably linked. Other methods include recombination techniques, e.g. resulting in the insertion of a foreign sequence to disrupt the gene or a deletion from the host cell's genome. Such a *catB*/*catC*/*pcaB* gene may be partially or fully inactivated, disrupted or otherwise blocked.

A knock-down of the *catB*/*catC*/*pcaB gene,* resulting in a reduced expression of said gene(s), is also conceivable. Several methods for gene knock-down are known in the art, and may involve either genetic modification or treatment with a reactant (the latter resulting in a transient knock-down). Genetic modifications resulting in a gene knock-down include, e.g., the incorporation of mutations into the target gene or a regulatory element operably linked thereto. In order to knock-down an endogenous gene, a heterologous molecule, such as a nucleic acid molecule, can be introduced into the host cell and upon introduction into a host cell reduces the expression of a target gene, typically through transcriptional and/or post-transcriptional silencing. Said reactant may be a nucleic acid molecule may be a silencing RNA, e.g. so-called "antisense RNA". Said antisense RNA typically includes a sequence of at least 20 consecutive nucleotides having at least 95% sequence identity to the complement of the sequence of the target nucleic acid, such as the coding sequence of the target gene, but may as well be directed to regulatory sequences of target genes, including the promoter sequences and transcription termination and polyadenylation signals. Other reactants useful for knock-down of target genes include small interfering RNAs (siRNAs), aptamers, Spiegelmers^{®}, nc-RNAs (including anti-sense-RNAs, L-RNA Spiegelmer, silencer RNAs, micro-RNAs (miRNAs), short hairpin RNAs (shRNAs), small interfering RNAs (siRNAs), repeat-associated small interfering RNA (rasiRNA), and molecules or an RNAs that interact with Piwi proteins (piRNA). Such non-coding nucleic acid molecules can for instance be employed to direct mRNA degradation or disrupt mRNA translation. A respective reactant, in particular RNA molecule, may in principle be directly synthesized within the host cell, or may be introduced into the host cell.

A different means of silencing exogenous DNA that has been discovered in prokaryotes is a mechanism involving loci called 'Clustered Regularly Interspaced Short Palindromic Repeats', or CRISPRs. Proteins called 'CRISPR-associated genes' (cas genes) encode cellular machinery that cuts exogenous DNA into small fragments and inserts them into a CRISPR repeat locus. When this CRISPR region of DNA is expressed by the cell, the small RNAs produced from the exogenous DNA inserts serve as a template sequence that other Cas proteins use to silence this same exogenous sequence. The transcripts of the short exogenous sequences are used as a guide to silence these foreign DNA when they are present in the cell.

Another technology involves the use of transcription activator-like effector nucleases (TALENs). TALENs are nucleases that have two important functional components: a DNA binding domain and a DNA cleaving domain. The DNA binding domain is a sequence-specific transcription activator-like effector sequence while the DNA cleaving domain originates from a bacterial endonuclease and is non-specific. TALENs can be designed to cleave a sequence specified by the sequence of the transcription activator-like effector portion of the construct. Once designed, a TALEN is introduced into a cell as a plasmid or mRNA. The TALEN is expressed, localizes to its target sequence, and cleaves a specific site. After cleavage of the target DNA sequence by the TALEN, the cell uses non-homologous end joining as a DNA repair mechanism to correct the cleavage. The cell's attempt at repairing the cleaved sequence can render the encoded protein non-functional, as this repair mechanism introduces insertion or deletion errors at the repaired site.

The capability of the host cell to degrade cis-cis-muconic acid to downstream products, in particular (S)-5-oxo-2,5-dihydro-2-furylacetate (in case of *catB* silencing or deletion) and/or 5-oxo-4,5-dihydrofuran-2-acetate (in case of *catC* silencing or deletion) and/or carboxy muconolactone (in case of *pcaB* silencing or deletion) may thus be reduced in comparison to a wild type cell, including entirely absent.

### Organic product

As will be readily understood by the skilled artisan, the nature and characteristics of the organic product obtained from the methods of the invention depends on the choice of organic educt, the obtained intermediate product and the biocatalyst contacted with said intermediate product to catalyze its conversion.

In the lignin processing method as described herein, it is envisaged to obtain cis-cis-muconic acid ((2Z,4Z)-2,4-Hexadienedioate, also referred to as muconate or cis-cis-muconate) according to formula (2) which can advantageously be used, e.g., as raw material for new functional resins, pharmaceuticals, and agrochemicals.

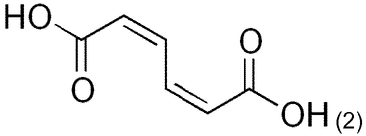

For example, cis-cis-muconic acid can be easily converted to adipic acid, caprolactam, and terephthalic acid which are used as a commodity chemical for production of value-added or valuable products including nylon-6 (fibers and resins), nylon-6,6, polyurethane, PVC, polyethylene terephthalate (PET), polyesters and/or polyamides. Furthermore, highly stereoregular polymers, useful functional resins, can be produced through topochemical polymerization of muconic acid esters. Verrucarin is an antibiotic that can be synthesized from cis-cis-muconic acid by organic synthesis.

It is in particular envisaged that cis-cis-muconic acid as obtained from the lignin processing method of the invention is white in colour, which is envisaged to greatly increase its economic value. Without wishing to be bound by theory, this advantageous property of the end product is thought to be due to its substantially complete chemical conversion from catechol.

### Recovery and purification

In the methods of the invention, an organic product is recovered. E.g., the organic product(s), e.g. cis-cis-muconic acid, is secreted by the biocatalyst, in particular a bacterial host cell, so that recovering the fermentation/culture medium includes recovering the organic product(s). Further the method may include a step of purifying the organic product(s). Purification of the organic product(s) preferably results in an increased concentration of organic product(s) compared to the starting solution and may include membrane filtration, for example for clarification, buffer exchange or concentration purposes, filtration or dialysis, which may e.g. be directed at the removal of molecules below a certain molecular weight, or a precipitation using organic solvents or ammonium sulphate. In lignin processing as described herein, to extract cis-cis-muconic acid, the cell culture medium can be acidified. At a pH of 2.5 the solvability of cis, cis-muconate in water is <5% or at a pH of 2.0 measured at 25°C the solvability of cis, cis-muconate in water is 1%. After the acidification the insoluble product may sediment over time. Subsequently the supernatant can be discarded. To reduce the salt concentrations the product may be washed several times with water, after which a pulver can be produced by spray drying. The product (cis-cis-muconic acid) obtained by lignin processing as described herein is of high purity and white in color. Chromatography may for example be carried out in the form of a liquid chromatography such as capillary electrochromatography, HPLC (high performance liquid chromatography) or UPLC (ultrahigh pressure liquid chromatography) or as a gas chromatography. The chromatography technique may be a process of column chromatography, of batch chromatography, of centrifugal chromatography or a method of expanded bed chromatography, as well as electrochromatographic, electrokinetic chromatography. It may be based on any underlying separation technique, such as adsorption chromatography, hydrophobic interaction chromatography or hydrophobic charge induction chromatography, size exclusion chromatography (also termed gel-filtration), ion exchange chromatography or affinity chromatography and may also be a method of capillary gas chromatography. Another example of a purification is an electrophoretic technique, such as preparative capillary electrophoresis including isoelectric focusing. Examples of electrophoretic methods are for instance free flow electrophoresis (FFE), polyacrylamide gel electrophoresis (PAGE), capillary zone or capillary gel electrophoresis. An isolation may include may include the combination of similar methods.

### Host cell

In accordance with the foregoing, a host cell for the production of cis,cis-muconic acid from catechol is provided herein, said host cell comprising at least one (optionally heterologous) *catA* gene as defined elsewhere herein and at least one (optionally heterologous) *catA2* gene as defined elsewhere herein. The *catA* gene may in particular be PP_3713 of P. putida KT2440 and comprise a sequence corresponding to SEQ ID No. 2 or a variant thereof, and the *catA2* gene may in particular be PP_3166 of *P. putida* KT2440 and comprise a sequence corresponding to SEQ ID No. 4 or a variant thereof. Said host cell may further comprise at least one (optionally heterologous) promoter sequence operably linked to, e.g. upstream of, the (optionally heterologous) *catA* gene, the (optionally heterologous) *catA2* gene, or both. Said promoter sequence is envisaged to comprise a sequence selected from a sequence corresponding to SEQ ID No. 5, SEQ ID NO. 6, SEQ ID No. 7, SEQ ID No. 8, SEQ ID No. 9 SEQ ID No. 10, SEQ ID No. 11, SEQ ID No. 12, SEQ ID No. 13, SEQ ID No. 14, SEQ ID No. 15, or SEQ ID No. 16, or SEQ ID No. 88, or SEQ ID No. 89, or SEQ ID No. 90, or SEQ ID No. 91, or SEQ ID No. 92, or SEQ ID No. 93, or SEQ ID No. 94, or SEQ ID No. 95, or SEQ ID No. 96, or SEQ ID No. 97, or SEQ ID No. 98, or SEQ ID No. 99, or SEQ ID No. 100, or SEQ ID No. 101, or SEQ ID No. 102, or SEQ ID No. 103, or SEQ ID No. 104, or SEQ ID No. 105. The host cell may in particular be characterized in that it does not comprise a functional *catB* gene; a functional *catC* gene and/or a functional *pcaB* gene. The host cell may be selected from any type of host cell as described herein, including bacteria, yeast, filamentous fungi, cyanobacteria, algae, and plant cells. The host cell is in particular be envisaged to be selected from *Pseudomonas* spec., e.g. the host cell may be *Pseudomonas putida.* Otherwise, if the host cell is a recombinant host cell comprising heterologous nucleic acid sequences, in particular heterologous *catA* and/or heterologous *catA2* genes, said genes are preferably derived from *Pseudomonas putida.* The host cell may comprise further (optionally heterologous) genes that enable utilization of by-products, e.g. AroY, KpdB, pheA and/or pcmh as described elsewhere herein. The skilled person will readily acknowledge that all details provided in the context of the methods of the invention apply to the host cell provided herein, *mutatis mutandis.*

### Lignin processing

The present invention relates to means and methods for converting organic compounds into preferably useful organic end products. One particularly preferred field of application is the valorization of lignin. Lignin processing according to the invention may preferably be achieved as follows:

### (1) Hydrothermal conversion of lignin

Lignin (for example, guaiacol, alkali lignin namely kraft lignin, and organosolv lignin) is subjected to hydrothermal conversion (i.e. supercritical-water assisted conversion). A preferred protocol for hydrothermal conversion has been described elsewhere herein and is also set out in the appended examples. Briefly, lignin is subjected to conversion in sub- and supercritical water at a temperature between about 350°C-420°C (e.g. about 380°C) and a pressure of 22 mPa-40mPa, such as 23, 24, 25, 26, 27, 28, 29, 30, 31, 32, 33, 34, 35, 36, 37, 38, or 39 mPa. A suitable retention time is 0-160 minutes or preferably 0-60 minutes. Further parameters for sub- and supercritical water for the decomposition of lignin are disclosed by Wahyudiono et al (Chemical Engineering and Processing; 2008, vol. 47, p. 1609-1619) resulting in the generation of more than 28 wt% catechol.

### (2) Intermediate product

After conversion is completed, a reaction product is obtained that comprises catechol as an intermediate product. The catechol yield is preferably envisaged to exceed 5 % w/w, 10 % w/w, 15% w/w, 20% w/w, 25% w/w, 30% w/w. Other potential by-products comprise (m-, p-, o-)cresol, phenol and guaiacol. Catechol is recovered from the reaction product using suitable measures, e.g. steam bath distillation. After distillation, the amount of catechol is preferably higher than 90% w/w, higher than 95% w/w or higher than 99% w/w.

### (3) Biokatalytic conversion

Subsequently, a suitable biocatalyst is employed, using catechol as a substrate to generate cis-cis-muconic acid. Said biocatalyst is preferably a host cell as described in the foregoing. Said host cell preferably comprises at least one (optionally heterologous) *catA* gene as defined elsewhere herein and at least one (optionally heterologous) *catA2* gene as defined elsewhere herein. The *catA* gene may in particular be PP_3713 of *P. putida* KT2440 or a variant thereof and comprise a sequence corresponding to SEQ ID No. 1, and the *catA2* gene may in particular be PP_3166 of *P. putida* KT2440 or a variant thereof and comprise a sequence corresponding to SEQ ID No. 3. Said host cell may further comprise at least one (optionally heterologous) promoter sequence operably linked to, e.g. upstream of, the (optionally heterologous) *catA* gene, the (optionally heterologous) *catA2* gene, or both. The promoter preferably enables constitutive expression of the genes operably linked thereto. Said promoter sequence is envisaged to comprise a sequence selected from a sequence corresponding to SEQ ID No. 5, SEQ ID NO. 6, SEQ ID No. 7, SEQ ID No. 8, SEQ ID No. 9 SEQ ID No. 10, SEQ ID No. 11, SEQ ID No. 12, SEQ ID No. 13, SEQ ID No. 14,SEQ ID No. 15, or SEQ ID No. 16, or SEQ ID No. 88, or SEQ ID No. 89, or SEQ ID No. 90, or SEQ ID No. 91, or SEQ ID No. 92, or SEQ ID No. 93, or SEQ ID No. 94, or SEQ ID No. 95, or SEQ ID No. 96, or SEQ ID No. 97, or SEQ ID No. 98, or SEQ ID No. 99, or SEQ ID No. 100, or SEQ ID No. 101, or SEQ ID No. 102, or SEQ ID No. 103, or SEQ ID No. 104, or SEQ ID No. 105. The host cell may in particular be characterized in that it does not comprise a functional *catB* gene; a functional *catC* gene and/or a functional *pcaB* gene. The host cell may be selected from any type of host cell as described herein, including bacteria, yeast, filamentous fungi, cyanobacteria, algae, and plant cells. The host cell is in particular be envisaged to be selected from *Pseudomonas* spec., e.g. the host cell may be *Pseudomonas putida.* Otherwise, if the host cell is a recombinant host cell comprising heterologous nucleic acid sequences, in particular heterologous *catA* and/or heterologous *catA2* genes, said genes are preferably derived from *Pseudomonas putida.* The host cell may comprise further (optionally heterologous) genes that enable utilization of by-products such as protochatechuate, phenol and/or cresol, e.g. AroY, KpdB, pheA and/or pcmh as described elsewhere herein.

The host cell is contacted with the substrate under conditions rendering conversion of catechol to cis-cis-muconic acid feasible. After the reaction is completed, cis-cis-muconic acid is recovered from the cell culture medium. Lignin processing as described in the foregoing enables to obtain cis-cis-muconic acid in high amounts and at high reaction rates. The product is also of high purity, and is typically white in color.

### Example 1: Strain development and cultivation conditions

### Strain and cultivation conditions

The bacterial strains used in this study are listed in Table 1. Unless otherwise stated bacteria were usually grown in LB (10 g/l tryptone, 5.0 g/l yeast extract, 5 g/l NaCl, dissolve in H₂O and autoclave). Batch cultivations were done in Erlenmeyer flasks that were shaken at 200 rpm. *Escherichia coli* cells were grown at 37°C while *Pseudomonas putida* was cultured at 30°C. Selection of *P. putida* cells was performed by plating onto M9 minimal medium with citrate (2 g/L) as a sole carbon source. The following four stock solutions were prepared and autoclaved separately: 10× stock solution of M9: weight 42.5 g Na₂HPO₄ 2H₂O, 15 g KH₂PO₄, 2.5 g NaCl and 5 g NH₄Cl and dissolve in 500 ml of H₂O, 120.37 g/L MgSO₄, 200 g/L citrate (as selective carbon source for *Pseudomonas*)*,* and an 16 g/L agar solution. The components were diluted in sterile water to final concentrations of 1× M9 salts, 0.24 g/L MgSO₄, 20 g/l citrate and where required, 14 g/L agar. If needed, additionally antibiotics were added at the following final concentration: ampicillin (Amp) 100 µg/ml for *E. coli* cells and at 500 µg/ml for *P. putida;* kanamycin (Km) 50 µg/ml. Other supplements were added in following concentrations: 5-bromo-4-chloro-3-indolyl-β-D-galactopyranoside (X-gal) 80 µg/ml; isopropyl-β-D-1-thiogalactopyrano side (IPTG) 0.48 g/L; 3-methyl-benzoate (3MB) 2.25 g/L; 102.69 g/L sucrose. The cultivation of *P. putida* or *E. coli* cells were monitored during growth by OD₆₀₀ using UV-1600PC Spectrophotometers (Radnor, Pennsylvania, USA).

### Strain development and Cloning Targeting Sequence into pEMG

The pEMG plasmid was generally used to perform modifications in the genome of P. putida KT2440. The procedure is based on the homologous recombination forced by double-strand breaks in the genome of the *P. putida* after cleavage in vivo by I-Scel. (encoded on the plasmid pSW-I). Transient expression of the nuclease is controlled in pSW-I by Pm, a promoter induced in presence of 3-methylbenzoate-inducible. The deletion of catB/catC and pcaB (KT2440 JD2S and BN14, respectively), the integration of Pem7, Pem7* upstream of catA (BN6 an BN12, respectively), catA2 downstream of Pcat:catA (BN15) and a copy of Pcat:catA:catA2 (BN18 and BN19) in P. putida were performed one after another as follows:

For the genetic modifications of P. putida KT2440 JD1 and JD2S, KT2440 BN6-BN19 (table 1) the upstream (TS1) and downstream (TS2) regions flanking the region to be deleted/inserted and the insertion Pem7, Pem7*, catA2 and Pcat: catA: catA2 were amplified separately using Phusion High-Fidelity Polymerase (Thermo Fisher) (Primer listed in table 3). For the deletion of ΔcatB/catC and pcaB, the resulting products (TS1 and TS2) were joined together by using Gibson assemply. For the deletion of ΔcatB/catC and pcaB, the resulting products (TS1 and TS2) were joined together by using Gibson assemply. The fused fragment was ligated into Smal linearized plasmid pEMG, resulting in pEMG-AcatB/catC and pEMG-ΔpcaB -for the construction of KT2440 JD2S and KT2440 BN14. For the insertion of Pem7 and Pem7* upsteam of catA, catA2 downstream of catA and the copy of Pcat: catA: catA2, TS1, TS2 and the to be inserted fragments were ligated into Smal digested pEMG via Gibson assembly. Each of the resulting plasmids pEMG-ΔcatB/catC, pEMG-ΔpcaB, pEMG-Pem7, pEMG-Pem7*, pEMG-catA2 and pEMG-Pcat:catA:catA2 were transformed separately into E. coli DH5αλpir via electroporation and the culture was plated onto LB-Km plates supplemented with Xgal and IPTG to discriminate potential positive clones by visual screening.Putative positive clones were checked for the presence of the TS1/TS2 insertions by colony PCR using pEMG-F/pEMG-R (see table 3) and confirmed by sequencing the corresponding plasmids. The pEMG derivates were isolated form *E. coli* DH5αλ*pir* with Miniprep Kit (Quiagen) and transformed into *E. coli* CC118I*pir* for the delivery into *P. putida* KT2440 via mating as described in (de Lorenzo and Timmis, Methods Enzymol., 1994; 235:386-405; Martinez-Garcia and de Lorenzo, Environ Microbiol., 2011, 13(10):2702-16).

The bacterial mixtures were resuspended in 10 mM MgSO4 and appropriate dilutions plated onto M9 citrate plus kanamycin. Since pEMG-derived plasmids cannot proliferate in *P. putida,* KmR clones raised after conjugation can grow only by co-integration of the construct in the genome of the recipient strain. The delivery of the pSW-I plasmid into competent *P. putida* was done by electroporation of 50 ng of pSW-I in the Km resistant cells as described in (Martinez-Garcia and de Lorenzo, Environ Microbiol., 2011, 13(10):2702-16) and plated onto LB-Km 50 µg/ml + Amp 500 µg/ml. The induction of the I-Scel enzyme in cointegrated clones that harbors the pSW-I plasmid was started by adding 15 mM 3-methylbenzoate in a 5 ml LB-Amp medium. The culture was incubated for 14 h at 30 °C and plated on LB-Amp 500 plates. The loss of cointegrated plasmid were checked by selecting kanamycin sensitive clones on LB-Kan 50 µg/ml plates. Deletions and insertions into the genome were generally confirmed by PCR with primer that hybridize upstream of TS1-F and downstream of TS2-R in the genome. The curation of pSW-I from P. putida was achieved by several passages of the deleted clone in LB without antibiotics.

### Strain development and Cloning Targeting Sequence into pJNNmod

The pJNNmod plasmid was used for the episomal expression of *catA* under control of PGro and PGro_2 in *P. putida* BN15. The construction of pJNNmod-PGro:*catA* and pJNNmod-PGro_2:catA leading to BN20 and BN21, respectively, was performed as follows:

For the genetic modifications of BN20 and BN21 (table 1) *catA* and the promoter PGro and PGro_2 were amplified separately using Phusion High-Fidelity Polymerase (Thermo Fisher) (Primer *catA*-F and *catA*-F: *catA* : TS1_catB/C-F/TS1_catB/C-R; PGro-F and PGro-R: PGro and PGro_2; listed in table 3).For the insertion of *catA* with each promoter (PGro and PFro_2) in the plasmid pJNNmod, the fragments were ligated into Smal digested PJNNmod via Gibson assembly. Each of the three resulting plasmids pJNNmod-Gro:*catA* and pJNNmod-Gro_2:catA were transformed separately into *E. coli* DH5αλ*pir* via electroporation and the culture was plated onto LB-Amp plates. Putative positive clones were checked for the presence of the promoter/*catA* insertions by colony PCR using pJNNmod-F/pJNNmod-R (see table 3) and confirmed by sequencing the corresponding plasmids.

**Table 1. Strains**

| **Strain** | **Description/relevant characteristics** | **Reference** |
|---|---|---|
| *E. coli* | | |
| DH5α | *supE44, ΔlacU169 (φ80 lacZΔM15), hsdR17 (rk-mk+), recA1, endA1, thi1, gyrA, relA* | Hanahan and Meselson (Methods Enzymol. 1983; 100:333-42) |
| DH5αλpir | λ*pir* lysogen of DH5α | Martinez-Garcia and de Lorenzo (Environ Microbiol. 2011; 13(10):2702-16) |
| CC118 | Δ(*ara-leu*), *araD,* Δ*lac*X174, *galE, galK, phoA, thi1, rpsE, rpoB, argE (Am), recA1,* lysogenic λ*pir* | de Lorenzo and Timmis (Methods Enzymol. 1994; 235:386-405) |
| HB101 | SmR, *hsdR-M+, pro, leu, thi, recA* | Sambrook et al. (Molecular cloning. A laboratory manual, 2nd Ed. New York: Cold spring harbor laboratory press, 1989) |
| *P. putida* | | |
| KT2440 | mt-2 derivative cured of the TOL plasmid pWW0 | Bagdasarian et al. (Gene. 1981 ,16(1-3):237-47) |
| JD2S | KT2440 Δ*catB*/*C* | unpublished |
| BN6 | KT2440 Δ*catB*/*C* Pem7:catA | unpublished |
| JD1 | KT2440 Δ*catR* | Van Duuren et al. (J Biotechnol. 2011;156(3):163-72) |
| BN12 | KT2440 Δ*catB*/*C* Pem7*:catA | unpublished |
| BN14 | KT2440 Δ*catB*/*C* Δ*pcaB* Pem7:catA | unpublished |
| BN15 | KT2440 Δ*catB*/*C Pcat:catA:catA2* | unpublished |
| BN18 | JD2 *Pcat:catA:catA2* | unpublished |
| BN19 | BN15 *Pcat:catA:catA2* | unpublished |
| BN20 | pJNNmod-PGro:catA | unpublished |
| BN21 | PJNNmod-PGro_2:*catA* | unpublished |

**Table 2. Plamids**

| **Plamids** | **Genome** | **Reference** |
|---|---|---|
| pSW-I | ApR, oriRK2, xyIs, PmIscel (transcriptional fusion of I-scel to Pm) | Wong and Mekalanos (Proc Natl Acad Sci U S A., 2000; 97(18):10191-6) |
| pEMG | KmR, *ori*R6K, *lacZ*α with two flanking I-Scel sites | Martinez-Garcia and de Lorenzo (Environ Microbiol. 2011; 13(10):2702-16) |
| pRK600 | CmR; oriColE1, RK2 mob+, tra+ | de Lorenzo and Timmis (Methods Enzymol. 1994; 235:386-405) |
| pSEVA247C | neo,R, pRO1600/ColE1, CFP | Silva-Rocha et al. (Nucleic Acids Res. 2013 Jan; 41) |
| pSEVA247R | neo,R, pRO1600/ColE1, RFP | Silva-Rocha et al. (Nucleic Acids Res. 2013 Jan; 41) |
| pJNNmod | P_{TAC}, *laclq,* ColE1 origin of replication | Rodrigues et al. (Metab Eng. 2013; 20:29-41) |

**Table 3**

| **Primer** | **Sequence** | **Function** | **SEQ ID NO:** |
|---|---|---|---|
| pEMG-F | CCATTCAGGCTGCGCAACTGTTG | Vector primer pEMG | 37 |
| pEMG-R | CTTTACACTTTATGCTTCCGGC | Vector primer pEMG | 38 |
| pSW-F | GGACGCTTCGCTGAAAACTA | Check of plasmid curation | 39 |
| pSW-R | AACGTCGTGACTGGGAAAAC | Check of plasmid curation | 40 |
| Check-F | GGCACATCGAACACGCTGTAGTTG | Confirm *catB*/*C* deletion | 41 |
| Check-R | CCTCCAGGGTATGGTGGGAGATTC | Confirm *catB*/*C* deletion | 42 |
| TS1_catB/C-F | TGAACGCTTCGCCAGCCAACT ACCTTCGCCAGCC | Amplification TS1 *catB*/*C* | 43 |
| TS1_catB/C-R | GCTCGATACCCAGGCCAGCAGGCCAGCA | Amplification TS1 *catB*/*C* | 44 |
| TS2_catB/C-F | CATATGTGTTGCCAGGTCCCGTCAGGTC | Amplification TS2 *catB*/*C* | 45 |
| TS2_catB/C-R | AAAAACATATGCAGCTCAAGGCCGACGAAAAG G | Amplification TS2 *catB*/*C* | 46 |
| TS1_Pem7-F | TGAATTCGAGCTCGGTACCCTGGGCGATGTGC AGCAGCTC | Amplification TS1 Pem7 | 47 |
| TS1_Pem7-R | CGATGATTAATTGTCAACAACGTGCTTACCTCG TATTGTTC | Amplification TS1 Pem7 | 48 |
| TS2_Pem7-F | TTAAAGAGGAGAAATTAAGCATGACCGTGAAA ATTTCCCACA | Amplification TS2 Pem7 | 49 |
| TS2_Pem7-R | GTCGACTCTAGAGGATCCCCTCGAAGTACGAA TAGGTGCCC | Amplification TS2 Pem7 | 50 |
| Pem7-F | GCCTGACAAGAACAATACGAGGTAAGCACGTT GTTGACAATTAATCATCGG | Amplification of Pem7 | 51 |
| Pem7-R | GTCGGCAGTGTGGGAAATTTTCACGGTCATGC TTAATTTCTCCTCTTTAACCTAGGG | Amplification of Pem7 | 52 |
| Ptuf_s-F | CAAGCTTAGGAGGAAAAACAAACTGGAAGCG GTGTCAAAG | Amplification short Ptuf | 56 |
| Ptuf_s-R | TCCTCGCCCTTGCTCACCATGCTTAATTTCTCCT CTTTGTGGCCGGCATTCTATTTGTC | Amplification short Ptuf | 57 |
| Ptuf_sM-F | AACTGGAAGCGGTGTCAAAGC | Mutagenesis short Ptuf | 58 |
| Ptuf_sM-R | GTGGCCGGCATTCTATTTG | Mutagenesis short Ptuf | 59 |
| Ptuf-F | CAAGCTTAGGAGGAAAAACACCGCTTCACAGG GAACACCA | Amplification short Ptuf | 60 |
| Ptuf-R | CCTCGCCCTTGCTCACCATCGATACAATCCTCC GCAGAAG | Amplification short Ptuf | 61 |
| Ptuf_M-F | CCGCTTCACAGGGAACAC | Mutagenesis Ptuf | 62 |
| Ptuf_M-R | CGATACAATCCTCCGCAGAAG | Mutagenesis Ptuf | 63 |
| PGro-F | CAAGCTTAGGAGGAAAAACAGAAGGACCGGG GCCGCGCAA | Amplification of PGroES | 64 |
| Pgro-F | TCCTCGCCCTTGCTCACCATTGTCGATCTCTCCC AAATTG | Amplification of PGroES | 65 |
| TS1_pcaB-F | TGAATTCGAGCTCGGTACCCACACCGCGGGCA TGACCGCC | Amplification TS1 for pcaB deletion (BN14) | 66 |
| TS1_pcaB-R | GTGCGCCACAGCGGTCTCCTGCAGCGTCCTTA ATCATCAT | Amplification TS1 for pcaB deletion (BN14) | 67 |
| TS2_pcaB-F | ATGATGATTAAGGACGCTGCAGGAGACCGCTG TGGCGCAC | Amplification TS2 for pcaB deletion (BN14) | 68 |
| TS2_pcaB-R | GTCGACTCTAGAGGATCCCCCTGGGCAAAGCC CGGGGTGA | Amplification TS2 for pcaB deletion (BN14) | 69 |
| TS1_catA2-F | TAATCTGAATTCGAGCTCGGTACCCCGTTGGCC GGTGCCACCGTC | Amplification TS1 catA2 Integration (BN15) | 70 |
| TS1_catA2-R | GTTCACGGTCATGCTTAATTTCTCCTCTTTTCAG CCCTCCTGCAACGCCC | Amplification TS1 catA2 Integration (BN15) | 71 |
| TS2_catA2-F | GTTCGAGGTTATGTCACTGT | Amplification TS2 catA2 Integration (BN15) | 72 |
| TS2_catA2-R | TGCAGGTCGACTCTAGAGGATCCCCGGCGGGC AGATCCTGTGCGTAG | Amplification TS2 catA2 Integration (BN15) | 73 |
| CatA2-F | GGGCTGAAAAGAGGAGAAATTAAGCATGACC GTGAACATTTCCCA | Amplification catA2 (BN15) | 74 |
| CatA2-R | AAATCACAGTGACATAACCTCGAACTCAGGCC TCCTGCAAAGCTC | Amplification catA2 (BN15) | 75 |
| TS1_Pcat:catA/2 -F | TAATCTGAATTCGAGCTCGGTACCCCGCGCCTG AACGCCGGGCAG | Amplification TS1 for Pcat:catA:catA2 Integration (BN18/19) | 76 |
| TS1_Pcat:catA/2 -R | TCTCCCACCATACCCTGGAGGTCTGACACACCA TGCCCACAGGGG | Amplification TS1 for Pcat:catA:catA2 Integration (BN18/19) | 77 |
| TS2_Pcat:catA/2 -F | GCCGCGAGCTTTGCAGGAGGCCTGATCATATG GCCTGTTGCTCGA | Amplification TS2 for Pcat:catA:catA2 Integration (BN18/19) | 78 |
| TS2_Pcat:catA/2 -R | TGCAGGTCGACTCTAGAGGATCCCCTGACCAC CTTGCAACAGGTG | Amplification TS2 for Pcat:catA:catA2 Integration (BN18/19) | 79 |
| Pcat:catA/2-F | CAGACCTCCAGGGTATGGTG | Amplification of Pem7 | 80 |
| Pcat:catA/2-R | TCAGGCCTCCTGCAAAGCTC | Amplification of Pem7 | 81 |
| catA-F | GTCGACTCTAGAGGATCCCCTCAGCCCTCCTGC AACGCCC | Amplification of catA (BN20/21) | 82 |
| catA-R | ATGACCGTGAAAATTTCCCA | Amplification of catA (BN20/21) | 83 |
| PGro-F | ATATGTCGAGCTCGGTACCCGAAGGACCGGGG CCGCGCAA | Amplification of PGro and PGro_2 (BN20/21) | 84 |
| PGro-R | TGGGAAATTTTCACGGTCATTGTCGATCTCTCC CAAATTG | Amplification of PGro and PGro_2 (BN20/21) | 85 |
| pJNN-F | CGCGAATTGCAAGCTGATCC | Check primer forward | 86 |
| PJNN-R | CTCTCATCCGCCAAAACAGC | Check primer reverse | 87 |

| **construct** | **SEQ ID NO:** |
|---|---|
| pEMG-ΔcatB catC | **53** |
| pEMG-ΔpcaB | **54** |
| pEMG-pEM7 | **55** |

### Step-wise strain optimization towards higher catechol conversion rates

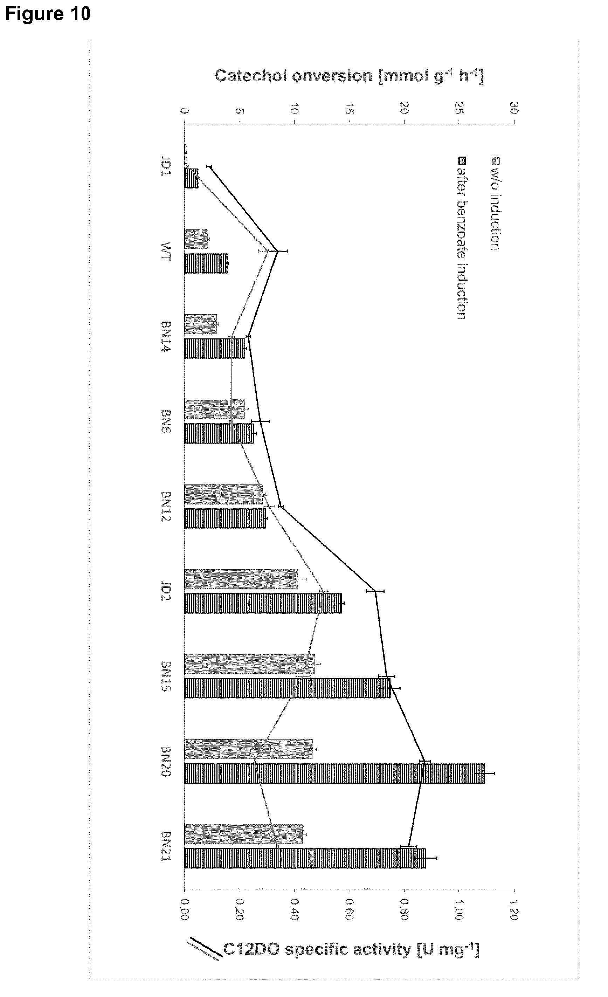

Strains were grown on E2 minimal medium in the absence or presence of 5 mM benzoic acid. At an optical cell density (600 nm) of 0.5, 2.5 mM of catechol was added to the medium. Catechol conversion was monitored in 10 min intervals via HPLC. Conversion rates are reported in mmol catechol per gram dry cell weight per hour (mmol gDCW-1 h-1, (figure 10).

Crude extracts were obtained via centrifugation and homogenization of cell pellets using silica beads. Catechol 1,2-dioxygenase (C12DO) activity was monitored after addition of 20 µM catechol in 30 mM Tris-HCl buffer (pH 8.2) at 260 nm corresponding to formation of cis,cis-muconic acid (ε = 16,800 M-1 cm-1) as described previously (Jiménez et al., Environ Microbiol. 2014 Jun; 16(6):1767-78). Total protein was determined using a BCA protein assay kit and a BSA standard. One unit U corresponds to the conversion of 1 µmol of catechol per minute (figure 10).

By the extra homologous expression of catA and catA2 under the control of the Pcat promoter specific in vitro C12DO activity, as well as the cells ability to convert toxic catechol into cis,cis-muconic acid could be strongly increased (figure 10). The effect is most pronounced when cells are additionally induced by the supplementation of benzoic acid.

### Application of promoter with higher activity to increase the catechol conversion rates in P. putida

Increased production performance of P. putida production strains caused by a promoter upstream of catA with increased promoter activity could be demonstrated in P. putida BN6 (SEQ ID No. 5 [Pem7]) with a conversion rate of 5.5 mmol g-1 h-1 versus BN12 (SEQ ID No. 5 [Pem7*] with a conversion rate of 7.11 mmol g-1 h-1. Hence, Pem7* can be applied as heterologous promoter in P. putida to express genes like catA at a high level leading to an significant increase in the catechol conversion rate compared to the original promoter (see figure 10).

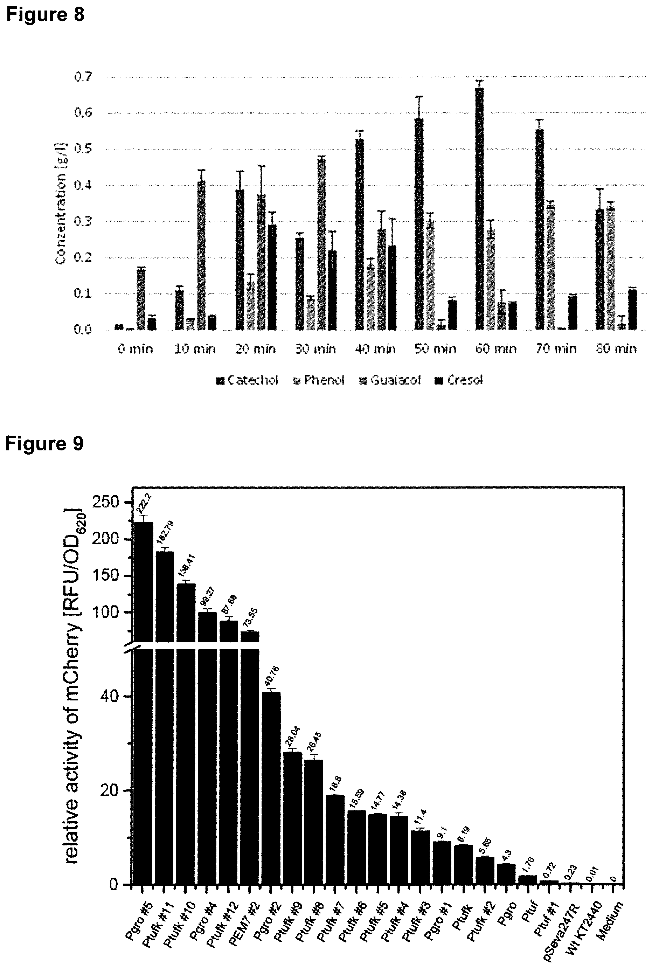

To proof the functionality of homologous promoter created within the promoter library, the native promoter Pgro (SEQ ID No. 101 [Pgro]) and a mutated version of Pgro (SEQ ID No. 102 [Pgro_1]) with almost double promoter activity (figure 9), was cloned episomally upstream of catA and integrated in P. putida BN15. The expression of catA under control of a much higher active version of Pgro resulted in a significantly increased catechol conversion rate compared to the native promoter (Pgro: 8.24 mmol g-1 h-1; Pgro_1: 15.08 mmol g-1 h-1).

The promoter library consisting of several homologue and heterologous promoter variants displayed a broad range of activities (~2% to > 5000%). Thereby, a fine-tuning of gene expression in *P. putida* was possible and demonstrated by stable genomic integration of Pem7 and Pem7* and episomal expression of *catA* using Pgro and Pgro_1. In both cases, the higher promoter activity was applicable to an improved product formation of *cis, cis-*muconate from catechol.

### Example 2: Hydrothermal conversion and distillation, cultivation of biocatalysts

### Materials and Methods

### Hydrothermal Conversion

The hydrothermal conversion of commercial available guaiacol (Cas Number: 90-05-1) (Sigma-Aldrich, USA), kraft lignin (Cas Number 8068-05-1) (Sigma-Aldrich, USA), kraft lignin (ECN, Netherlands), organosolv lignin (ECN, Netherlands), kraft lignin (Cas Number 9005-53-2) (TCI, Deutschland), IndulinAT (Cas Number 8068-05-1) (S3Chemicals, Germany), and organosolv lignin (Fraunenhofer Centre for Chemical-Biotechnological Processes, Germany) was performed in a 4575A-type batch reactor of 500 mL (Parr, USA). An overview of the experiments is shown in table 4.

**Table 4: Overview of the experiments**

| **Experiment Number** | **Substrate type** | **Substrate mass [g]** | **Water mass [g]** | **Temperature [°C]** | **Reaction time [min]** |
|---|---|---|---|---|---|
| 4 | Guaiacol (Sigma-Aldrich, USA) | 47 | 250 | 383 | 30 |
| 5 | Kraft-Lignin (Sigma-Aldrich, USA) | 28.3 | 150 | 383 | 30 |
| 6 | Kraft-Lignin (ECN, the Netherlands) | 5 | 250 | 383 | 30 |
| 7 | Organosolvent Lignin (ECN, the Netherlands) | 5 | 250 | 383 | 30 |
| 8 | Organosolvent Lignin (ECN, the Netherlands) | 5 | 250 | 383 | 30 |
| 9 | Kraft Lignin (Sigma-Aldrich, USA) | 5 | 250 | 383 | 30 |
| 10 | Kraft Lignin (Sigma-Aldrich, USA) | 5 | 250 | 383 | 30 |
| 16 | Kraft Lignin (TCI, Germany) | 5 | 350 | 350 | 45 |
| 17 | IndulinAT (S3Chemicals, Germany) | 5 | 250 | 383 | 60 |
| 21 | Organosolvent Lignin (Fraunhover-CBP. Germany) | 5 | 250 | 395 | 60 |

For experiment 9 and 10 degassed water was used. In experiment 10 and 17 NaCl (5 g) was added to the reactor.

The reactor was loaded with the suspension, closed and purged 5 times with nitrogen. Subsequently, the reactor was heated up to the desired temperature of either 383 °C and 24 MPa with the addition of 5 g NaCl, or 383°C and 25 MPa without the addition of NaCl, while being stirred with 150 - 400 rpm. Additionally, the reactor was heated to 350 °C, 383 °C and 395 °C in experiment 16, 17, and 21, which lead to the particular pressures of 24, 23.5 and 30 MPa, respectively. The heat-up time was about 1 hour, the final temperature was held for 30 - 60 minutes, and the cooled down time was about 1.5 hours. From experiment 8 the reactor was cooled down to 50 °C within 30 minutes using the inner cooling coil and a fan. The reactor was again purged with nitrogen (3 times), after which the liquid content was transferred in an argon-purged bottle and stored at -20 °C. The reactor was rinsed with methanol to a total volume of 300 ml. Solids and liquids were separated by centrifugation (10000 xg, 5 min., at room temperature).

### Following the hydrothermal conversion the liquid phase of the reactor was either used for concentration or distillation.

The liquid phase was concentrated in a vacuum evaporator (AVC 2-33 IR, Christ, Germany). The evaporator was heated up for 15 min before loading. The evaporation process lasted for 3 hours at 40 °C and a reduced pressure of 15 mbar. The resulting concentrate was stored at -20 °C.

For the steam bath distillation, 75 mL of the liquid content from the hydrothermal conversion were filled into a 500 mL round-bottomed flask with some boiling granules. The flask was placed in an oil-bath that was heated to 100 °C - 130 °C. The steam for the distillation process was generated by boiling water in a 5 liter flask. The distillate was cooled down and collected in a 1 liter flask. The distillation process was carried out in 3 hours. The residue from the 500 mL flask was transferred in an Argon-purged bottle and stored at -20 °C.

### Hydrothermal conversion with small scale reactors

Small-scale hydrothermal conversion experiments were conducted in batch reactors made of stainless steel 1.4571 with a top and bottom cap (Swagelok, USA). Total volume of the reactors was 5 mL (length 100 mm, inner diameter 8 mm, outer diameter 12 mm).

Two different lignins were used for the experiments. Either Kraft lignin from Sigma Aldrich, USA or Kraft lignin from TCI, Germany. 0.1 g was loaded into each reactor together with pure water, ranging from 0.25 to 0.50 g/cm³ water density. Optionally, other components (e.g. NaCl or NaOH) were added.

The reactors were purged with nitrogen or argon as inert gas, sealed and incubated in a preheated sand bath in an oven (Nabertherm, Germany) installed at 300 or 400 °C. After the desired reaction time plus 15 min for heat up, which was measured once with a thermocouple in one reactor, the reactors were quickly quenched in a water bath.

The content of each reactor was collected and the reactor was rinsed with methanol to a total volume of 10 ml. Solids and liquid were separated by centrifugation (10000 xg, 5 min at room temperature).

The amount of remaining ash was determined by weighing the centrifuged pellets after 24 h.

All experiments were conducted in triplicates.

### Analytics

Concentrations of catechol, phenol, guaiacol and *o*-, *p-, m*- cresol in the liquid phase were measured by HPLC analysis via an Agilent, USA system with either a Gemini, USA 5 µm column (150x4.6 mm) or a PurospherSTAR, USA column and 0.025 % H₃PO₄ in pure acetonitrile as eluent at a temperature of 25 °C at 210 nm. *cis, cis*-Muconic acid was analyzed with the same system and setup but at 260 nm.

### Cultivation

For the production of *cis, cis*-Muconic acid the strain BN6 was used for the experiments 4,5, 8, 16 and 17. Cultivation was done in E-2 minimal medium (Table 5) with glucose (pH 7) consisting of the following ingredients:

**Table 5: Composition of E-2 minimal medium with 5.5 g/L glucose**

| **Compound** | **Concentration** | **Unit** | **Sterilization** | **Storage** |
|---|---|---|---|---|
| K₂HPO₄ | 7.75 | g/L | Autoclave | RT |
| NaH₂PO₄ · H₂O | 3.76 | g/L | Autoclave | RT |
| (NH₄)₂SO₄ | 2.00 | g/L | Autoclave | RT |
| C₆H₁₂O₆ · H₂O | 5.50 | g/L | Autoclave | RT |
| MgCl₂ · 6H₂O | 100.00 | mg/L | Sterile Filtration | 4 °C |
| C₁₀H₁₆N₂O₈ | 12.70 | mg/L | Sterile Filtration | 4 °C |
| FeSO₄ · 7H₂O | 5.00 | mg/L | Autoclave | 4 °C |
| ZnSO₄ · 7H₂O | 2.00 | mg/L | Sterile Filtration | 4 °C |
| MnCl₂ · 4H₂O | 1.22 | mg/L | Sterile Filtration | 4 °C |
| CaCl₂ · 2H₂O | 1.00 | mg/L | Sterile Filtration | 4 °C |
| CoCl₂ · 6H₂O | 0.40 | mg/L | Sterile Filtration | 4 °C |
| Na₂MoO₄ · 2H₂O | 0.20 | mg/L | Sterile Filtration | 4 °C |
| CuSO₄ · 5H₂O | 0.20 | mg/L | Sterile Filtration | 4 °C |

Cells from cryo-culture (-80 °C) were grown on plates with the described medium and agar at 30 °C. A pre-culture was grown in shake flasks (30 °C, 230 rpm). The cultivation for *cis,cis-*Muconic acid was done in 250 mL shake flasks at the same conditions, with the exception of adding catechol from the hydrothermal conversion and distillation at various concentrations

(It was aimed to start in experiment 4 and 5 with 5 mM catechol, and in experiment 8, 16 and 17 with 1.25 mM catechol).

### Results

Listed below, based on the experiments described in Table 4, the results are summarized, including hydrothermal conversion, distillation, and performed cultivations (Tables 6, 7 and 8).

**Table 6: Hydrothermal Conversion. Catechol yield refers to the mass of the produced catechol in relation to the initial substrate mass (wt %). The total yield relates to yield obtained when besides catechol also phenol, guaiacol, and o, p, m-cresol (cresol total) were taken into account.**

| **Experiment** | **Substrate [g]** | **Catechol [g]** | **Phenol [g]** | **Guaiacol [g]** | **Cresol total [g]** | **Catechol Yield [%]** | **Total Yield [%]** |
|---|---|---|---|---|---|---|---|
| 4 | 47.0 | 5.5 | 0.4 | 3.0 | 0.3 | 11.7 | 19.5 |
| 5 | 28.3 | 0.8 | 0.6 | 0.1 | 0.2 | 2.9 | 6.0 |
| 6 | 5.0 | 0.2 | 0.1 | 0.1 | 0.0 | 4.7 | 9.7 |
| 7 | 5.0 | 0.2 | 0.1 | 0.1 | 0.0 | 3.4 | 8.5 |
| 8 | 5.0 | 0.2 | 0.1 | 0.1 | 0.0 | 3.2 | 7.0 |
| 9 | 5.0 | 0.2 | 0.1 | 0.1 | 0.1 | 4.5 | 10.5 |
| 10 | 5.0 | 0.4 | 0.1 | 0.0 | 0.0 | 7.8 | 10.8 |
| 16 | 5.0 | 0.2 | 0.0 | 0.0 | 0.0 | 3.7 | 4.0 |
| 17 | 5.2 | 0.3 | 0.1 | 0.0 | 0.0 | 5.0 | 7.3 |
| 21 | 5.0 | 0.2 | 0.1 | 0.0 | 0.0 | 4.0 | 7.0 |

**Table 7: Distillation. The substrate is the amount of catechol provided for distillation and catechol is the remaining amount after distillation.**

| **Experiment** | **Substrate [g]** | **Catechol [g]** | **Yield [%]** | **Temperature [°C]** |
|---|---|---|---|---|
| 4 | 2.1 | 2.0 | 94.3 | 100 |
| 5 | 0.5 | 0.5 | 105.7 | 100 |
| 7 | 0.1 | 0.0 | 90.9 | 100 |
| 8 | 0.1 | 0.1 | 98.4 | 100 |
| 10 | 0.1 | 0.1 | 44.7 | 130 |
| 17 | 0.2 | 0.1 | 70.1 | 120 |

**Table 8: Cultivation. The concentration of catechol and cis, cis-muconic acid at the beginning and end of the cultivation, respectively.**

| **Experiment** | **Catechol [mM]** | ***cis, cis*-Muconic acid [mM]** | **Yield [%]** |
|---|---|---|---|
| 4A | 4.1 | 4.1 | 99.2 |
| 4B | 4.2 | 4.0 | 94.4 |
| 5A | 3.3 | 3.0 | 89.7 |
| 5B | 3.3 | 3.1 | 94.8 |
| 8A | 1.1 | 1.0 | 88.6 |
| 8B | 1.1 | 1.0 | 91.1 |
| 16 (not distilled) | 0.8 | 0.8 | 100 |
| 17 | 1.4 | 1.3 | 94.4 |

Clearly the obtained catechol from lignin by the described hydrothermal conversion can be used for the metabolic production of cis, cis-Muconic acid with the P. putida BN6 strain.

### Influence of temperature on distillation

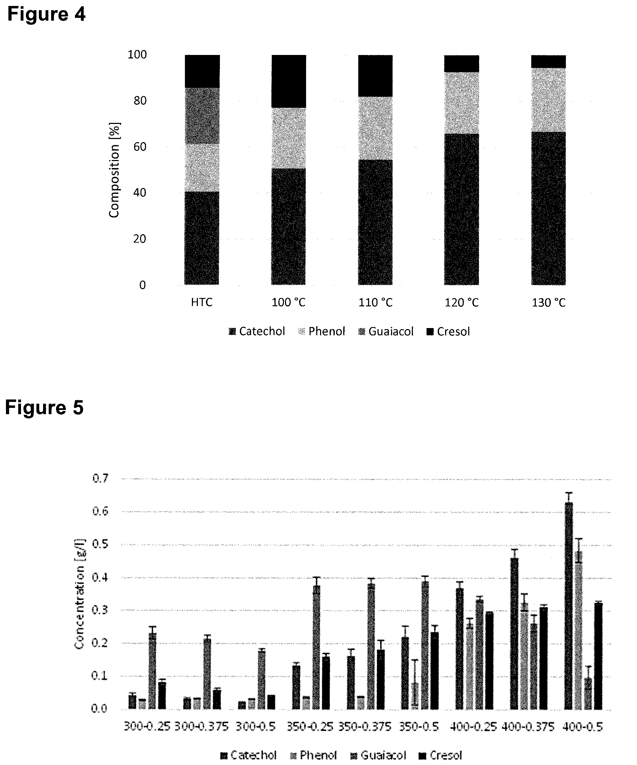

To examine the influence of temperature on distillation, the same liquid phase from a hydrothermal conversion (HTC) was distilled at four different temperatures (100, 110, 120 and 130 °C). In Figure 4 the composition of the original liquid phase from HTC and the compositions of the remaining solutions after the various distillations are shown. Guaiacol was easily separated. However, to separate cresol a higher temperature was needed. At a temperature of 120 °C and higher less cresol was found, but also about 30% of the catechol was lost during the distillation.

Based on these results, the catechol substrate and other compounds can be enriched with this method in order to provide a mixture of aromatics that can metabolically be converted by the cells.

### To define an operation window, experiments for hydrothermal conversion of lignin in water were performed

The dependency on the water density in g/cm3 and temperature in °C causing the accumulation of catechol by the hydrothermal conversion of lignin was defined based on a Design of Experiment (DoE) experiment performed in small reactors (Table 9). All experiments used 0.1 g Kraft lignin from Sigma Aldrich, USA, and the reaction time was 30 min (plus 15 min for heat up). Furthermore, all experiments were conducted in triplicate. The yield is determined by the mass of produced catechol compared to the initial mass of lignin. Besides the concentration of catechol, also the concentrations for phenol, guaiacol, and o-, p-, and m-cresol (in Figure 5 described as cresol) were measured. Based on literature it can be expected that these compounds can also be converted to catechol in the near future by metabolic engineering.

**Table 9: Results of the DoE Experiment**

| **Temperature [°C]** | **Water density [g/cm³]** | **Catechol Yield [%]** | **Total Yield [%]** |
|---|---|---|---|
| 300 | 25 | 0.42 | 3.78 |
| 300 | 37.5 | 0.33 | 3.38 |
| 300 | 50 | 0.23 | 2.78 |
| 350 | 25 | 1.32 | 7.00 |
| 350 | 37.5 | 1.62 | 7.64 |
| 350 | 50 | 2.27 | 9.59 |
| 350 | 64.4 | 0.62 | 4.45 |
| 350 | 65.9 | 0.94 | 5.46 |
| 350 | 67.2 | 1.93 | 8.53 |
| 400 | 25 | 3.57 | 12.18 |
| 400 | 37.5 | 4.46 | 13.12 |
| 400 | 50 | 6.22 | 15.17 |

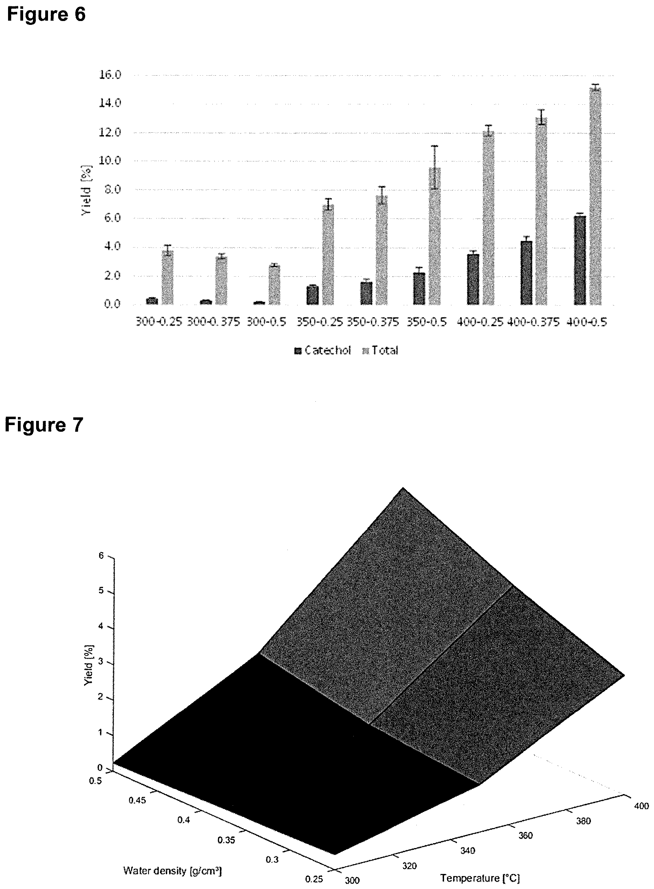

When looking at the outcome of the DoE experiment clearly the temperature and the water density have an influence on the yield of catechol (Fig. 5, 6 and 7).

### Further critical parameters

Experiments investigating the influence of the retention time of the hydrothermal conversion on the yield of catechol showed that maximum values were reached of 6.81% after 60 min. Guaiacol was formed earlier and the concentration declined after 30 min, whereas the amount of phenol was constantly rising over time (Figure 8). For these experiments Kraft lignin from Sigma Aldrich, USA was used. Temperature was set to 400 °C and the water density was 0.50 g/cm3.

At the same conditions at retention times of 30 min, the addition of salts (NaCl, MgCl₂ and CaCl₂) to the reactor at concentrations of 20 g/L (NaCl) or 40 g/L (MgCl₂ and CaCl₂) enhanced the yield of catechol to 7.58, 7.70 and 7.21 g/L, respectively.

Comparison of several lignins at 400°C and 0.50 g/ cm³ water density for 30 min showed that from IndulinAT, Germany, a commercial lignin, a yield of catechol of 5.65 % could be obtained, with Kraft lignin from ECN, the Netherlands a yield of 4.42 % could be reached and with lignin from TCI, Germany only a yield of 1.18% could be reached. Interestingly the lignin from TCI, Germany was much more soluble in water and higher yields of catechol could be obtained at shorter reaction times (3.51% after only 5 min and 15 min heating time). With organosolvent lignin from ECN, the Netherlands and Frauenhofer CBP, Germany yields of 3.98 and 2.06% could be reached, respectively. It is worth mentioning that the total yield, which includes phenol, guaiacol and *o*-, *p-*, *m*-cresol of the last lignin was 10.6% due to its high yield in phenol. This particular yield is in range with that of the other tested lignins (9.4 to 12.1%).

After the addition of NaOH (1M) almost no catechol was obtained when using Kraft lignin from Sigma Aldrich, USA at 400 °C, 0.50 g/cm³ water density and 30 minutes, but when using Kraft lignin from TCI, Germany a pH-shift into the alkaline region improved the yield significantly (350 °C, 0.67 g/cm³ water density, 15 min). With the latter lignin a yield of 2.2% was reached when no NaOH was added. The untreated pH is at about 8.7, when shifting the pH to 11 and 12, a yield of 3.25% and 4.03% were reached, respectively. In the same manner the yield declined when the pH was lowered to acid conditions. Contrary to the yield, the amount of solids rose with the decline of the pH.

### Example 3: pH-controlled fed-batch process using glucose as growth substrate to convert catechol to cis,cis muconic acid

Production performance of P. putida strains JD2S (ΔcatBC Pcat:catA) and BN15 (ΔcatBC Pcat:catA-catA2) was demonstrated in a fed-batch process using catechol as model lignin compound. Cells were grown in E2 minimal medium with glucose as sole carbon source (Hartmans et al., Appl Environ Microbiol. 1989 Nov; 55(11):2850-5). After a short batch phase exponential glucose feeding was started. After 6 hours, catechol was fed pulse-wise into the reactor. Further addition of catechol was coupled to the pH-regulation with the simultaneous addition of NaOH in a molar ratio of 1:2.4.

The fed-batch cultivation was carried out in a 1 L bioreactor (DASGIP, Jülich, Germany) with a working volume of 0.5 L and 1.8 g L-1 glucose in batch. Cultivation temperature was 30°C and pH was adjusted to 7.0 using 6 M NaOH. Aeration rate was 1 vvm and the dissolved oxygen level was maintained above 50% saturation adjusted by the stirrer speed. The glucose feed was composed of E2 minimal medium with 600 g L-1 glucose and 50 g L-1 of ammonium sulfate. Catechol feed contained E2 minimal medium buffer and 2.5 M catechol, and was degassed using nitrogen to prevent oxidation. To avoid foaming 0.02% antifoam 204 (Sigma-Aldrich, Taufkirchen, Germany) was added to batch medium and all feeds. During fed-batch operation pH control was coupled to separate addition of catechol and 6 M NaOH.

After 24 hours 25 g L-1 of cis, cis-muconic acid accumulated in the broth using strain JD2S. The maximum volumetric productivity and the maximum specific productivity were 5.5 g cis,cis-muconic acid per liter and hour (g L-1 h-1) and 0.8 g cis,cis-muconic acid per g dry cell weight (DCW) and hour (i.e. g DCW-1 h-1), respectively. Strain BN15 produced 40 g L-1 in 24 hours with a maximum specific productivity of 0.9 g DCW-1 h-1. A final titer of 61 g L-1¬ was reached.

### Example 4: Generation of a promoter library

Ptuf is a translation elongation factor known as a housekeeping gene in many organisms (Patek et al., Microb Biotechnol. 2013; 6(2):103-17; Becker and Wittmann, Curr Opin Biotechnol. 2012; 23(5):718-26; Kim et al., Appl Microbiol Biotechnol. 2009; 81(6):1097-106). Two versions of the Ptuf have been randomly mutated (i) whole 500 bp sequence of Ptuf (SEQ ID No. 7) and (ii) a short version of 118 bp containing the consensus sequence (-10 and -35 region) predicted by bioinformatic tools (SEQ ID No. 89).

Pgro-co-chaperonin GroES (PP_1360) is responsible for mediating the folding and assembly of many proteins in Pseudomonas (Venturi et al., Mol Gen Genet. 1994; 245(1):126-32). PgroES was identified as a strong promoter under various conditions using RNAseq analysis. A promoter library of Pgro has been constructed using random mutagenesis.

### Materials and Methods

### Mutagenesis PCR and Cultivation

For that purpose the JBS dNTP mutagenesis kit (Jena Bioscience GmbH, Jena, Germany) was used, which contains the dNTP analogues 8-Oxo-dGTP and dPTP. 8-Oxo-dGTP causes transitions from adenine to cytosine and thymine to guanine according at a rate of mutagenesis of approximately 2 % (Zaccolo et al., J Mol Biol. 1996; 255(4):589-603; Cadwell and Joyce, PCR Methods Appl. 1992; 2(1):28-33). DPTP can be inserted in place of any nucleotide with a rate of mutagenesis of approximately 19 %. Both analogues combined raise the mutation rate of over 20 %. For the construction of the promoter library, the parameter of the PCR was set-up to cause a mutagenesis rate of 2-20 %, according to manufactures recommendations.

The analysis of further promoters was performed using the red fluorescence protein (RFP) mCherry as reporter. Therefore, the high copy plasmid pSEVA247R was used for the fusion of the promoter with mCherry. For the analysis of fluorescence, micro scale cultivations (150µl) were performed in E2 minimal medium in a micro bioreactor system that performs high-throughput batch cultivation. Cultivation temperature was 30 °C and 1300 rpm. Monitoring of growth and fluorescence was done every hour by an IEMS microplate reader at OD620 and a fluorescence microplate reader CF (excitation at 544nm, emission at 620nm), respectively. Fluorescence, which is proportional to the amount of reporter protein. The mean value of the three replicates was presented as the experimental promoter activity which was described by the red fluorescence intensity normalized by the biomass.

### Results

The activity of the promoter is measured as RFU per OD600. Results of the measurements are shown in Figure 9.

Table 10 further shows the promoter activity of native and mutated versions of Ptuf and Pgro in Fluorescence units (RFUs) normalized to optical density. S (short), SD (standard deviation)

**Table 10: Results of promoter activity measurements**

| **Promoter** | **RFU (fluorescence/OD₆₀₀)** | **SD** |
|---|---|---|
| Ptuf_native | 1.6 | ±0.09 |
| Ptuf_1 | 0.72 | ±0.04 |
| Ptuf_s_native | 8.19 | ±0.22 |
| Ptuf_s_1 | 0.15 | ±0.02 |
| Ptuf_s_2 | 5.65 | ±0.32 |
| Ptuf_s_3 | 11.4 | ±0.62 |
| Ptuf_s_4 | 14.36 | ±0.87 |
| Ptuf_s_5 | 14.77 | ±0.34 |
| Ptuf_s_6 | 15.59 | ±0.02 |
| Ptuf_s_7 | 18.8 | ±0.3 |
| Ptuf_s_8 | 26.45 | ±1.22 |
| Ptuf_s_9 | 28.04 | ±0.94 |
| Ptuf_s_10 | 138.41 | ±5.21 |
| Ptuf_s_11 | 182.79 | ±6.23 |
| Ptuf_s_12 | 87.68 | ±6.41 |
| Pgro_native | 4.3 | ±0.16 |
| Pgro_1 | 9.1 | ±0.45 |
| Pgro_2 | 40.76 | ±0.92 |
| Pgro_4 | 99.27 | ±6.26 |
| Pgro_5 | 222.2 | ±9.7 |

## Claims

1. A method of producing cis-cis-muconic acid, comprising
i) fluid-assisted decomposition of an organic educt under sub- or supercritical conditions;
ii) obtaining an intermediate product from step i), wherein the intermediate product is catechol; and
iii) subjecting the intermediate product to biocatalytic conversion, by contacting the intermediate product obtained in step ii) with a biocatalyst, wherein said biocatalyst is a host cell selected from the group consisting of bacteria, yeast, filamentous fungi, cyanobacteria, algae, and plant cells, wherein said host cell comprises at least one gene encoding a polypeptide having catechol 1,2-dioxygenase activity, wherein said host cell comprises at least one *catA* gene and at least one *catA2* gene.

2. The method of claim 1, wherein step (ii) comprises steam bath distillation, thereby obtaining the intermediate product.

3. The method of claim 1 or 2, wherein the organic educt comprises lignin, guaiacol; p-coumaryl alcohol; coniferyl alcohol; sinapyl alcohol; cresol; phenol; catechol; polysaccharides; cellulose hemicellulose; xylose; glucose; fructose; proteins; amino acids; triacylglycerides; and/or fatty acids.

4. The method of any of the preceding claims, wherein the intermediate product from step ii) has a degree of purity of 70% or more, preferably 75% or more, more preferably of 80% or more, and/or
wherein the intermediate product comprises phenol and/or cresol.

5. The method of any of the preceding claims, wherein said host cell is
a. selected from *Pseudomonas,* preferably *Pseudomonas putida,* more preferably *Pseudomonas putida* strain KT2440;
b. a non-genetically modified host cell,
c. a recombinant host cell comprising at least one heterologous gene, wherein said at least one heterologous gene is preferably stably integrated into the host cell's genome;
d. a bacterial host cell selected from the group consisting of *Bacillus* bacteria (e.g., *B. subtilis, B. megaterium*), *Acinetobacter* bacteria, *Norcardia* baceteria, *Xanthobacter* bacteria, *Escherichia* bacteria (e.g., *E. coli* (e.g., strains DH10B, Stbl2, DH5-alpha, DB3, DB3.1 ), DB4, DB5, JDP682 and ccdA-over (e.g., U.S. Application No. 09/518,188 ))), *Streptomyces* bacteria, *Erwinia* bacteria, *Klebsiella* bacteria, *Serratia* bacteria (e.g., *S. marcescens*), *Pseudomonas* bacteria (e.g., *P. aeruginosa, P. putida*), *Salmonella* bacteria (e.g., *S. typhimurium, S. typhi*), *Megasphaera* bacteria (e.g., *Megasphaera elsdenii*), photosynthetic bacteria (e.g., green non-sulfur bacteria (e.g., *Choroflexus* bacteria (e.g., *C. aurantiacus*), *Chloronema* bacteria (e.g., *C. gigateum*)), green sulfur bacteria (e.g., *Chlorobium* bacteria (e.g., *C. limicola*), *Pelodictyon* bacteria (e.g., *P. luteolum*), purple sulfur bacteria (e.g., *Chromatium* bacteria (e.g., C. *okenii*)), and purple non-sulfur bacteria (e.g., *Rhodospirillum* bacteria (e.g., *R. rubrum*), *Rhodobacter* bacteria (e.g., *R. sphaeroides, R. capsulatus*), and *Rhodomicrobium* bacteria (e.g., *R. vanellii*));.
e. a yeast host cell selected from the group consisting of *Yarrowia* yeast (e.g., *Y. lipolytica* (formerly classified as Candida lipolytica)), *Candida* yeast (e.g., C. *revkaufi, C. pulcherrima, C. tropicalis, C. utilis*), *Rhodotorula* yeast (e.g., R. *glutinus, R. graminis), Rhodosporidium* yeast (e.g., *R. toruloides*), *Saccharomyces* yeast (e.g., *S. cerevisiae, S. bayanus, S. pastorianus, S. carlsbergensis*), *Cryptococcus* yeast, *Trichosporon* yeast (e.g., *T. pullans, T. cutaneum*), *Pichia* yeast (e.g., *P. pastoris*) and *Lipomyces* yeast (e.g., *L. starkeyii, L. lipoferus*); and/or
f. a fungal host cell selected from the group consisting of *Aspergillus* fungi (e.g., *A. parasiticus, A. nidulans*), *Thraustochytrium fungi, Schizochytrium* fungi and *Rhizopus* fungi (e.g., R. arrhizus, R. oryzae, R. nigricans), e.g. an *A. parasiticus* strain such as strain ATCC24690, or an *A. nidulans* strain such as strain ATCC38163.

6. The method of any of the preceding claims,
wherein said at least one *catA* gene preferably encodes a polypeptide comprising a sequence corresponding to SEQ ID No. 1 and said at least one *catA2* gene preferably encodes a polypeptide comprising a sequence corresponding to SEQ ID No. 3, and/or. wherein said at least one *catA* gene preferably comprises a sequence corresponding to SEQ ID No. 2, and said at least one *catA2* gene preferably comprises a sequence corresponding to SEQ ID No. 4.

7. The method of any of the preceding claims, wherein the host cell comprises
i) at least one *catA* gene encoding a catA polypeptide comprising a sequence corresponding to SEQ ID No. 1; and
ii) at least one *catA2* gene encoding a catA2 polypeptide comprising a sequence corresponding to SEQ ID No. 3.

8. The method of any of the preceding claims, wherein said host cell comprises, operably linked to, e.g. upstream of, the at least one gene, a promoter sequence corresponding to
i) SEQ ID No. 5 [Pem7]; or
ii) SEQ ID No. 6 [Pem7*]; or
iii) SEQ ID No. 7 [Ptuf]; or
iv) SEQ ID No. 8 [PrpoD]; or
v) SEQ ID No. 9 [Plac]; or
vi) SEQ ID No. 10 [PgyrB];
vii) SEQ ID No. 11; or
viii) SEQ ID No. 12; or
ix) SEQ ID No. 13; or
x) SEQ ID No. 14; or
xi) SEQ ID No. 15; or
xii) SEQ ID No. 16; or
xiii) SEQ ID No. 88 [Ptuf_1]; or
xiv) SEQ ID No. 89 [Ptuf_short]; or
xv) SEQ ID No. 90 [Ptuf_s_2]; or
xvi) SEQ ID No. 91 [Ptuf_s_3]; or
xvii) SEQ ID No. 92 [Ptuf_s_4]; or
xviii) SEQ ID No. 93 [Ptuf_s_5]; or
xix) SEQ ID No. 94 [Ptuf_s_6]; or
xx) SEQ ID No. 95 [Ptuf_s_7]; or
xxi) SEQ ID No. 96 [Ptuf_s_8]; or
xxii) SEQ ID No. 97 [Ptuf_s_9]; or
xxiii) SEQ ID No. 98 [Ptuf_s_10]; or
xxiv) SEQ ID No. 99 [Ptuf_s_11]; or
xxv) SEQ ID No. 100 [Ptuf_s_12]; or
xxvi) SEQ ID No. 101 [Pgro]; or
xxvii) SEQ ID No. 102 [Pgro_1]; or
xxviii)SEQ ID No. 103 [Pgro_2]; or
xxix) SEQ ID No. 104 [Pgro_4]; or
xxx) SEQ ID No. 105 [Pgro_5].

9. The method of any of the preceding claims, wherein the at least one gene is constitutively expressed.

10. The method of any one of claims 1 or 6 to 9, wherein the gene is a heterologous gene.

11. The method of any of claims 5 to 10, wherein said at least one heterologous gene is derived from *Pseudomonas,* preferably *Pseudomonas putida,* more preferably *Pseudomonas putida* strain KT2440.

12. The method of any of claims 5 to 11, wherein said host cell is further **characterized in that** it does not express a functional catB polypeptide, and/or **in that** it does not express a functional catC polypeptide, and/or **in that** it does not express a functional pcaB polypeptide.

13. The method of claim 12, wherein the *catB* gene, *catC* gene or *pcaB* gene is silenced, preferably knocked-down or knocked-out, or deleted from the chromosome.

14. The method of claim 13, yielding cis-cis-muconic acid which is white in color, wherein the yield in cis-cis-muconic acid from catechol is preferably greater than 95% w/w, or greater than 99% w/w.

## Patentansprüche

1. Verfahren zur Herstellung von *cis-cis-Muconsäure,* umfassend
i) flüssigkeitsgestützte Zersetzung eines organischen Edukts unter sub- oder überkritischen Bedingungen;
ii) Gewinnung eines Zwischenprodukts aus Schritt i), wobei das Zwischenprodukt Catechol ist; und
iii) Unterziehen des Zwischenprodukts einer biokatalytischen Umwandlung, indem das in Schritt ii) erhaltene Zwischenprodukt mit einem Biokatalysator in Kontakt gebracht wird, wobei der Biokatalysator eine Wirtszelle ist, ausgewählt aus der Gruppe bestehend aus Bakterien, Hefen, filamentösen Pilzen, Cyanobakterien, Algen und Pflanzenzellen, wobei die Wirtszelle mindestens ein Gen umfasst, das für ein Polypeptid mit Catechol-1,2-Dioxygenase-Aktivität kodiert, wobei die Wirtszelle mindestens ein *catA* Gen und mindestens ein *catA2* Gen umfasst.

2. Verfahren nach Anspruch 1, wobei Schritt (ii) die Dampfbaddestillation umfasst, wodurch das Zwischenprodukt erhalten wird.

3. Verfahren nach Anspruch 1 oder 2, wobei das organische Edukt Lignin, Guajakol; p-Cumarylalkohol; Nadelholzalkohol; Sinapylalkohol; Kresol; Phenol; Catechol; Polysaccharide; Zellulose Hemizellulose; Xylose; Traubenzucker; Fruchtzucker; Proteine; Aminosäuren; Triacylglyceride; und/oder Fettsäuren umfasst.

4. Verfahren nach einem der vorhergehenden Ansprüche, wobei das Zwischenprodukt aus Schritt ii) einen Reinheitsgrad von 70 % oder mehr, bevorzugt 75 % oder mehr, mehr bevorzugt von 80 % oder mehr aufweist und/oder
wobei das Zwischenprodukt Phenol und/oder Kresol umfasst.

5. Verfahren nach einem der vorhergehenden Ansprüche, wobei die Wirtszelle
a. ausgewählt aus *Pseudomonas,* bevorzugt *Pseudomonas putida,* besonders bevorzugt *Pseudomonas putida* Stamm KT2440;
b. eine nicht genetisch veränderte Wirtszelle,
c. eine rekombinante Wirtszelle, die mindestens ein heterologes Gen umfasst, wobei das mindestens eine heterologe Gen vorzugsweise stabil in das Genom der Wirtszelle integriert ist;
d. eine bakterielle Wirtszelle, ausgewählt aus der Gruppe bestehend aus *Bacillus* Bakterien (z. B. *subtilis, B. megaterium), Acinetobacter* Bakterien, *Norcardia* baceteria, *Xanthobacter* Bakterien, Escherichia Bakterien (z.B. *E. coli*) (z.B. Stämme DH10B, Stbl2, DH5-alpha, DB3, DB3.1 ), DB4, DB5, JDP682 und ccdA-over (z.B. U.S. Application No. 09/518,188 ))), *Streptomyces* Bakterien, *Erwinia* Bakterien, *Klebsiella* Bakterien, *Serratia* Bakterien (z.B. S. *marcescens*), *Pseudomonas* Bakterien (z.B. *P. aeruginosa, P. putida*), *Salmonellen* Bakterien (z.B. *S. typhimurium, S. typhi*), *Megasphaera* Bakterien (z.B. *Megasphaera elsdenii*), photosynthetische Bakterien (z.B. grüne Nicht-Schwefelbakterien (z.B. *Choroflexus-Bakterien* (z.B. *C. aurantiacus*), *Chloronem* Bakterien (z.B. *C. gigateum*)), grüne Schwefelbakterien (z.B. *Chlorobium* Bakterien (z.B. *C. limicola*), *Pelodictyon* Bakterien (z.B. *P. luteolum*), violette Schwefelbakterien (z.B. *Chromatium* Bakterien (z.B. *C. okenit*)) und violette Nicht-Schwefelbakterien (z.B. *Rhodospirillum* Bakterien (z.B. *R. rubrum*), *Rhodobacter* Bakterien (z.B. *R. sphaeroides, R. capsulatus)* und *Rhodomikrobium* Bakterien (z.B. *R. vanellii*));.
e. eine Hefewirtszelle, ausgewählt aus der Gruppe bestehend aus *Yarrowia* Hefe (z.B. *Y. lipolytica* (früher klassifiziert als Candida lipolytica)), *Candida* Hefe (z.B. *C. revkaufi*, *C. pulcherrima, C. tropicalis, C. utilis*), *Rhodotorula* Hefe (z.B. *R. glutinus, R. graminis*), *Rhodosporidium* Hefe (z.B. *R. toruloides*), *Saccharomyces* Hefe (z.B. *S. cerevisiae, S. bayanus, S. pastorianus, S. carlsbergensis*), *Cryptococcus* Hefe, *Trichosporon-Hefe* (z.B. *T. pullans, T. cutaneum*), *Pichia* Hefe (z.B., *P. pastoris*) und *Lipomyces* Hefe (z.B. *L. starkeyii, L. lipoferus*); und/oder
f. eine pilzliche Wirtszelle, ausgewählt aus der Gruppe, die aus *Aspergillus* Pilzen (z.B. *A. parasiticus, A. nidulans*), *Thraustochytrium* Pilzen, *Schizochytrium* Pilzen und *Rhizopus* Pilzen (z.B. R. arrhizus, R. oryzae, R. nigricans) besteht, z.B. ein *A. parasiticus* Stamm wie Stamm ATCC24690 oder ein *A. nidulans* Stamm wie Stamm ATCC38163.

6. Verfahren nach einem der vorhergehenden Ansprüche,
wobei das mindestens eine *catA* Gen vorzugsweise für ein Polypeptid kodiert, das eine Sequenz umfasst, die der SEQ-ID Nr. 1 entspricht, und das mindestens eine *catA2* Gen vorzugsweise für ein Polypeptid kodiert, das eine Sequenz umfasst, die der SEQ-ID Nr. 3 entspricht, und/oder.
wobei das mindestens eine *catA* Gen vorzugsweise eine Sequenz aufweist, die der SEQ-ID Nr. 2 entspricht, und das mindestens eine *catA2* Gen vorzugsweise eine Sequenz aufweist, die der SEQ-ID Nr. 4 entspricht.

7. Verfahren nach einem der vorhergehenden Ansprüche, wobei die Wirtszelle umfasst
i) mindestens ein *catA* Gen, das für ein catA-Polypeptid kodiert, das eine Sequenz umfasst, die der SEQ-ID Nr. 1 entspricht; und
ii) mindestens ein *catA2* Gen, das für ein catA2-Polypeptid kodiert, das eine Sequenz umfasst, die der SEQ-ID Nr. 3 entspricht.

8. Verfahren nach einem der vorhergehenden Ansprüche, wobei die Wirtszelle umfasst, operativ verbunden mit, z.B. stromaufwärts, dem mindestens einen Gen, eine Promotorsequenz umfasst, die der
i) SEQ-ID-Nr. 5 [Pem7]; oder
ii) SEQ-ID-Nr. 6 [Pem7*]; oder
iii) SEQ ID Nr. 7 [Ptuf]; oder
iv) SEQ-ID-Nr. 8 [PrpoD]; oder
v) SEQ-ID-Nr. 9 [Plac]; oder
vi) SEQ-ID-Nr. 10 [PgyrB];
vii) SEQ-ID-Nr. 11; oder
viii) SEQ-ID-Nr. 12; oder
ix) SEQ-ID-Nr. 13; oder
x) SEQ-ID-Nr. 14; oder
xi) SEQ-ID-Nr. 15; oder
xii) SEQ-ID-Nr. 16; oder
xiii) SEQ-ID-Nr. 88 [Ptuf_1]; oder
xiv) SEQ-ID-Nr. 89 [Ptuf_short]; oder
xv) SEQ-ID-Nr. 90 [Ptuf_s_2]; oder
xvi) SEQ-ID-Nr. 91 [Ptuf_s_3]; oder
xvii) SEQ-ID-Nr. 92 [Ptuf_s_4]; oder
xviii) SEQ-ID-Nr. 93 [Ptuf_s_5]; oder
xix) SEQ-ID-Nr. 94 [Ptuf_s_6]; oder
xx) SEQ-ID-Nr. 95 [Ptuf_s_7]; oder
xxi) SEQ-ID-Nr. 96 [Ptuf_s_8]; oder
xxii) SEQ-ID-Nr. 97 [Ptuf_s_9]; oder
xxiii) SEQ-ID-Nr. 98 [Ptuf_s_10]; oder
xxiv) SEQ-ID-Nr. 99 [Ptuf_s_11]; oder
xxv) SEQ-ID-Nr. 100 [Ptuf_s_12]; oder
xxvi) SEQ-ID-Nr. 101 [Pgro]; oder
xxvii) SEQ-ID-Nr. 102 [Pgro_1]; oder
xxviii)SEQ-ID-Nr. 103 [Pgro_2]; oder
xxix) SEQ-ID-Nr. 104 [Pgro_4]; oder
xxx) SEQ-ID-Nr. 105 [Pgro_5].

9. Verfahren nach einem der vorhergehenden Ansprüche, wobei das mindestens eine Gen konstitutiv exprimiert wird.

10. Verfahren nach einem der Ansprüche 1 oder 6 bis 9, wobei das Gen ein heterologes Gen ist.

11. Verfahren nach einem der Ansprüche 5 bis 10, wobei das mindestens eine heterologe Gen von *Pseudomonas,* vorzugsweise *Pseudomonas putida,* besonders bevorzugt *Pseudomonas putida,* Stamm KT2440, abgeleitet ist.

12. Verfahren nach einem der Ansprüche 5 bis 11, wobei die Wirtszelle ferner **dadurch gekennzeichnet ist, dass** sie kein funktionelles catB-Polypeptid exprimiert und/oder dass sie kein funktionelles catC-Polypeptid exprimiert und/oder dass sie kein funktionelles pcaB-Polypeptid exprimiert.

13. Verfahren nach Anspruch 12, wobei das *catB* Gen, das *catC* Gen oder das *pcaB* Gen zum Schweigen gebracht, vorzugsweise heruntergeschaltet oder ausgeschaltet oder vom Chromosom entfernt wird.

14. Verfahren nach Anspruch 13, bei dem cis-cis-Muconsäure eine weiße Farbe ergibt, wobei die Ausbeute an cis-cis-Muconsäure aus Catechol vorzugsweise größer als 95 Gew.-% oder größer als 99 Gew.-% ist.

## Revendications

1. Procédé de production d' acide cis-cis-muconique, comprenant
i) décomposition assistée par fluide d'un conduit organique dans des conditions subcritiques ou supercritiques ;
ii) l'obtention d'un produit intermédiaire à partir de l'étape I), dans lequel le produit intermédiaire est le catéchol ; et
iii) soumettre le produit intermédiaire à une conversion biocatalytique, en mettant en contact le produit intermédiaire obtenu à l'étape ii) avec un biocatalyseur, dans lequel ledit biocatalyseur est une cellule hôte choisie dans le groupe constitué de bactéries, de levures, de champignons filamenteux, de cyanobactéries, d'algues et de cellules végétales, ladite cellule hôte comprenant au moins un gène codant pour un polypeptide ayant une activité catéchol 1,2-dioxygénase, dans laquelle ladite cellule hôte comprend au moins un gène *catA* et au moins un gène *catA2.*

2. Procédé selon la revendication 1, dans lequel l'étape (ii) comprend la distillation par bain de vapeur, obtenant ainsi le produit intermédiaire.

3. Procédé selon la revendication 1 ou 2, dans lequel l'éducteur organique comprend de la lignine, du gaïacol; alcool p-coumarylique ; alcool conifère ; alcool sinapylique ; crésol; phénol; Catéchol; polysaccharides ; l'hémicellulose de cellulose ; xylose; glucose; fructose; protéine; acides aminés ; triacylglycérides ; et/ou d'acides gras.

4. Procédé selon l'une quelconque des revendications précédentes, dans lequel le produit intermédiaire de l'étape ii) a un degré de pureté de 70 % ou plus, de préférence 75 % ou plus, de préférence de 80 % ou plus, et/ou
dans lequel le produit intermédiaire comprend du phénol et/ou du crésol.

5. Procédé selon l'une quelconque des revendications précédentes, dans lequel ladite cellule hôte est
a. choisie parmi les *Pseudomonas,* de préférence *Pseudomonas putida,* plus préférentiellement la souche KT2440 de *Pseudomonas putida* ;
b. une cellule hôte non génétiquement modifiée,
c. une cellule hôte recombinante comprenant au moins un gène hétérologue, dans laquelle ledit au moins un gène hétérologue est de préférence intégré de manière stable dans le génome de la cellule hôte ;
d. une cellule hôte bactérienne choisie dans le groupe constitué des bactéries *Bacillus* (p. ex., *B. subtilis, B. megaterium),* des bactéries *Acinetobacter,* des bactéries *Norcardia* baceteria, des bactéries *Xanthobacter,* des bactéries *Escherichia* (p. ex., *E. coli* (p. ex., souches DH10B, Stbl2, DH5-alpha, DB3, DB3.1), DB4, DB5, JDP682 et ccdA-over (p. ex., demande américaine n° 09/518,188))), les bactéries *Streptomyces,* les bactéries *Erwinia,* les bactéries Klebsiella, les bactéries *Serratia* (p. ex., *S. marcescens*), les bactéries *Pseudomonas* (p. ex., *P. aeruginosa, P. putida*), *Salmonella* bactéries (p. ex., *S. typhimurium, S. typhi*), bactéries *Megasphaera* (p. ex., *Megasphaera elsdenii*), bactéries photosynthétiques (p. ex., bactéries vertes non soufrées (p. ex., bactéries *Choroflexus* (p. ex., *C. aurantiacus*), bactéries *Chloronema* (p. ex., *C. gigateum*)), bactéries vertes du soufre (p. ex., *Chlorobium* bactéries (p. ex., *C. limicola*), les bactéries *Pelodictyon* (p. ex., *P. luteolum*), les bactéries soufrées pourpres (p. ex., les bactéries *Chromatium* (p. ex., *C. okenii*)), les bactéries violettes non soufrées (p. ex., les bactéries *Rhodospirillum* (p. ex., *R. rubrum*), les bactéries *Rhodobacter* (p. ex., *R. sphaeroides, R. capsulatus*) et les bactéries *Rhodomicrobium* (p. ex., *R. vanellii*)) ;.
e. une cellule hôte de levure choisie dans le groupe constitué de levures *Yarrowia* (par exemple, *Y. lipolytica* (anciennement classée comme Candida lipolytica)), de levures *Candida* (par exemple, *C. revkaufi, C. pulcherrima, C. tropicalis, C. utilis*), de levures *Rhodotorula* (par exemple, *R. glutinus, R. graminis*), Levure *Rhodosporidium* (p. ex., *R. toruloides*), levure *Saccharomyces* (p. ex., *S*. *cerevisiae, S. bayanus, S. pastorianus, S. carlsbergensis*), levure *Cryptococcus*, *levure Trichosporon* (p. ex., *T. pullans, T. cutaneum*), levure *Pichia* (p. ex. P. *pastoris*) et les levures *Lipomyces* (p. ex., *L. starkeyii, L. lipoferus*) ; et/ou
f. une cellule hôte fongique choisie dans le groupe constitué des champignons *Aspergillus* (p. ex., *A. parasiticus, A. nidulans*), des champignons *Thraustochytrium*, des champignons *Schizochytrium* et des champignons *Rhizopus* (p. ex., R. arrhizus, R. oryzae, R. nigricans), p. ex. une souche d'A. *parasiticus* telle que la souche ATCC24690, ou une souche *d'A. nidulans* telle que la souche ATCC38163.

6. Procédé selon l'une quelconque des revendications précédentes,
où au moins un *catA* code de préférence pour un polypeptide comprenant une séquence correspondant à SEQ ID n° 1 et ledit au moins un *catA2* code de préférence pour un polypeptide comprenant une séquence correspondant à l'ID SEQ n° 3, et/ou.
où au moins un *catA* comprend de préférence une séquence correspondant à SEQ ID n° 2, et ledit gène au moins un *catA2* comprend de préférence une séquence correspondant à l'ID SEQ n° 4.

7. Procédé selon l'une quelconque des revendications précédentes, dans lequel la cellule hôte comprend
i) au moins un gène *catA* codant pour un polypeptide catA comprenant une séquence correspondant à l'ID SEQ n° 1 ; et
ii) au moins un gène *catA2* codant pour un polypeptide catA2 comprenant une séquence correspondant à l'ID SEQ n° 3.

8. Procédé selon l'une quelconque des revendications précédentes, dans lequel ladite cellule hôte comprend, liée de manière opérante, par exemple en amont du ou des gènes, une séquence promotrice correspondant à
i) Numéro d'identification SEQ n° 5 [Pem7] ; ou
ii) Numéro d'identification SEQ n° 6 [Pem7*] ; ou
iii) Numéro d'identification SEQ n° 7 [Ptuf] ; ou
iv) Numéro d'identification SEQ n° 8 [PrpoD] ; ou
v) SEQ ID n° 9 [Plac] ; ou
vi) Numéro d'identification SEQ n° 10 [PgyrB] ;
vii) Numéro d'identification SEQ n° 11 ; ou
viii) Numéro d'identification SEQ n° 12 ; ou
ix) Numéro d'identification SEQ n° 13 ; ou
x) SEQ ID n° 14 ; ou
xi) Numéro d'identification SEQ n° 15 ; ou
xii) Numéro d'identification SEQ n° 16 ; ou
xiii) Numéro d'identification SEQ n° 88 [Ptuf_1] ; ou
xiv) Numéro d'identification SEQ n° 89 [Ptuf_short] ; ou
xv) Numéro d'identification SEQ n° 90 [Ptuf_s_2] ; ou
xvi) Numéro d'identification SEQ n° 91 [Ptuf_s_3] ; ou
xvii) Numéro d'identification SEQ n° 92 [Ptuf_s_4] ; ou
xviii) Numéro d'identification SEQ n° 93 [Ptuf_s_5] ; ou
xix) Numéro d'identification SEQ n° 94 [Ptuf_s_6] ; ou
xx) Numéro d'identification SEQ n° 95 [Ptuf_s_7] ; ou
xxi) Numéro d'identification SEQ n° 96 [Ptuf_s_8] ; ou
xxii) Numéro d'identification SEQ n° 97 [Ptuf_s_9] ; ou
xxiii) Numéro d'identification SEQ n° 98 [Ptuf_s_10] ; ou
xxiv) Numéro d'identification SEQ n° 99 [Ptuf_s_11] ; ou
xxv) Numéro d'identification SEQ n° 100 [Ptuf_s_12] ; ou
xxvi) Numéro d'identification SEQ n° 101 [Pgro] ; ou
xxvii) Numéro d'identification SEQ n° 102 [Pgro_1] ; ou
xxviii) Numéro d'identification SEQ n° 103 [Pgro_2] ; ou
xxix) Numéro d'identification SEQ n° 104 [Pgro_4] ; ou
xxx) Numéro d'identification SEQ n° 105 [Pgro_5].

9. Procédé selon l'une quelconque des revendications précédentes, dans lequel le ou les gènes sont exprimés de manière constitutive.

10. Procédé selon l'une quelconque des revendications 1 ou 6 à 9, dans lequel le gène est un gène hétérologue.

11. Procédé selon l'une quelconque des revendications 5 à 10, dans lequel ledit au moins un gène hétérologue est dérivé de *Pseudomonas,* de préférence *Pseudomonas putida,* plus préférentiellement de la souche KT2440 de *Pseudomonas putida.*

12. Procédé selon l'une quelconque des revendications 5 à 11, dans lequel ladite cellule hôte est en outre **caractérisée en ce qu'**elle n'exprime pas un polypeptide catB fonctionnel, et/ou **en ce qu'**elle n'exprime pas un polypeptide fonctionnel de catC, et/ou **en ce qu'**elle n'exprime pas un polypeptide fonctionnel de type pcaB.

13. Procédé selon la revendication 12, dans lequel le gène *catB,* le gène *catC* ou le gène *pcaB* est réduit au silence, de préférence inactivé ou assommé, ou supprimé du chromosome.

14. Procédé selon la revendication 13, produisant de l'acide cis-cis-muconique de couleur blanche, dans lequel le rendement en acide cis-cis-muconique du catéchol est de préférence supérieur à 95 % p/p, ou supérieur à 99 % p/p.
